# EUROPEAN PATENT SPECIFICATION

(11) **EP 4 496 785 B1**
(45) Date of publication and mention of the grant of the patent: **24.12.2025**
(21) Application number: 23718850.3
(22) Date of filing: 20.03.2023
(51) Int. Cl.: C07C 229/30, C07C 229/46, C07C 311/51, A01N 37/18, A01N 1/00

(54) **PESTICIDAL AND HERBICIDAL COMPOUNDS AND METHODS OF USE THEREOF**
PESTIZIDE UND HERBIZIDE VERBINDUNGEN UND VERFAHREN ZUR VERWENDUNG DAVON
COMPOSÉS PESTICIDES ET HERBICIDES ET LEURS PROCÉDÉS D'UTILISATION

(30) Priority: 21.03.2022 IL 29157522
(43) Date of publication of application: 29.01.2025
(73) Proprietor: AG Plenus Ltd, 7612002 Rehovot (IL)
(72) Inventor: INBAL, Boaz, 9978800 Kfar Shmu'el (IL); WEXSELBLATT, Ezequiel, 9085500 Shoeva (IL); FREUD, Yehoshua, 9841107 Maale Adumim (IL); LOTHAR, Willms, 56204 Hillscheid (DE)
(74) Representative: BIP Patents
(86) International application number: PCT/IL2023/050289
(87) International publication number: WO 2023/181030

(56) References cited:
- WO-A1-2021/018833

## Description

### FIELD OF THE INVENTION

The present invention relates to novel pesticidally and/or herbicidally active compounds, agrochemical composition thereof, methods of preparation thereof, and uses thereof for controlling the growth of undesirable plants (e.g., weeds), for example in crop fields.

### BACKGROUND OF THE INVENTION

Weeds often interfere with efficient utilization of land and water resources and typically compete with desired plants for water, nutrients, light, carbon dioxide, and space. Many weeds are also aesthetically displeasing, especially when the weeds appear within a stand of a desired plant, such as St. Augustine grass or Kentucky bluegrass in a homeowners lawn. Weeds may also obstruct visibility, become fire hazards around buildings, and reduce the efficiency of irrigation systems. When weeds appear in watercourses, such as rivers and lakes, the weeds may contribute to poor water quality, making the water unsuitable for culinary and industrial uses. Furthermore, some weeds act in a poisonous fashion against other plants, animals, and humans by secreting toxic substances known as allelopathic compounds or by spreading agents that may cause allergies and/or disease. Finally, weeds provide shelter for insects and rodents that spread disease or are otherwise harmful to desired plants, animals, or humans.

Weeds cause agricultural losses to crops that consistently exceed losses caused by other classes of agricultural pests, year after year. Besides reducing the quality of the crop, weed infestation may reduce achievable crop yield by up to 100% of the theoretically achievable yield. A number of approaches, including mechanical, agricultural, biological, and chemical techniques, have evolved in an attempt to control weed infestation.

Mechanical means, such as hand pulling, hoeing or cultivation, deep plowing, clipping, mowing, burning and/or mulching, may be employed in an attempt to eradicate or control weeds. Also, cover crops may be planted to keep the ground covered when not growing more valuable crops and thus weed infestation that would ordinarily be expected to occur in bare ground areas is typically minimized. Crop rotation and planting of "smother" crops that are adapted to grow more vigorously than weeds have also been attempted as means of controlling weed infestations. Besides these mechanical and agricultural techniques, biological methods of weed control, such as introduction of predator populations that feed on the weeds and thereby reduce weed population, have also been attempted.

Mechanical, agricultural, and biological methods of weed control, while sometimes helping to reduce the extent of weed infestations, are not fully satisfactory. First, mechanical and agricultural techniques are quite labor intensive and require use of limited physical and capital resources. Furthermore, environmental factors beyond the control of the farmer or homeowner, such as excessive rainfall, may diminish the effectiveness of these mechanical and agricultural techniques. Likewise, biological techniques,
such as introduction of predator populations, are not entirely satisfactory, since the predators may not be selective for only the weed population.

Chemically active herbicides represent another potential weed control technique. These chemical herbicides may be broken down into pre-emergent herbicides and post-emergent herbicides. Pre-emergent herbicides typically interfere with germination of weed seeds, whereas post-emergent herbicides kill the weeds after the weed seeds have germinated and weed growth has begun. WO2021/018833A1 relates to herbicidal cyclohexanedione compounds.

Pre-emergent herbicides may be effective when present at the required dosage at the time weed seed germination is ready to occur. However, this timing issue points out a major problem with respect to pre-emergent herbicides. Specifically, if the pre-emergent herbicide is not applied, or degrades, prior to weed seed germination, the weed seeds are free to germinate and begin growing into mature weeds. Additionally, pre-emergent herbicides are typically weed specific and are not equally effective against all types of weeds. The timing problem present with pre-emergent herbicides may be avoided by employing post-emergent herbicides and by applying the post-emergent herbicide only after the weed seeds have germinated and the weeds are actively growing. However, many presently available post-emergent herbicides are non-selective herbicides and therefore will kill desirable plants in addition to weeds.

Many pre- and post-emergent herbicides also suffer from another problem. Specifically, many pre-emergent herbicides and post-emergent herbicides are either moderately or highly toxic to humans and animals and may thereby have damaging effects far beyond the intended weed control effect. Toxic herbicides may cause injury either immediately or over the long term to persons applying the herbicides and to persons present when the herbicides are applied. Also, residual concentrations of toxic herbicides that remain in the soil or water after application of the herbicide may pose a significant threat to human beings and to animals, including land-based animals and amphibians and fish, upon contact with the treated area or runoff from the treated area. Furthermore, public alarm about the use of toxic chemicals as herbicides and their potential widespread and long-term effects on environmental quality dictate against the continued use of these toxic herbicides.

There is a need for a pesticidal and/or herbicidal solution that avoids the critical timing issues of pre-emergent herbicide applications. Furthermore, there is a need for a pesticidal and/or herbicidal solution that avoids the toxic effects of presently available pre-emergent and post-emergent herbicides on human beings, animals and the environment generally. Furthermore, there is a need for an economically efficient post-emergent weed technique that selectively controls weeds without destroying or hindering the growth of desired plants. In addition, there is a need for a composition that reduces the amount of herbicides necessary to obtain sufficient weed control while minimizing the harm to crop plants.

As more weeds become resistant to herbicides, alternative compositions with high weed control are desired. Further, as no-till farming continues to increase in popularity, there is a greater need for effective herbicides. A composition with effective weed control and lower dosage rate will lead to increased crop plant yields, and decreased environmental, human, and mammalian health concerns.

### SUMMARY OF THE INVENTION

In various embodiments, this invention is directed to a compound represented by the structure of formula **I, II(a)-II(d),** and **III(a)-III(c)** as defined herein below or its agrochemically acceptable salt, zwitterion (inner salt), stereoisomer, tautomer, hydrate, *N*-oxide, reverse amide analog, isotopic variant (e.g., deuterated analog), or any combination thereof.

In various embodiments, this invention is directed to a compound represented by the structure of formula **I** as described hereinbelow wherein at least one of **A, B** and **C** rings is not absent.

In various embodiments, this invention is directed to a compound represented by the structure of formula **I** as described hereinbelow wherein one of **R₃** and **R₄** is NH₂ and the other one is OH.

In various embodiments, this invention is directed to a compound represented by the structure of formula **I** as described hereinbelow wherein at least one of **R₃** and **R₄** is NH₂.

In some embodiments, the compound is not (*E*)-7-aminooct-4-enoic acid. In some embodiments, the compound is not 7-aminooct-4-enoic acid. In some embodiments, the compound is represented by the structure of formula **II(a), II(b), II(c), II(d), III(a), III(b)** or **III(c)** as described hereinbelow. In some embodiments, **R₂₀** is F or methyl. In some embodiments, **R₁** is H and **R₂** is CH₃ or CH₂CH₃, or **R₁** and **R₂** are joined to form a cyclohexyl. In some embodiments, **R₂'** is H or CH₃ and **R₃** is H, OH or NH₂, or **R₃** and **R₂'** are joined to form a cyclopropyl. In some embodiments, **R₄** is H or NH₂. In some embodiments, **R**₄₀ is H, and **R₃** is H, OH or NH₂ or **R₃** and **R₄₀** are joined together to form a 3-8 membered cycloalkyl ring. In some embodiments, **X₁** is O. In some embodiments, **R₅** is H.

In some embodiments, the compound is a substantially pure single stereoisomer. In some embodiments, the substantially pure stereoisomer has a purity higher than 90%. In some embodiments, the substantially pure stereoisomer has a purity higher than 95%. In some embodiments, the substantially pure stereoisomer has a purity higher than 98%. In some embodiments, the compound is compound any one of the compounds listed in **Table 1** herein below or an agrochemically acceptable salt, zwitterion (inner salt), stereoisomer, tautomer, hydrate, N-oxide, reverse amide analog, or isotopic variant thereof; each represents a separate embodiment according to this invention. In some embodiments, the compound is an herbicide, a pesticide or combination thereof.

In various embodiments, this invention is directed to an agrochemical composition comprising the compound of this invention and an agrochemically acceptable carrier or diluent.

In various embodiments, this invention is directed to a pesticidal and/or an herbicidal compound represented by the structure of formula **I, II(a)-II(d),** and **III(a)-III(c)** as defined herein below, or an agrochemically acceptable salt, zwitterion (inner salt), stereoisomer, tautomer, hydrate, *N*-oxide, reverse amide analog, or isotopic variant (e.g., deuterated analog) thereof; each represents a separate embodiment according to this invention.

In various embodiments, this invention is directed to an agrochemical composition comprising a pesticidally and/or herbicidally effective amount of a compound according to this invention.

In various embodiments, this invention is directed to a method of controlling the growth of undesired plants, comprising applying a compound according to this invention or an agrochemical composition according to this invention, to fields of useful crops.

In various embodiments, this invention is directed to a compound according to this invention, or an agrochemical composition according to this invention, for use in controlling the growth of undesired plants. In some embodiments, the plant is a eudicot (dicot) or a monocotyledon (monocot). In some embodiments, the plant is a weed. In some embodiments, the weed comprises: *Abutilon theophrasti , Amaranthus palmeri, Ambrosia artemisiifolia , Alopecurus myosuroides , Avena sterilis , Chenopodium album , Conyza Canadensis , Digitaria sanguinalis , Echinochloa colona, Euphorbia heterophylla , Lolium perenne , Lolium rigidum , Matricaria chamomilla , Phalaris paradoxa , Poa annua , Portulaca oleracea , Setaria viridis, Solanum nigrum* or any combination thereof. In some embodiments, the dicot plant is *Arabidopsis thaliana,* and/or the monocot plant is *Dactyloctenium aegyptium* or *Eragrostis teff.* In some embodiments, the compound is for use in pre-plant treatments, pre-emergence treatments, post-emergence treatments, or any combination thereof; each represents a separate embodiment according to this invention.

### DETAILED DESCRIPTION OF THE INVENTION

In various embodiments, this invention is directed to a compound represented by the structure of formula (**I**): 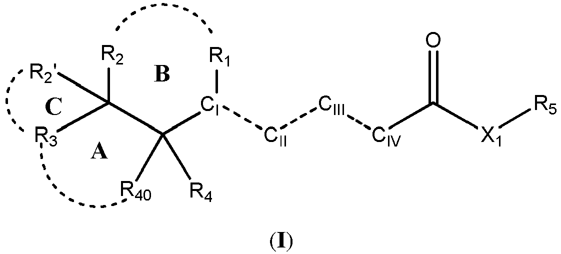 wherein
**C_{I}, C_{II}**, **C_{III}** and **C_{IV}** are each independently an sp, sp² or sp³ carbon atom depending on whether they are part of a triple, double, or a single bond respectively; and are each independently C; CH or **CR₂₀**; or CH₂, CH**R₂₀** or C(**R₂₀**)₂ for sp; sp² or sp³ carbon atom respectively;
   wherein **R₂₀** is a halogen (e.g., F) or a C₁-C₅ linear of branched alkyl (e.g., methyl);
**C_{I}---C_{II}**, **C_{II}---C_{III}**, **C_{III}---C_{IV}** are each independently a single bond or a double bond, wherein at least one of **C_{I}---C_{II}**, **C_{II}---C_{III},** and **C_{III}---C_{IV}** is a double bond; or **C_{I}---C_{II}**, **C_{II}---C_{III}**, and **C_{III}---C_{IV}** are each independently a single bond or a triple bond, wherein at least one of **C_{I}---C_{II}, C_{II}---C_{III},** and **C_{III}---C_{IV}** is a triple bond;
   **R₁** is H, F, Cl, Br, I, C₁-C₅ linear or branched, substituted or unsubstituted alkyl (e.g., methyl, ethyl, propyl, iso-propyl, t-Bu, iso-butyl, pentyl, benzyl);
   wherein if **C_{I}---C_{II}** is a triple bond then **R₁** is absent;
**R₂** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
   or **R₁** and **R₂** are joined to form a 3-8 membered substituted or unsubstituted, saturated or unsaturated carbocyclic ring **B** (e.g., cyclohexyl, cyclohexenyl, cyclopentyl);
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
   or **R₃** and **R₂'** are joined to form a 3-8 membered substituted or unsubstituted, cycloalkyl ring **C** (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
   or **R₃** and **R₄₀** are joined together to form a 3-8 membered substituted or unsubstituted, cycloalkyl ring **A** (e.g., cyclopropyl, cyclobutyl, cyclopentyl, cyclohexyl);
   or **R₄** and **R₄₀** are joined to form a 3-8 membered substituted or unsubstituted, heterocyclic or cycloalkyl ring;
**R₅** is H, C₁-C₅ linear or branched, substituted or unsubstituted alkyl (e.g., methyl, CH₂SH, ethyl, butyl, CH₂-CCH, iso-propyl, CH₂-C(O)-OCH₃), C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, CH₂, NH or N-R₅₀;
**R₈** is [CH₂]ₚ
   wherein **p** is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, i-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**B** ring is absent, or is a 3-8 membered substituted or unsubstituted, saturated or unsaturated carbocyclic ring (e.g., cyclohexyl, cyclohexenyl, cyclopentyl);
**C** ring is absent, or is a 3-8 membered substituted or unsubstituted, cycloalkyl ring (e.g., cyclopropyl);
or its agrochemically acceptable salt, zwitterion (inner salt), stereoisomer, tautomer, hydrate, *N*-oxide, reverse amide analog, isotopic variant (e.g., deuterated analog), or any combination thereof.

In some embodiments, at least one of **R₃** and **R₄** is NH₂. In some embodiments, at least one of **A, B and C** rings is not absent. In some embodiments, one of **R₃** and **R₄** is NH₂ and the other one is OH. In some embodiments, the compound is not (*E*)-7-aminooct-4-enoic acid. In some embodiments, the compound is not 7-aminooct-4-enoic acid. In some embodiments, the compound is a substantially pure single stereoisomer.

In some embodiments, the compound is any one of the compounds listed in **Table 1.** In various embodiments, the compound is an herbicidal compound. In various embodiments, the compound is for use in controlling the growth of undesired plants. In various embodiments, the compound is a pesticide. In various embodiments, the compound is a pesticidal compound.

In various embodiments, the compound of formula **I** is represented by any one of the structures presented in **Table 1:**

**Table 1:**

| **Compound Number** | **Structure** |
|---|---|
| **100** | 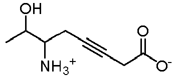 |
| **101** | 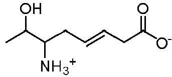 |
| **102** | 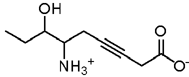 |
| **103** | 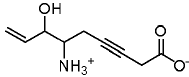 |
| **104** | 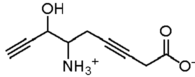 |
| **105** | 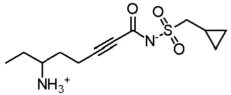 |
| **106** | 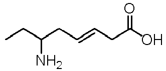 |
| **107** | 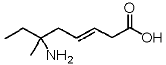 |
| **108** | 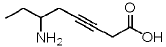 |
| **109** | 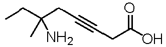 |
| **110** | 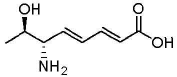 |
| **111** | 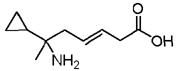 |
| **112** | 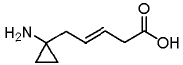 |
| **113** | 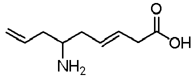 |
| **114** | 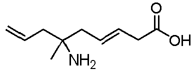 |
| **115** | 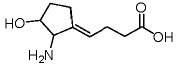 |
| **116** | 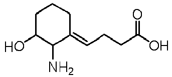 |
| **117** | 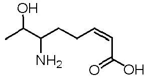 |
| **118** | 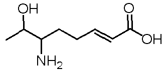 |
| **119** | 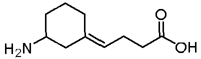 |
| **120** | 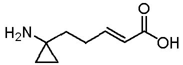 |
| **121** | 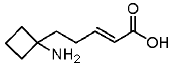 |
| **122** | 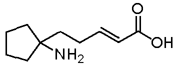 |
| **123** | 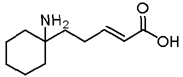 |
| **124** | 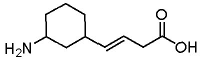 |
| **125** | 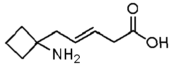 |
| **126** | 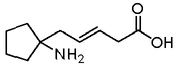 |
| **127** | 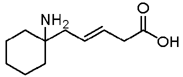 |
| **128** | 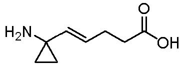 |
| **129** | 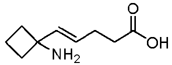 |
| **130** | 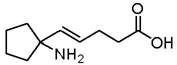 |
| **131** | 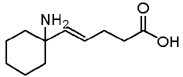 |
| **132** | 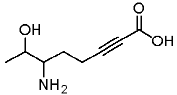 |
| **133** | 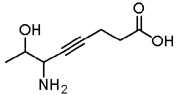 |
| **134** | 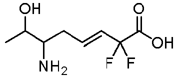 |
| **135** | 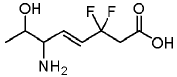 |
| **136** | 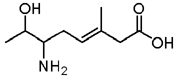 |
| **137** | 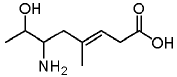 |
| **138** | 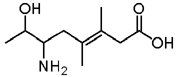 |
| **139** | 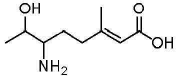 |
| **140** | 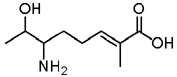 |
| **141** | 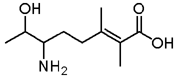 |
| **142** | 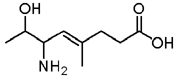 |
| **143** | 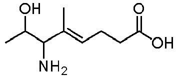 |
| **144** | 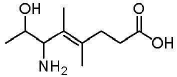 |
| **145** | 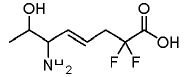 |
| **146** | 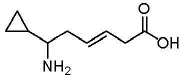 |
| **147** | 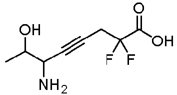 |
| **148** | 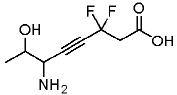 |
| **149** | 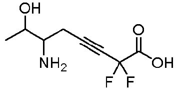 |
| **150** | 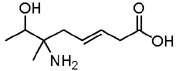 |
| **151** | 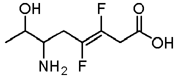 |
| **152** | 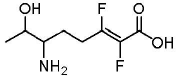 |
| **153** | 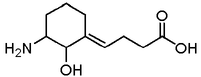 |
| **154** | 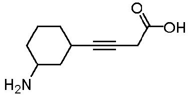 |
| **155** | 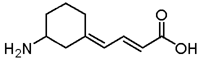 |
| **156** | 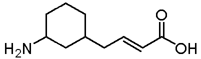 |
| **157** | 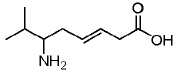 |
| **158** | 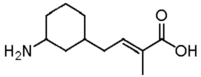 |
| **159** | 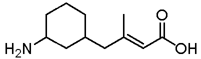 |
| **160** | 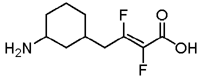 |
| **161** | 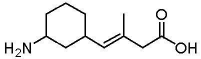 |
| **162** | 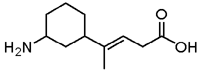 |
| **163** | 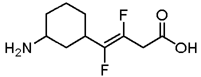 |
| **164** | 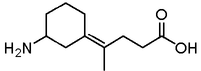 |
| **165** | 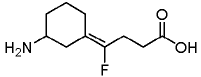 |
| **166** | 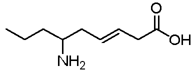 |
| **167** | 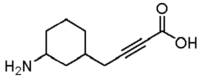 |
| **168** | 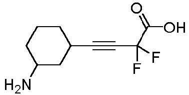 |
| **169** | 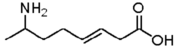 |
| **170** | 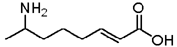 |
| **171** | 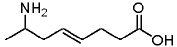 |
| **172** | 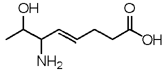 |
| **173** | 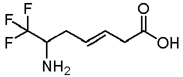 |
| **174** | 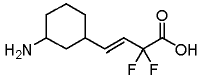 |
| **175** | 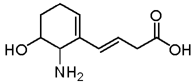 |
| **176** | 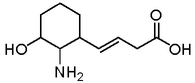 |
| **177** | 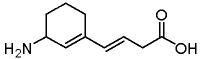 |
| **178** | 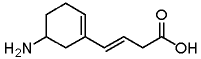 |

In various embodiments, this invention is directed to a compound represented by the structure of formula **II**(a): 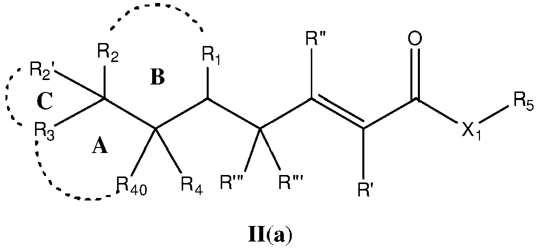 wherein
**R', R"** and **R‴** are each independently selected from a halogen (e.g., F) or a C₁-C₅ linear of branched alkyl (e.g., methyl);
**R₁** is H, F, Cl, Br, I, C₁-C₅ linear or branched, substituted or unsubstituted alkyl (e.g., methyl, ethyl, propyl, iso-propyl, t-Bu, iso-butyl, pentyl, benzyl);
**R₂** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
   or **R₁** and **R₂** are joined to form a 3-8 membered substituted or unsubstituted, saturated or unsaturated carbocyclic ring **B** (e.g., cyclohexyl, cyclohexenyl, cyclopentyl);
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
   or **R₃** and **R₂'** are joined to form a 3-8 membered substituted or unsubstituted, cycloalkyl ring C (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
   or **R₃** and **R₄₀** are joined together to form a 3-8 membered substituted or unsubstituted, cycloalkyl ring A (e.g., cyclopropyl, cyclobutyl, cyclopentyl, cyclohexyl);
   or **R₄** and **R₄₀** are joined to form a 3-8 membered substituted or unsubstituted, heterocyclic or cycloalkyl ring;
**R₅** is H, C₁-C₅ linear or branched, substituted or unsubstituted alkyl (e.g., methyl, CH₂SH, ethyl, butyl, CH₂-CCH, iso-propyl, CH₂-C(O)-OCH₃), C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, CH₂, NH or N-R₅₀;
**R₈** is [CH₂]ₚ
   wherein **p** is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, i-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**B** ring is absent, or is a 3-8 membered substituted or unsubstituted, saturated or unsaturated carbocyclic ring (e.g., cyclohexyl, cyclohexenyl, cyclopentyl);
**C** ring is absent, or is a 3-8 membered substituted or unsubstituted, cycloalkyl ring (e.g., cyclopropyl);
or its agrochemically acceptable salt, zwitterion (inner salt), stereoisomer, tautomer, hydrate, N-oxide, reverse amide analog, isotopic variant (e.g., deuterated analog), or any combination thereof.

In some embodiments, at least one of **R₃** and **R₄** is NH₂. In some embodiments, at least one of **A, B** and **C** rings is not absent. In some embodiments, one of **R₃** and **R₄** is NH₂ and the other one is OH.

In some embodiments, the compound is any one of the compounds listed in **Table 1.** In various embodiments, the compound is an herbicidal compound. In various embodiments, the compound is for use in controlling the growth of undesired plants. In various embodiments, the compound is a pesticide. In various embodiments, the compound is a pesticidal compound.

In various embodiments, the compound of formula **II**(**a**) is represented by any one of the following structures:

| **Compound Number** | **Structure** |
|---|---|
| **117** | 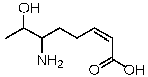 |
| **118** | 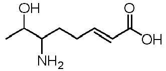 |
| **120** | 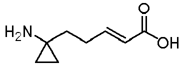 |
| **121** | 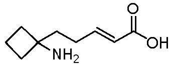 |
| **122** | 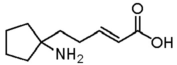 |
| **123** | 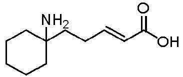 |
| **139** | 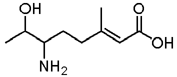 |
| **140** | 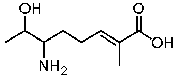 |
| **141** | 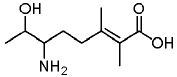 |
| **152** | 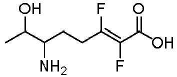 |
| **156** | 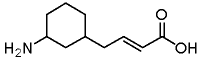 |
| **158** | 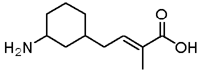 |
| **159** | 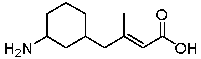 |
| **160** | 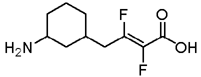 |
| **170** | 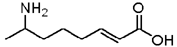 |

In various embodiments, this invention is directed to a compound represented by the structure of formula **II(b):** 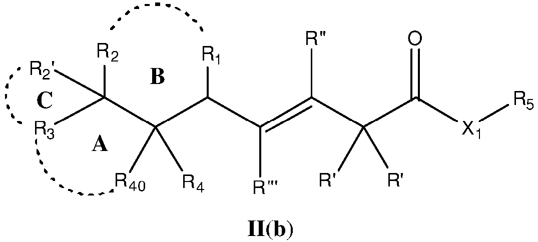 wherein
**R', R"** and **R‴** are each independently selected from a halogen (e.g., F) or a C₁-C₅ linear of branched alkyl (e.g., methyl);
**R₁** is H, F, Cl, Br, I, C₁-C₅ linear or branched, substituted or unsubstituted alkyl (e.g., methyl, ethyl, propyl, iso-propyl, t-Bu, iso-butyl, pentyl, benzyl);
**R₂** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
   or **R₁** and **R₂** are joined to form a 3-8 membered substituted or unsubstituted, saturated or unsaturated carbocyclic ring **B** (e.g., cyclohexyl, cyclohexenyl, cyclopentyl);
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
   or **R₃** and **R₂'** are joined to form a 3-8 membered substituted or unsubstituted, cycloalkyl ring **C** (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
   or **R₃** and **R₄₀** are joined together to form a 3-8 membered substituted or unsubstituted, cycloalkyl ring A (e.g., cyclopropyl, cyclobutyl, cyclopentyl, cyclohexyl);
   or **R₄** and **R₄₀** are joined to form a 3-8 membered substituted or unsubstituted, heterocyclic or cycloalkyl ring;
**R₅** is H, C₁-C₅ linear or branched, substituted or unsubstituted alkyl (e.g., methyl, CH₂SH, ethyl, butyl, CH₂-CCH, iso-propyl, CH₂-C(O)-OCH₃), C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, CH₂, NH or N-R₅₀;
**R₈** is [CH₂]ₚ
   wherein **p** is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, i-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**B** ring is absent, or is a 3-8 membered substituted or unsubstituted, saturated or unsaturated carbocyclic ring (e.g., cyclohexyl, cyclohexenyl, cyclopentyl);
**C** ring is absent, or is a 3-8 membered substituted or unsubstituted, cycloalkyl ring (e.g., cyclopropyl);
or its agrochemically acceptable salt, zwitterion (inner salt), stereoisomer, tautomer, hydrate, N-oxide, reverse amide analog, isotopic variant (e.g., deuterated analog), or any combination thereof.

In some embodiments, at least one of **R₃** and **R₄** is NH₂. In some embodiments, at least one of **A, B** and **C** rings is not absent. In some embodiments, one of **R₃** and **R₄** is NH₂ and the other one is OH.

In some embodiments, the compound is any one of the compounds listed in **Table 1.** In various embodiments, the compound is an herbicidal compound. In various embodiments, the compound is for use in controlling the growth of undesired plants. In various embodiments, the compound is a pesticide. In various embodiments, the compound is a pesticidal compound.

In various embodiments, the compound of formula **II(b)** is represented by any one of the following structures:

| **Compound Number** | **Structure** |
|---|---|
| **101** | 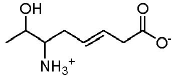 |
| **106** | 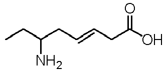 |
| **107** | 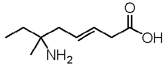 |
| **111** | 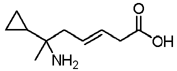 |
| **112** | 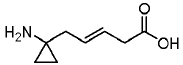 |
| **113** | 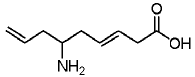 |
| **114** | 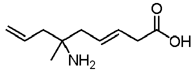 |
| **124** | 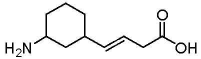 |
| **125** | 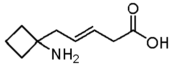 |
| **126** | 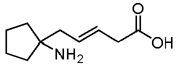 |
| **127** | 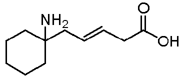 |
| **134** | 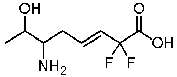 |
| **136** | 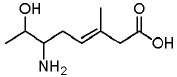 |
| **137** | 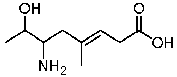 |
| **138** | 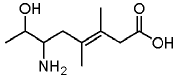 |
| **146** | 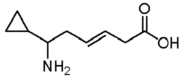 |
| **150** | 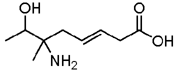 |
| **151** | 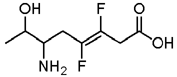 |
| **157** | 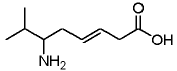 |
| **161** | 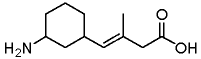 |
| **162** | 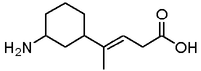 |
| **163** | 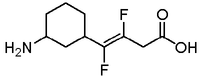 |
| **166** | 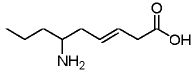 |
| **169** | 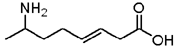 |
| **173** | 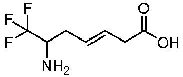 |
| **174** | 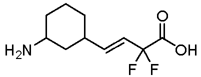 |
| **175** | 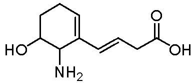 |
| **176** | 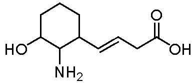 |
| **177** | 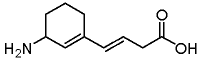 |
| **178** | 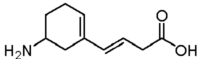 |

In various embodiments, this invention is directed to a compound represented by the structure of formula **II**(**c**): 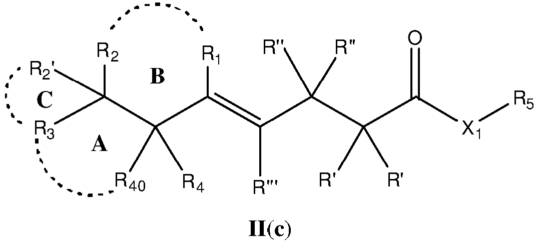 wherein
**R', R"** and **R‴** are each independently selected from a halogen (e.g., F) or a C₁-C₅ linear of branched alkyl (e.g., methyl);
**R₁** is H, F, Cl, Br, I, C₁-C₅ linear or branched, substituted or unsubstituted alkyl (e.g., methyl, ethyl, propyl, iso-propyl, t-Bu, iso-butyl, pentyl, benzyl);
**R₂** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
   or **R₁** and **R₂** are joined to form a 3-8 membered substituted or unsubstituted, saturated or unsaturated carbocyclic ring **B** (e.g., cyclohexyl, cyclohexenyl, cyclopentyl);
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
   or **R₃** and **R₂'** are joined to form a 3-8 membered substituted or unsubstituted, cycloalkyl ring **C** (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
   or **R₃** and **R₄₀** are joined together to form a 3-8 membered substituted or unsubstituted, cycloalkyl ring **A** (e.g., cyclopropyl, cyclobutyl, cyclopentyl, cyclohexyl);
   or **R₄** and **R₄₀** are joined to form a 3-8 membered substituted or unsubstituted, heterocyclic or cycloalkyl ring;
**R₅** is H, C₁-C₅ linear or branched, substituted or unsubstituted alkyl (e.g., methyl, CH₂SH, ethyl, butyl, CH₂-CCH, iso-propyl, CH₂-C(O)-OCH₃), C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, CH₂, NH or N-R₅₀;
**R₈** is [CH₂]ₚ
   wherein **p** is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, *i*-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**B** ring is absent, or is a 3-8 membered substituted or unsubstituted, saturated or unsaturated carbocyclic ring (e.g., cyclohexyl, cyclohexenyl, cyclopentyl);
**C** ring is absent, or is a 3-8 membered substituted or unsubstituted, cycloalkyl ring (e.g., cyclopropyl);
or its agrochemically acceptable salt, zwitterion (inner salt), stereoisomer, tautomer, hydrate, N-oxide, reverse amide analog, isotopic variant (e.g., deuterated analog), or any combination thereof.

In some embodiments, at least one of **R₃** and **R₄** is NH₂. In some embodiments, at least one of **A, B** and **C** rings is not absent. In some embodiments, one of **R₃** and **R₄** is NH₂ and the other one is OH.

In some embodiments, the compound is not (*E*)-7-aminooct-4-enoic acid. In some embodiments, the compound is not 7-aminooct-4-enoic acid.

In some embodiments, the compound is any one of the compounds listed in **Table 1.** In various embodiments, the compound is an herbicidal compound. In various embodiments, the compound is for use in controlling the growth of undesired plants. In various embodiments, the compound is a pesticide. In various embodiments, the compound is a pesticidal compound.

In various embodiments, the compound of formula **II**(**c**) is represented by any one of the following structures:

| **Compound Number** | **Structure** |
|---|---|
| **115** | 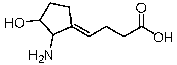 |
| **116** | 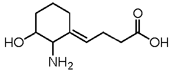 |
| **119** | 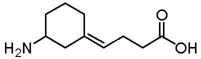 |
| **128** | 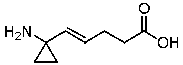 |
| **129** | 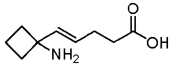 |
| **130** | 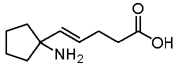 |
| **131** | 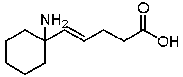 |
| **135** | 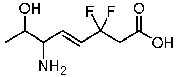 |
| **142** | 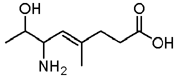 |
| **143** | 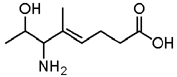 |
| **144** | 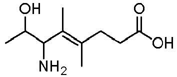 |
| **145** | 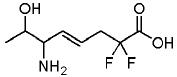 |
| **153** | 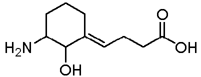 |
| **164** | 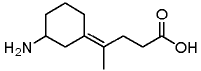 |
| **165** | 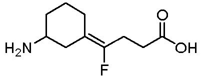 |
| **171** | 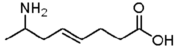 |
| **172** | 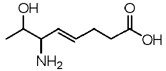 |

In various embodiments, this invention is directed to a compound represented by the structure of formula **II**(**d**): 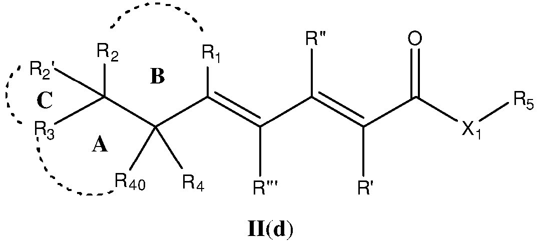 wherein
**R', R"** and **R‴** are each independently selected from a halogen (e.g., F) or a C₁-C₅ linear of branched alkyl (e.g., methyl);
**R₁** is H, F, Cl, Br, I, C₁-C₅ linear or branched, substituted or unsubstituted alkyl (e.g., methyl, ethyl, propyl, iso-propyl, t-Bu, iso-butyl, pentyl, benzyl);
**R₂** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
   or **R₁** and **R₂** are joined to form a 3-8 membered substituted or unsubstituted, saturated or unsaturated carbocyclic ring **B** (e.g., cyclohexyl, cyclohexenyl, cyclopentyl);
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
   or **R₃** and **R₂'** are joined to form a 3-8 membered substituted or unsubstituted, cycloalkyl ring **C** (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
   or **R₃** and **R₄₀** are joined together to form a 3-8 membered substituted or unsubstituted, cycloalkyl ring **A** (e.g., cyclopropyl, cyclobutyl, cyclopentyl, cyclohexyl);
   or **R₄** and **R₄₀** are joined to form a 3-8 membered substituted or unsubstituted, heterocyclic or cycloalkyl ring;
**R₅** is H, C₁-C₅ linear or branched, substituted or unsubstituted alkyl (e.g., methyl, CH₂SH, ethyl, butyl, CH₂-CCH, iso-propyl, CH₂-C(O)-OCH₃), C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, CH₂, NH or N-R₅₀;
**R₈** is [CH₂]ₚ
   wherein **p** is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, i-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**B** ring is absent, or is a 3-8 membered substituted or unsubstituted, saturated or unsaturated carbocyclic ring (e.g., cyclohexyl, cyclohexenyl, cyclopentyl);
**C** ring is absent, or is a 3-8 membered substituted or unsubstituted, cycloalkyl ring (e.g., cyclopropyl);
or its agrochemically acceptable salt, zwitterion (inner salt), stereoisomer, tautomer, hydrate, N-oxide, reverse amide analog, isotopic variant (e.g., deuterated analog), or any combination thereof.

In some embodiments, at least one of **R₃** and **R₄** is NH₂. In some embodiments, at least one of **A, B** and **C** rings is not absent. In some embodiments, one of **R₃** and **R₄** is NH₂ and the other one is OH.

In some embodiments, the compound is any one of the compounds listed in **Table 1.** In various embodiments, the compound is an herbicidal compound. In various embodiments, the compound is for use in controlling the growth of undesired plants. In various embodiments, the compound is a pesticide. In various embodiments, the compound is a pesticidal compound.

In various embodiments, the compound of formula **II**(**d**) is represented by any one of the following structures:

| **Compound Number** | **Structure** |
|---|---|
| **110** | 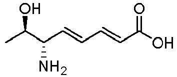 |
| **155** | 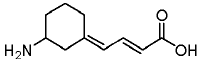 |

In various embodiments, this invention is directed to a compound represented by the structure of formula **III**(**a**): 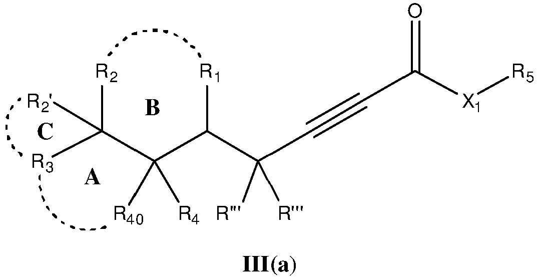 wherein
**R‴** is a halogen (e.g., F) or a C₁-C₅ linear of branched alkyl (e.g., methyl);
**R₁** is H, F, Cl, Br, I, C₁-C₅ linear or branched, substituted or unsubstituted alkyl (e.g., methyl, ethyl, propyl, iso-propyl, t-Bu, iso-butyl, pentyl, benzyl);
**R₂** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
   or **R₁** and **R₂** are joined to form a 3-8 membered substituted or unsubstituted, saturated or unsaturated carbocyclic ring **B** (e.g., cyclohexyl, cyclohexenyl, cyclopentyl);
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
   or **R₃** and **R₂'** are joined to form a 3-8 membered substituted or unsubstituted, cycloalkyl ring **C** (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
   or **R₃** and **R₄₀** are joined together to form a 3-8 membered substituted or unsubstituted, cycloalkyl ring **A** (e.g., cyclopropyl, cyclobutyl, cyclopentyl, cyclohexyl);
   or **R₄** and **R₄₀** are joined to form a 3-8 membered substituted or unsubstituted, heterocyclic or cycloalkyl ring;
**R₅** is H, C₁-C₅ linear or branched, substituted or unsubstituted alkyl (e.g., methyl, CH₂SH, ethyl, butyl, CH₂-CCH, iso-propyl, CH₂-C(O)-OCH₃), C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, CH₂, NH or N-R₅₀;
**R₈** is [CH₂]ₚ
   wherein **p** is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, i-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**B** ring is absent, or is a 3-8 membered substituted or unsubstituted, saturated or unsaturated carbocyclic ring (e.g., cyclohexyl, cyclohexenyl, cyclopentyl);
**C** ring is absent, or is a 3-8 membered substituted or unsubstituted, cycloalkyl ring (e.g., cyclopropyl);
or its agrochemically acceptable salt, zwitterion (inner salt), stereoisomer, tautomer, hydrate, N-oxide, reverse amide analog, isotopic variant (e.g., deuterated analog), or any combination thereof.

In some embodiments, at least one of **R₃** and **R₄** is NH₂. In some embodiments, at least one of **A, B** and **C** rings is not absent. In some embodiments, one of **R₃** and **R₄** is NH₂ and the other one is OH.

In some embodiments, the compound is any one of the compounds listed in **Table 1.** In various embodiments, the compound is an herbicidal compound. In various embodiments, the compound is for use in controlling the growth of undesired plants. In various embodiments, the compound is a pesticide. In various embodiments, the compound is a pesticidal compound.

In various embodiments, the compound of formula **III(a)** is represented by any one of the following structures:

| **Compound Number** | **Structure** |
|---|---|
| **105** | 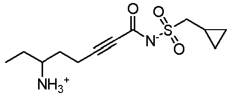 |
| **132** | 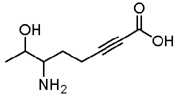 |
| **167** | 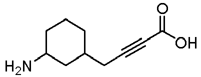 |

In various embodiments, this invention is directed to a compound represented by the structure of formula **III**(**b**): 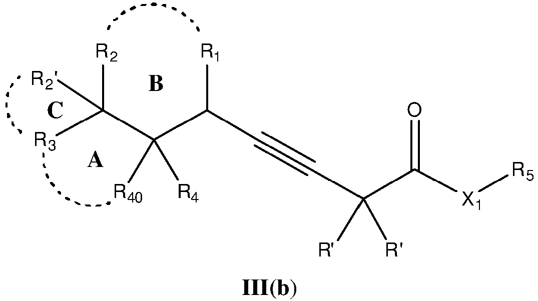 wherein
**R'** is a halogen (e.g., F) or a C₁-C₅ linear of branched alkyl (e.g., methyl);
**R₁** is H, F, Cl, Br, I, C₁-C₅ linear or branched, substituted or unsubstituted alkyl (e.g., methyl, ethyl, propyl, iso-propyl, t-Bu, iso-butyl, pentyl, benzyl);
**R₂** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
   or **R₁** and **R₂** are joined to form a 3-8 membered substituted or unsubstituted, saturated or unsaturated carbocyclic ring **B** (e.g., cyclohexyl, cyclohexenyl, cyclopentyl);
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
   or **R₃** and **R₂'** are joined to form a 3-8 membered substituted or unsubstituted, cycloalkyl ring **C** (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
   or **R₃** and **R₄₀** are joined together to form a 3-8 membered substituted or unsubstituted, cycloalkyl ring **A** (e.g., cyclopropyl, cyclobutyl, cyclopentyl, cyclohexyl);
   or **R₄** and **R₄₀** are joined to form a 3-8 membered substituted or unsubstituted, heterocyclic or cycloalkyl ring;
**R₅** is H, C₁-C₅ linear or branched, substituted or unsubstituted alkyl (e.g., methyl, CH₂SH, ethyl, butyl, CH₂-CCH, iso-propyl, CH₂-C(O)-OCH₃), C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, CH₂, NH or N-R₅₀;
**R₈** is [CH₂]ₚ
   wherein **p** is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, i-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**B** ring is absent, or is a 3-8 membered substituted or unsubstituted, saturated or unsaturated carbocyclic ring (e.g., cyclohexyl, cyclohexenyl, cyclopentyl);
**C** ring is absent, or is a 3-8 membered substituted or unsubstituted, cycloalkyl ring (e.g., cyclopropyl);
or its agrochemically acceptable salt, zwitterion (inner salt), stereoisomer, tautomer, hydrate, N-oxide, reverse amide analog, isotopic variant (e.g., deuterated analog), or any combination thereof.

In some embodiments, at least one of **R₃** and **R₄** is NH₂. In some embodiments, at least one of **A, B** and **C** rings is not absent. In some embodiments, one of **R₃** and **R₄** is NH₂ and the other one is OH.

In some embodiments, the compound is any one of the compounds listed in **Table 1.** In various embodiments, the compound is an herbicidal compound. In various embodiments, the compound is for use in controlling the growth of undesired plants. In various embodiments, the compound is a pesticide. In various embodiments, the compound is a pesticidal compound.

In various embodiments, the compound of formula **III(b)** is represented by any one of the following structures:

| **Compound Number** | **Structure** |
|---|---|
| **100** | 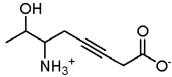 |
| **102** | 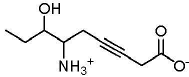 |
| **103** | 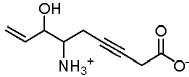 |
| **104** | 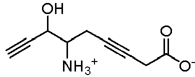 |
| **108** | 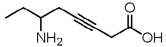 |
| **109** | 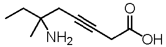 |
| **149** | 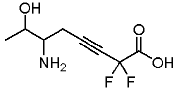 |
| **154** | 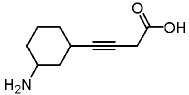 |
| **168** | 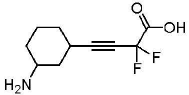 |

In various embodiments, this invention is directed to a compound represented by the structure of formula **III**(**c**): 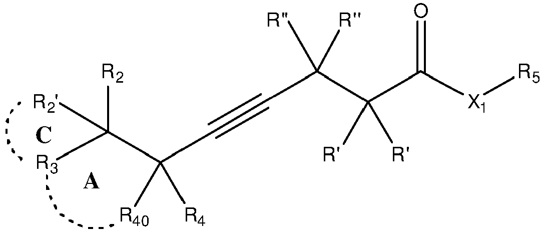 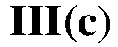 wherein
**R'** and **R"** are each independently selected from a halogen (e.g., F) or a C₁-C₅ linear of branched alkyl (e.g., methyl);
**R₂** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
   or **R₃** and **R₂'** are joined to form a 3-8 membered substituted or unsubstituted, cycloalkyl ring **C** (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
   or **R₃** and **R₄₀** are joined together to form a 3-8 membered substituted or unsubstituted, cycloalkyl ring **A** (e.g., cyclopropyl, cyclobutyl, cyclopentyl, cyclohexyl);
   or **R₄** and **R₄₀** are joined to form a 3-8 membered substituted or unsubstituted, heterocyclic or cycloalkyl ring;
**R₅** is H, C₁-C₅ linear or branched, substituted or unsubstituted alkyl (e.g., methyl, CH₂SH, ethyl, butyl, CH₂-CCH, iso-propyl, CH₂-C(O)-OCH₃), C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, CH₂, NH or N-R₅₀;
**R₈** is [CH₂]ₚ
   wherein **p** is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, i-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**C** ring is absent, or is a 3-8 membered substituted or unsubstituted, cycloalkyl ring (e.g., cyclopropyl);
or its agrochemically acceptable salt, zwitterion (inner salt), stereoisomer, tautomer, hydrate, N-oxide, reverse amide analog, isotopic variant (e.g., deuterated analog), or any combination thereof.

In some embodiments, at least one of **R₃** and **R₄** is NH₂. In some embodiments, at least one of **A, B** and **C** rings is not absent. In some embodiments, one of **R₃** and **R₄** is NH₂ and the other one is OH.

In some embodiments, the compound is any one of the compounds listed in **Table 1.** In various embodiments, the compound is an herbicidal compound. In various embodiments, the compound is for use in controlling the growth of undesired plants. In various embodiments, the compound is a pesticide. In various embodiments, the compound is a pesticidal compound.

In various embodiments, the compound of formula **III(c)** is represented by any one of the following structures:

| **Compound Number** | **Structure** |
|---|---|
| **133** | 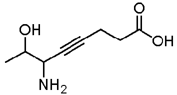 |
| **147** | 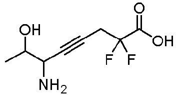 |
| **148** | 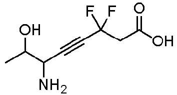 |

In various embodiments, the compound is an herbicidal compound. In various embodiments, the compound is for use in controlling the growth of undesired plants. In various embodiments, the compound is a pesticide. In various embodiments, the compound is a pesticidal compound.

In various embodiments, this invention is directed to a compound represented by any one of the structures presented in Table 1 above. In various embodiments, the compound is an herbicidal compound. In various embodiments, the compound is for use in controlling the growth of undesired plants. In various embodiments, the compound is a pesticide. In various embodiments, the compound is a pesticidal compound.

In various embodiments, this invention is directed to a pesticidal and/or an herbicidal compound and/or to a use of a compound represented by any one of the structures presented in Table 1, or an agrochemical composition thereof, in controlling the growth of undesired plants.

In various embodiments, ring **A** of compound of formula **I, II(a-d)** and **III(a-c)** is a 3-8 membered substituted or unsubstituted, cycloalkyl. In various embodiments, ring **A** is absent. In various embodiments, ring **A** is an unsubstituted 3-8 membered cycloalkyl. In various embodiments, ring **A** is a substituted 3-8 membered cycloalkyl. In various embodiments, ring **A** is a cyclopropyl. In various embodiments, ring **A** is a cyclobutyl. In various embodiments, ring **A** is a cyclopentyl. In various embodiments, ring **A** is a cyclohexyl.

In various embodiments, ring **B** of compound of formula **I, II(a-d)** and **III(a-b)** is a 3-8 membered, substituted or unsubstituted, saturated or unsaturated, carbocyclic ring. In various embodiments, ring **B** is absent. In various embodiments, ring **B** is a 3-8 membered, substituted saturated ring. In various embodiments, ring **B** is a 3-8 membered, substituted cycloalkyl. In various embodiments, ring **B** is a 3-8 membered, unsubstituted saturated ring. In various embodiments, ring **B** is a cyclohexyl. In various embodiments, ring **B** is a 3-8 membered, unsubstituted cycloalkyl. In various embodiments, ring **B** is a 3-8 membered, substituted unsaturated ring. In various embodiments, ring **B** is a 3-8 membered, substituted cycloalkenyl. In various embodiments, ring **B** is a 3-8 membered, unsubstituted unsaturated ring. In various embodiments, ring **B** is a 3-8 membered, unsubstituted cycloalkenyl. In various embodiments, ring **B** is a substituted or unsubstituted cyclohexenyl. In various embodiments, ring **B** is a substituted or unsubstituted cyclopentyl. In various embodiments, ring **B** is an amine substituted cycloalkyl. In various embodiments, ring **B** is an amine substituted cycloalkenyl. In various embodiments, ring **B** is substituted with **R₄, R₄₀, R₂'** and **R₃**. In various embodiments, ring **B** is substituted with **R₄, R₄₀, R₂'** and **R₃** wherein at least one of **R₃** and **R₄** is an amine (e.g., NH₂). In various embodiments, ring **B** is represented by formula (**B**): 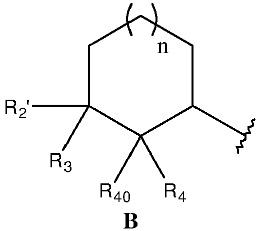 wherein n is 0, 1 or 2.

It is understood that the wigly line in ring **B** above, represents the connection point of ring B to the rest of the molecule.

In various embodiments, ring **C** of compound of formula **I, II(a-d)** and **III(a-c)** is a 3-8 membered substituted or unsubstituted, cycloalkyl. In various embodiments, ring **C** is absent. In various embodiments, ring **C** is an unsubstituted cyclopropyl. In various embodiments, ring **C** is a substituted cyclopropyl. In various embodiments, ring **C** is a cyclobutyl, cyclopentyl, cyclohexyl; each represents a separate embodiment according to this invention.

In some embodiments, at least one of **A, B** and **C** rings is not absent.

In some embodiments, **C_{I}** of formula (I**)** is a substituted or unsubstituted carbon center. In some embodiments, **C_{I}** is an unsubstituted carbon center, and R₁ is H. In some embodiments, **C_{I}** is a substituted carbon center, and R₁ is as defined below. In some embodiments, **C_{I}** is a substituted carbon center, and R₁ is **R₂₀** as defined below. In some embodiments, **C_{I}** is an sp, sp² or sp³ carbon atom depending on whether it is a part of a triple, double, or a single bond respectively. In some embodiments, **C_{I}** of formula **I** is an sp carbon atom; in such case **R₁** is absent. In some embodiments, **C_{I}** is C. In some embodiments, **C_{I}** is an sp² carbon atom. In some embodiments, **C_{I}** is CH. In some embodiments, **C_{I}** is C(**R₂₀**). In some embodiments, **C_{I}** is C-R₁. In some embodiments, **C_{I}** is an sp³ carbon atom. In some embodiments, **C_{I}** is CH₂. In some embodiments, **C_{I}** is CH(**R₂₀**). In some embodiments, **C_{I}** is CH-R₁. In some embodiments, **C_{I}** is C(**R₂₀**)₂.

In some embodiments, **C_{II}** of formula (**I**) is a substituted or unsubstituted carbon center. In some embodiments, **C_{II}** is an unsubstituted carbon center. In some embodiments, **C_{II}** is a substituted carbon center. In some embodiments, **C_{II}** is an sp, sp² or sp³ carbon atom depending on whether it is a part of a triple, double, or a single bond respectively. In some embodiments, **C_{II}** of formula **I** is an sp carbon atom. In some embodiments, **C_{II}** is C. In some embodiments, **C_{II}** is an sp² carbon atom. In some embodiments, **C_{II}** is CH. In some embodiments, **C_{II}** is C(**R₂₀**). In some embodiments, **C_{II}** is an sp³ carbon atom. In some embodiments, **C_{II}** is CH₂. In some embodiments, **C_{II}** is CH(**R₂₀**). In some embodiments, **C_{II}** is C(**R₂₀** )₂.

In some embodiments, **C_{III}** of formula (**I**) is a substituted or unsubstituted carbon center. In some embodiments, **C_{III}** is an unsubstituted carbon center. In some embodiments, **C_{III}** is a substituted carbon center. In some embodiments, **C_{III}** is an sp, sp² or sp³ carbon atom depending on whether it is a part of a triple, double, or a single bond respectively. In some embodiments, **C_{III}** of formula **I** is an sp carbon atom. In some embodiments, **C_{III}** is C. In some embodiments, **C_{III}** is an sp² carbon atom. In some embodiments, **C_{III}** is CH. In some embodiments, **C_{III}** is C(**R₂₀**). In some embodiments, **C_{III}** is an sp³ carbon atom. In some embodiments, **C_{III}** is CH₂. In some embodiments, **C_{III}** is CH(**R₂₀**). In some embodiments, **C_{III}** is C(**R₂₀**)₂.

In some embodiments, **C_{IV}** of formula (**I**) is a substituted or unsubstituted carbon center. In some embodiments, **C_{IV}** is an unsubstituted carbon center. In some embodiments, **C_{IV}** is a substituted carbon center. In some embodiments, **C_{IV}** is an sp, sp² or sp³ carbon atom depending on whether it is a part of a triple, double, or a single bond respectively. In some embodiments, **C_{IV}** of formula **I** is an sp carbon atom. In some embodiments, **C_{IV}** is C. In some embodiments, **C_{IV}** is an sp² carbon atom. In some embodiments, **C_{IV}** is CH. In some embodiments, **C_{IV}** is C(**R₂₀**). In some embodiments, **C_{IV}** is an sp³ carbon atom. In some embodiments, **C_{IV}** is CH₂. In some embodiments, **C_{IV}** is CH(**R₂₀**). In some embodiments, **C_{IV}** is C(**R₂₀**)₂.

In some embodiments, **R₂₀** of formula (**I**) is a halogen. In some embodiments, **R₂₀** is F. In some embodiments, **R₂₀** is Cl. In some embodiments, **R₂₀** is Br. In some embodiments, **R₂₀** is I. In some embodiments, **R₂₀** is a C₁-C₅ linear of branched alkyl. In some embodiments, **R₂₀** is a methyl. In some embodiments, **R₂₀** is an ethyl. In some embodiments, **R₂₀** is a propyl. In some embodiments, **R₂₀** is an iso-propyl. In some embodiments, **R₂₀** is a butyl. In other embodiments, **R₂₀** is not H. In some embodiments, **R₂₀** is halogen or C₁-C₅ linear of branched alkyl. In some embodiments, **R₂₀** is F or methyl.

In some embodiments, R of formula **(I), II(a-d)** and/or **III(a-c)** is a C₁-C₅ linear of branched alkyl. In some embodiments, R is a methyl. In some embodiments, R is an ethyl. In some embodiments, R is a propyl. In some embodiments, R is an iso-propyl. In some embodiments, R is a butyl. In some embodiments, R is C₁-C₅ linear or branched alkoxy. In other embodiments, R is methoxy. In some embodiments, R is phenyl. In some embodiments, R is aryl. In some embodiments, R is heteroaryl. In some embodiments, two gem R substituents are joined together to form a 5 or 6 membered heterocyclic ring. In other embodiments, **R** is C₁-C₅ linear or branched alkyl, C₁-C₅ linear or branched alkoxy, phenyl, aryl or heteroaryl. In other embodiments, **R** is not **H.**

In some embodiments, **C_{I}---C_{II}**, **C_{II}---C_{III}**, and **C_{III}---C_{IV}** of compound of formula (**I**) are each independently a single bond or a double bond, wherein at least one of **C_{I}---C_{II}**, **C_{II}---C_{III}**, and **C_{III}---C_{IV}** is a double bond. In some embodiments, **C_{I}---C_{II}** is a single bond. In some embodiments, **C_{I}---C_{II}** is a double bond. In some embodiments, **C_{II}---C_{III}** is a single bond. In some embodiments, **C_{II}---C_{III}** is a double bond. In some embodiments, **C_{III}---C_{IV}** is a single bond. In some embodiments, **C_{III}---C_{IV}** is a double bond. In some embodiments, **C_{I}---C_{II}**, **C_{II}---C_{III},** and **C_{III}---C_{IV}** are each independently a single bond or a triple bond, wherein at least one of **C_{I}---C_{II}**, **C_{II}---C_{III},** and **C_{III}---C_{IV}** is a triple bond. In some embodiments, **C_{I}---C_{II}** is a triple bond. In some embodiments, if **C_{I}---C_{II}** is a triple bond then **R₁** is absent. In some embodiments, **C_{II}---C_{III}** is a triple bond. In some embodiments, **C_{III}---C_{IV}** is a triple bond.

In some embodiments, **R₁** of compound of formula **I, II(a-d)** and/or **III(a-b)** is H. In some embodiments, **R₁** is F. In some embodiments, **R₁** is Cl. In some embodiments, **R₁** is Br. In some embodiments, **R₁** is I. In some embodiments, **R₁** is a substituted or unsubstituted C₁-C₅ linear or branched alkyl. In some embodiments, **R₁** is methyl. In some embodiments, **R₁** is ethyl. In some embodiments, **R₁** is propyl. In some embodiments, **R₁** is iso-propyl. In some embodiments, **R₁** is t-Bu. In some embodiments, **R₁** is iso-butyl. In some embodiments, **R₁** is pentyl. In some embodiments, **R₁** is benzyl. In some embodiments, if **C_{I}---C_{II}** is a triple bond then **R₁** is absent. In some embodiments **R₁** may be further substituted with at least one substitution selected from: F, Cl, Br, I, OH, SH, C₁-C₅ linear or branched alkyl (e.g. methyl, ethyl), C₂-C₅ linear or branched alkenyl, C₂-C₅ linear or branched alkynyl (e.g. CCH), C₃-C₈ cycloalkyl, linear, branched or cyclic alkoxy, COOH, COO(R), NH₂, N(R)₂, CF₃, aryl, phenyl, heteroaryl (e.g., imidazole), C₃-C₈ cycloalkyl (e.g., cyclohexyl), CN and NO₂; each represents a separate embodiment according to this invention.

In some embodiments, **R₂** of compound of formula **I, II(a-d)** and/or **III(a-c)** is H. In some embodiments, **R₂** is F. In some embodiments, **R₂** is Cl. In some embodiments, **R₂** is Br. In some embodiments, **R₂** is I. In some embodiments, **R₂** is a substituted or unsubstituted C₁-C₅ linear or branched alkyl. In some embodiments, **R₂** is methyl. In some embodiments, **R₂** is ethyl. In some embodiments, **R₂** is propyl. In some embodiments, **R₂** is iso-propyl. In some embodiments, **R₂** is t-Bu. In some embodiments, **R₂** is iso-butyl. In some embodiments, **R₂** is pentyl. In some embodiments, **R₂** is a substituted or unsubstituted C₂-C₅ linear or branched alkenyl. In some embodiments, **R₂** is ethenyl. In some embodiments, **R₂** is CH=CH₂. In some embodiments, **R₂** is a substituted or unsubstituted C₂-C₅ linear or branched alkynyl. In some embodiments, **R₂** is ethynyl. In some embodiments, **R₂** is CCH. In some embodiments R₂ may be further substituted with at least one substitution selected from: F, Cl, Br, I, OH, SH, C₁-C₅ linear or branched alkyl (e.g. methyl, ethyl), C₂-C₅ linear or branched alkenyl, C₂-C₅ linear or branched alkynyl (e.g. CCH), C₃-C₈ cycloalkyl, linear, branched or cyclic alkoxy, COOH, COO(R), NH₂, N(R)₂, CF₃, aryl, phenyl, heteroaryl (e.g., imidazole), C₃-C₈ cycloalkyl (e.g., cyclohexyl), CN and NO₂; each represents a separate embodiment according to this invention.

In some embodiments, **R₁** and **R₂** of formula **I, II(a-d)** and/or **III(a-b)** are joined to form ring **B** as defined above. In some embodiments, **R₁** and **R₂** are joined to form a substituted or unsubstituted, saturated or unsaturated, 3-8 membered carbocyclic ring. In some embodiments, **R₁** and **R₂** are joined to form a substituted, saturated, 3-8 membered carbocyclic ring. In some embodiments, **R₁** and **R₂** are joined to form a substituted, unsaturated, 3-8 membered carbocyclic ring. In some embodiments, **R₁** and **R₂** are joined to form an unsubstituted, saturated, 3-8 membered carbocyclic ring. In some embodiments, **R₁** and **R₂** are joined to form an unsubstituted, unsaturated, 3-8 membered carbocyclic ring. In some embodiments, **R₁** and **R₂** are joined to form a cyclohexyl. In some embodiments, **R₁** and **R₂** are joined to form a cyclohexenyl. In some embodiments, **R₁** and **R₂** are joined to form a cyclopentyl. In some embodiments, **R₁** and **R₂** are joined to form a cyclopropyl. In some embodiments, **R₁** and **R₂** are joined to form a cyclobutyl. In some embodiments, **R₁** and **R₂** are joined to form a cycloheptyl.

In some embodiments, **R₂'** of compound of formula **I, II(a-d)** and/or **III(a-c)** is H. In some embodiments, **R₂'** is F. In some embodiments, **R₂'** is Cl. In some embodiments, **R₂'** is Br. In some embodiments, **R₂'** is I. In some embodiments, **R₂'** is a substituted or unsubstituted C₁-C₅ linear or branched alkyl. In some embodiments, **R₂'** is methyl. In some embodiments, **R₂'** is ethyl. In some embodiments, **R₂'** is propyl. In some embodiments, **R₂'** is iso-propyl. In some embodiments, **R₂'** is t-Bu. In some embodiments, **R₂'** is iso-butyl. In some embodiments, **R₂'** is pentyl. In some embodiments, **R₂'** is a substituted or unsubstituted C₂-C₅ linear or branched alkenyl. In some embodiments, **R₂'** is ethenyl. In some embodiments, **R₂'** is CH=CH₂. In some embodiments, **R₂'** is a substituted or unsubstituted C₂-C₅ linear or branched alkynyl. In some embodiments, **R₂'** is ethynyl. In some embodiments, **R₂'** is CCH. In some embodiments **R₂'** may be further substituted with at least one substitution selected from: F, Cl, Br, I, OH, SH, C₁-C₅ linear or branched alkyl (e.g. methyl, ethyl), C₂-C₅ linear or branched alkenyl, C₂-C₅ linear or branched alkynyl (e.g. CCH), C₃-C₈ cycloalkyl, linear, branched or cyclic alkoxy, COOH, COO(R), NH₂, N(R)₂, CF₃, aryl, phenyl, heteroaryl (e.g., imidazole), C₃-C₈ cycloalkyl (e.g., cyclohexyl), CN and NO₂; each represents a separate embodiment according to this invention.

In some embodiments, **R₃** of compound of formula **I, II(a-d)** and/or **III(a-c)** is H. In some embodiments, **R₃** is F. In some embodiments, **R₃** is Cl. In some embodiments, **R₃** is Br. In some embodiments, **R₃** is I. In some embodiments, **R₃** is OH. In some embodiments, **R₃** is SH. In some embodiments, **R₃** is NH₂. In some embodiments **R₃** is NHR. In some embodiments, **R₃** is N(R)₂. In some embodiments, at least one of **R₃** and **R₄** is NH₂, NH(R) or N(R)₂. In some embodiments, at least one of **R₃** and **R₄** is NH₂.

In some embodiments, **R₃** and **R₂'** of compound of formula **I, II(a-d)** and/or **III(a-c)** are joined to form ring C as defined hereinabove. In some embodiments, **R₃** and **R₂'** are joined to form a substituted or unsubstituted 3-8 membered cycloalkyl. In some embodiments, **R₃** and **R₂'** are joined to form an unsubstituted 3-8 membered cycloalkyl. In some embodiments, **R₃** and **R₂'** are joined to form a cyclopropyl. In some embodiments, **R₃** and **R₂'** are joined to form a cyclobutyl, cyclopentyl, cyclohexyl, or cycloheptyl; each represents a separate embodiment according to this invention. In some embodiments, **R₃** and **R₂'** are joined to form a substituted 3-8 membered cycloalkyl.

In some embodiments, **R₄** of compound of formula **I, II(a-d)** and/or **III(a-c)** is H. In some embodiments, **R₄** is F. In some embodiments, **R₄** is Cl. In some embodiments, **R₄** is Br. In some embodiments, **R₄** is I. In some embodiments, **R₄** is OH. In some embodiments, **R₄** is SH. In some embodiments, **R₄** is NH₂. In some embodiments, **R₄** is NHR. In some embodiments, **R₄** is N(R)₂. In some embodiments, at least one of **R₃** and **R₄** is NH₂, NH(R) or N(R)₂. In some embodiments, at least one of **R₃** and **R₄** is NH₂.

In some embodiments, one of **R₃** and **R₄** is NH₂ and the other one is OH. In some embodiments, **R₃** is NH₂ and **R₄** is OH. In some embodiments, **R₄** is NH₂ and **R₃** is OH. In some embodiments, **R₃** is NH₂ and **R₄** is OH; or **R₄** is NH₂ and **R₃** is OH.

In some embodiments, **R₄₀** of compound of formula **I, II(a-d)** and/or **III(a-c)** is H. In some embodiments, **R₄₀** is a C₁-C₅ linear or branched, substituted or unsubstituted alkyl. In some embodiments, **R₄₀** is a C₁-C₅ linear, unsubstituted alkyl. In some embodiments, **R₄₀** is a C₁-C₅ linear, substituted alkyl. In some embodiments, **R₄₀** is a C₁-C₅ branched, substituted alkyl. In some embodiments, **R₄₀** is a C₁-C₅ branched, unsubstituted alkyl. In some embodiments, **R₄₀** is methyl. In some embodiments, **R₄₀** is ethyl. In some embodiments, **R₄₀** is propyl. In some embodiments, **R₄₀** is iso-propyl. In some embodiments, **R₄₀** is t-Bu. In some embodiments, **R₄₀** is iso-butyl. In some embodiments, **R₄₀** is pentyl. In some embodiments **R₄₀** may be further substituted with at least one substitution selected from: F, Cl, Br, I, OH, SH, C₁-C₅ linear or branched alkyl (e.g. methyl, ethyl), C₂-C₅ linear or branched alkenyl, C₂-C₅ linear or branched alkynyl (e.g. CCH), C₃-C₈ cycloalkyl, linear, branched or cyclic alkoxy, COOH, COO(R), NH₂, N(R)₂, CF₃, aryl, phenyl, heteroaryl (e.g., imidazole), C₃-C₈ cycloalkyl (e.g., cyclohexyl), CN and NO₂; each represents a separate embodiment according to this invention.

In some embodiments, **R₃** and **R₄₀** of compound of formula **I, II(a-d)** and/or **III(a-c)** are joined together to form ring A as described hereinabove. In some embodiments, **R₃** and **R₄₀** are joined together to form a substituted or unsubstituted 3-8 membered cycloalkyl. In some embodiments, **R₃** and **R₄₀** are joined together to form an unsubstituted 3-8 membered cycloalkyl. In some embodiments, **R₃** and **R₄₀** are joined together to form a substituted 3-8 membered cycloalkyl. In some embodiments, **R₃** and **R₄₀** are joined together to form a cyclopropyl. In some embodiments, **R₃** and **R₄₀** are joined together to form a cyclobutyl. In some embodiments, **R₃** and **R₄₀** are joined together to form a cyclopentyl. In some embodiments, **R₃** and **R₄₀** are joined together to form a cyclohexyl.

In some embodiments, **R₄** and **R₄₀** of compound of formula **I, II(a-d)** and/or **III(a-c)** are joined together to form a substituted or unsubstituted 3-8 membered heterocyclic ring. In some embodiments, **R₄** and **R₄₀** are joined to form a substituted or unsubstituted 3-8 membered cycloalkyl. In some embodiments, **R₄** and **R₄₀** are joined to form an unsubstituted 3-8 membered cycloalkyl. In some embodiments, **R₄** and **R₄₀** are joined to form a substituted 3-8 membered cycloalkyl. In some embodiments, **R₄** and **R₄₀** are joined to form a cyclopropyl. In some embodiments, **R₄** and **R₄₀** are joined to form a cyclobutyl. In some embodiments, **R₄** and **R₄₀** are joined to form a cyclopentyl. In some embodiments, **R₄** and **R₄₀** are joined to form a cyclohexyl.

In some embodiment, **R₅** of compound of formula **I, II(a-d)** and/or **III(a-c)** is H, C₁-C₅ linear or branched, substituted or unsubstituted alkyl (e.g., methyl, CH₂SH, ethyl, butyl, CH₂-CCH, iso-propyl, CH₂-C(O)-OCH₃), C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine); each represents a separate embodiment according to this invention. In some embodiments, R₅ is H. In some embodiments, R₅ is C₁-C₅ linear or branched, substituted or unsubstituted alkyl. In some embodiments, R₅ is methyl, ethyl, iso-propyl, butyl, CH₂-CCH, CH₂-C(O)-OCH₃; each represents a separate embodiment according to this invention. In some embodiments, R₅ is C₂-C₅ linear or branched, substituted or unsubstituted alkenyl. In some embodiments, R₅ is C₂-C₅ linear or branched, substituted or unsubstituted alkynyl. In some embodiments, R₅ is CCH. In some embodiments, R₅ is CH₂-CCH. In some embodiments, R₅ is C₁-C₅ linear or branched haloalkyl. In some embodiments, R₅ is substituted or unsubstituted alkyl sulfone. In some embodiments, R₅ is SO₂-CH₂-cyclopentyl. In some embodiments, R₅ is SO₂-CH₂-cyclopropyl. In some embodiments, R₅ is a substituted or unsubstituted aryl. In some embodiments, R₅ is a substituted or unsubstituted heteroaryl. In some embodiments, R₅ is pyridine. In some embodiments, R₅ may be further substituted with at least one substitution selected from: F, Cl, Br, I, OH, SH, C₁-C₅ linear or branched alkyl, C₂-C₅ linear or branched alkenyl, C₂-C₅ linear or branched alkynyl (e.g. CCH), C₃-C₈ cycloalkyl, linear, branched or cyclic alkoxy, COOH, COO(R), NH₂, N(R)₂, CF₃, aryl, phenyl, heteroaryl, C₃-C₈ cycloalkyl, CN and NO₂; each represents a separate embodiment according to this invention.

In some embodiments, **X₁** of formula **I, II(a-d)** and/or **III(a-c)** is O. In other embodiments, **X₁** is CH₂. In other embodiments, **X₁** is NH. In other embodiments, **X₁** is N-R₅₀.

In some embodiments, **R₈** of formula **I, II(a-d)** and/or **III(a-c)** is CH₂. In other embodiments, R₈ is CH₂CH₂. In other embodiments, R₈ is CH₂CH₂CH₂.

In some embodiments, **p** of formula **I, II(a-d)** and/or **III(a-c)** is 1. In other embodiments, p is 2. In other embodiments, **p** is 3.

In some embodiments, **R₁₀** of formula **I, II(a-d)** and/or **III(a-c)** is H. In other embodiments, **R₁₀** is H, CN, C₁-C₅ linear or branched alkyl, methyl, ethyl, C(O)R, C(O)(OCH₃) or S(O)₂R; each represents a separate embodiment according to this invention. In other embodiments, **R₁₀** is C₁-C₅ linear or branched alkyl. In other embodiments, **R₁₀** is CH₃. In other embodiments, **R₁₀** is CH₂CH₃. In other embodiments, **R₁₀** is CH₂CH₂CH₃. In other embodiments, **R₁₀** is CN. In other embodiments, **R₁₀** is C(O)R. In other embodiments, **R₁₀** is C(O)(OCH₃). In other embodiments, **R₁₀** is S(O)₂R.

In some embodiments, **R'** of formula **II(a-d)** and/or **III(b-c)** is a halogen. In some embodiments, **R'** is F. In some embodiments, **R'** is Cl. In some embodiments, **R'** is Br. In some embodiments, **R'** is I. In some embodiments, **R'** is a C₁-C₅ linear of branched alkyl. In some embodiments, **R'** is methyl. In some embodiments, **R'** is ethyl. In some embodiments, **R'** is propyl. In some embodiments, **R'** is iso-propyl. In some embodiments, **R'** is butyl. In some embodiments, **R'** is tert-butyl. In some embodiments, **R'** is pentyl. In some embodiments, **R'** is iso-pentyl.

In some embodiments, **R"** of formula **II(a-d)** and/or **III(c)** is a halogen. In some embodiments, **R"** is F. In some embodiments, **R"** is Cl. In some embodiments, **R"** is Br. In some embodiments, **R"** is I. In some embodiments, **R"** is a C₁-C₅ linear of branched alkyl. In some embodiments, **R"** is methyl. In some embodiments, **R"** is ethyl. In some embodiments, **R"** is propyl. In some embodiments, **R"** is iso-propyl. In some embodiments, **R"** is butyl. In some embodiments, **R"** is tert-butyl. In some embodiments, **R"** is pentyl. In some embodiments, **R"** is iso-pentyl.

In some embodiments, **R‴** of formula **II(a-d)** and/or **III(a)** is a halogen. In some embodiments, **R‴** is F. In some embodiments, **R‴** is Cl. In some embodiments, **R‴** is Br. In some embodiments, **R‴** is I. In some embodiments, **R‴** is a C₁-C₅ linear of branched alkyl. In some embodiments, **R‴** is methyl. In some embodiments, **R‴** is ethyl. In some embodiments, **R‴** is propyl. In some embodiments, **R‴** is iso-propyl. In some embodiments, **R‴** is butyl. In some embodiments, **R‴** is tert-butyl. In some embodiments, **R‴** is pentyl. In some embodiments, **R‴** is iso-pentyl.

In some embodiments, **R₅₀** of formula **I, II(a-d)** and/or **III(a-c)** is H. In some embodiments, **R₅₀** is C₁-C₅ linear or branched, substituted or unsubstituted alkyl. In some embodiments, **R₅₀** is methyl. In some embodiments, **R₅₀** is ethyl. In some embodiments, **R₅₀** is butyl. In some embodiments, **R₅₀** is i-propyl. In some embodiments, **R₅₀** can be further substituted with at least one substitution selected from: F, Cl, Br, I, OH, SH, C₁-C₅ linear or branched alkyl, C₂-C₅ linear or branched alkenyl, C₂-C₅ linear or branched alkynyl (e.g. CCH), C₃-C₈ cycloalkyl, linear, branched or cyclic alkoxy, COOH, COO(R), NH₂, N(R)₂, CF₃, aryl, phenyl, heteroaryl, C₃-C₈ cycloalkyl, CN and NO₂; each represents a separate embodiment according to this invention.

In some embodiments, **n** of formula **B** is 0. In other embodiments, **n** is 1. In other embodiments, **n** is 2.

In various embodiments, this invention is directed to any one of the compounds presented in Table 1 as described herein above, agrochemical compositions and/or method of use thereof in controlling the growth of undesired plants.

In various embodiments, this invention is directed to the use of any one of the compounds presented in Table 2 as described herein above, and/or agrochemical compositions thereof, in controlling the growth of undesired plants.

It is well understood that in structures presented in this invention wherein the carbon atom has less than 4 bonds, H atoms are present to complete the valence of the carbon. It is well understood that in structures presented in this invention wherein the nitrogen atom has less than 3 bonds, H atoms are present to complete the valence of the nitrogen.

In some embodiments, this invention is directed to the compounds listed hereinabove, agrochemical compositions and/or method of use thereof, wherein the compound is agrochemically acceptable salt, zwitterion (inner salt), stereoisomer, optical isomer, tautomer, hydrate, N-oxide, reverse amide analog, isotopic variant (deuterated analog), or any combination thereof. In some embodiments, the compounds are herbicides. In some embodiments, the compounds are pesticides. In some embodiments, the compounds control the growth of undesired plants.

In various embodiments, the compound according to the invention comprises a substantially pure stereoisomer. By substantially pure, it is intended that a stereoisomer is at least about 90% pure, more preferably at least about 95% pure, even more preferably at least about 98% pure, most preferably at least about 99% pure. In various embodiments, the compound comprises a single stereoisomer in a purity of >80%; >85%; >90%; >91%; >92%; >93%; >94%; >95%; >96%; >97%; >98%; >99%; >99.5% enantiomeric excess (*ee*); each represents a separate embodiment according to this invention. In various embodiments, the compound comprises a single stereoisomer in a purity >80%; >85%; >90%; >91%; >92%; >93%; >94%; >95%; >96%; >97%; >98%; >99%; >99.5% enantiomeric ratio (*er*); each represents a separate embodiment according to this invention. In various embodiments, the compound comprises a single stereoisomer in a purity higher than 80%; 85%; 90%; 91%; 92%; 93%; 94%; 95%; 96%; 97%; 98%; 99%; 99.5%; each represents a separate embodiment according to this invention.

In various embodiments, the compound is a substantially pure single enantiomer. In various embodiments, the compound comprises a mixture of stereoisomers. In various embodiments, the compound comprises a mixture of enantiomers. In various embodiments, the compound is a racemate.

In various embodiments, the compound has two chiral centers. In various embodiments, the compound comprises a mixture of stereoisomers. In various embodiments, the compound comprises a mixture of 2, 3, or 4 stereoisomers; each represents a separate embodiment according to this invention. In various embodiments, the compound is a single stereoisomer. In various embodiments, the compound is a substantially pure single stereoisomer. In various embodiments, the substantially pure stereoisomer is compound **110** as described herein below. In various embodiments, the substantially pure stereoisomer has at least 80%, 85%, 90%, 95%, 97%, 98%, 99% purity; each represents a separate embodiment according to this invention. In various embodiments, the compound is the substantially pure ***RR*** stereoisomer. In various embodiments, the compound is the substantially pure ***SS*** stereoisomer. In various embodiments, the compound is the substantially pure ***RS*** stereoisomer. In various embodiments, the compound is the substantially pure ***SR*** stereoisomer.

As used herein, the term "alkyl" can be any linear- or branched-chain alkyl group containing up to about 30 carbons unless otherwise specified. In various embodiments, an alkyl includes C₁-C₅ carbons. In some embodiments, an alkyl includes C₁-C₆ carbons. In some embodiments, an alkyl includes C₁-C₈ carbons. In some embodiments, an alkyl includes C₂-C₅ carbons. In some embodiments, an alkyl includes C₂-C₈ carbons. In some embodiments, an alkyl includes C₁-C₁₀ carbons. In some embodiments, an alkyl is a C₁-C₁₂ carbons. In some embodiments, an alkyl is a C₁-C₂₀ carbons. In some embodiments, branched alkyl is an alkyl substituted by alkyl side chains of 1 to 5 carbons. In various embodiments, the alkyl group may be unsubstituted. In some embodiments, the alkyl group may be substituted by a halogen, haloalkyl, hydroxyl, alkoxy, carbonyl, amido, alkylamido, dialkylamido, cyano, nitro, CO₂H, amino, alkylamino, dialkylamino, carboxyl, thio, thioalkyl, C₁-C₅ linear or branched haloalkoxy, CF₃, phenyl, halophenyl, (benzyloxy)phenyl, -CH₂CN, NH₂, NH-alkyl, N(alkyl)₂, - OC(O)CF₃, -OCH₂Ph, -NHC(O)-alkyl, -C(O)Ph, C(O)O-alkyl, C(O)H, -C(O)NH₂ or any combination thereof.

The alkyl group can be a sole substituent, or it can be a component of a larger substituent, such as in an alkoxy, alkoxyalkyl, haloalkyl, arylalkyl, alkylamino, dialkylamino, alkylamido, alkylurea, etc. Preferred alkyl groups are methyl, ethyl, and propyl, and thus halomethyl, dihalomethyl, trihalomethyl, haloethyl, dihaloethyl, trihaloethyl, halopropyl, dihalopropyl, trihalopropyl, methoxy, ethoxy, propoxy, arylmethyl, arylethyl, arylpropyl, methylamino, ethylamino, propylamino, dimethylamino, diethylamino, methylamido, acetamido, propylamido, halomethylamido, haloethylamido, halopropylamido, methyl-urea, ethyl-urea, propyl-urea, 2, 3, or 4-CH₂-C₆H₄-Cl, C(OH)(CH₃)(Ph), etc.

As used herein, the term "alkenyl" can be any linear- or branched-chain alkenyl group containing up to about 30 carbons as defined hereinabove for the term "alkyl" and at least one carbon-carbon double bond. Accordingly, the term alkenyl as defined herein includes also alkadienes, alkatrienes, alkatetraenes, and so on. In some embodiments, the alkenyl group contains one carbon-carbon double bond. In some embodiments, the alkenyl group contains two, three, four, five, six, seven or eight carbon-carbon double bonds; each represents a separate embodiment according to this invention. Non limiting examples of alkenyl groups include: Ethenyl, Propenyl, Butenyl (i.e., 1-Butenyl, *trans*-2-Butenyl, *cis*-2-Butenyl, and Isobutylenyl), Pentene (i.e., 1-Pentenyl, cis-2-Pentenyl, and *trans*-2-Pentenyl), Hexene (e.g., 1-Hexenyl, (*E*)-2-Hexenyl, (*Z*)-2-Hexenyl, (*E*)-3-Hexenyl, (*Z*)-3-Hexenyl, 2-Methyl-1-Pentene , etc.), which may all be substituted as defined herein above for the term "alkyl".

As used herein, the term "alkynyl" can be any linear- or branched-chain alkynyl group containing up to about 30 carbons as defined hereinabove for the term "alkyl" and at least one carbon-carbon triple bond. Accordingly, the term alkynyl as defined herein includes also alkadiynes, alkatriynes, alkatetraynes, and so on. In some embodiments, the alkynyl group contains one carbon-carbon triple bond. In some embodiments, the alkynyl group contains two, three, four, five, six, seven or eight carbon-carbon triple bonds; each represents a separate embodiment according to this invention. Non limiting examples of alkynyl groups include: acetylenyl, Propynyl, Butynyl (i.e., 1-Butynyl, 2-Butynyl, and Isobutylynyl), Pentyne (i.e., 1-Pentynyl, 2-Pentynyl), Hexyne (e.g., 1-Hexynyl, 2-Hexynyl, 3-Hexynyl, etc.), which may all be substituted as defined herein above for the term "alkyl".

As used herein, the term "aryl" refers to any aromatic ring that is directly bound to another group and can be either substituted or unsubstituted. The aryl group can be a sole substituent, or the aryl group can be a component of a larger substituent, such as in an arylalkyl, arylamino, arylamido, etc. Exemplary aryl groups include, without limitation, phenyl, tolyl, xylyl, furanyl, naphthyl, pyridinyl, pyrimidinyl, pyridazinyl, pyrazinyl, triazinyl, thiazolyl, oxazolyl, isooxazolyl, pyrazolyl, imidazolyl, thiophene-yl, pyrrolyl, indolyl, phenylmethyl, phenylethyl, phenylamino, phenylamido, 3-methyl-4H-1,2,4-triazolyl, 5-methyl-1,2,4-oxadiazolyl, etc. Substitutions include but are not limited to: F, Cl, Br, I, C₁-C₅ linear or branched alkyl, C₁-C₅ linear or branched haloalkyl, C₁-C₅ linear or branched alkoxy, C₁-C₅ linear or branched haloalkoxy, CF₃, phenyl, halophenyl, (benzyloxy)phenyl, CN, NO₂, -CH₂CN, NH₂, NH-alkyl, N(alkyl)₂, hydroxyl, -OC(O)CF₃, -OCH₂Ph, -NHC(O)-alkyl, COOH, -C(O)Ph, C(O)O-alkyl, C(O)H, -C(O)NH₂ or any combination thereof.

As used herein, "heteroaryl" can be any such ring, including but not limited to pyridinyl, (2-, 3-, and 4-pyridinyl), quinolinyl, pyrimidinyl, pyridazinyl, pyrazinyl, triazinyl, tetrazinyl, thiazolyl, isothiazolyl, oxazolyl, isoxazolyl, imidazolyl, 1-methylimidazole, pyrazolyl, pyrrolyl, furanyl, thiophene-yl, quinolinyl, isoquinolinyl, 2,3-dihydroindenyl, indenyl, tetrahydronaphthyl, 3,4-dihydro-2H-benzo[b][1,4]dioxepine , benzodioxolyl, benzo[d][1,3]dioxole, tetrahydronaphthyl, indolyl, 1H-indole, isoindolyl, anthracenyl, benzimidazolyl, 2,3-dihydro-1H-benzo[d]imidazolyl, indazolyl, 2H-indazole, triazolyl, 4,5,6,7-tetrahydro-2H-indazole, 3H-indol-3-one, purinyl, benzoxazolyl, 1,3-benzoxazolyl, benzisoxazolyl, benzothiazolyl, 1,3-benzothiazole, 4,5,6,7-tetrahydro-1,3-benzothiazole, quinazolinyl, quinoxalinyl, 1,2,3,4-tetrahydroquinoxaline, 1-(pyridin-1(2H)-yl)ethanone, cinnolinyl, phthalazinyl, quinolinyl, isoquinolinyl, acridinyl, benzofuranyl, 1-benzofuran, isobenzofuranyl, benzofuran-2(3H)-one, benzothiophenyl, benzoxadiazole, benzo[c][1,2,5]oxadiazolyl, benzo[c]thiophenyl, benzodioxolyl, thiadiazolyl, [1,3]oxazolo[4,5-b]pyridine, oxadiaziolyl, imidazo[2,1-b][1,3]thiazole, 4H,5H,6H-cyclopenta[d][1,3]thiazole, 5H,6H,7H,8H-imidazo[1,2-a]pyridine, 7-oxo-6H,7H-[1,3]thiazolo[4,5-d]pyrimidine, [1,3]thiazolo[5,4-b]pyridine, 2H,3H-imidazo[2,1-b][1,3]thiazole, thieno[3,2-d]pyrimidin-4(3H)-one, 4-oxo-4H-thieno[3,2-d][1,3]thiazin, imidazo[1,2-a]pyridine, 1H-imidazo[4,5-b]pyridine, 1H-imidazo[4,5-c]pyridine, 3H-imidazo[4,5-c]pyridine, pyrazolo[1,5-a]pyridine, imidazo[1,2-a]pyrazine, imidazo[1,2-a]pyrimidine, 1H-pyrrolo[2,3-b]pyridine, pyrido[2,3-b]pyrazine, pyrido[2,3-b]pyrazin-3(4H)-one, 4H-thieno[3,2-b]pyrrole, quinoxalin-2(1H)-one, 1H-pyrrolo[3,2-b]pyridine, 7H-pyrrolo[2,3-d]pyrimidine, oxazolo[5,4-b]pyridine, thiazolo[5,4-b]pyridine, thieno[3,2-c]pyridine, 3-methyl-4H-1,2,4-triazole, 5-methyl-1,2,4-oxadiazole, methyloxazolidin-2-one, etc; each represents a separate embodiment according to this invention.

As used herein, the term "alkoxy" refers to an ether group substituted by an alkyl group as defined above. Alkoxy refers both to linear and to branched alkoxy groups. Nonlimiting examples of alkoxy groups are methoxy, ethoxy, propoxy, *iso*-propoxy, *tert*-butoxy.

A "haloalkyl" group refers, in some embodiments, to an alkyl group as defined above, which is substituted by one or more halogen atoms, e.g. by F, Cl, Br or I. The term "haloalkyl" includes but is not limited to fluoroalkyl, i.e., to an alkyl group bearing at least one fluorine atom. Nonlimiting examples of haloalkyl groups are CF₃, CF₂CF₃, CF₂CH₃, CH₂CF₃, CF₂CH₂CH₃, CH₂CH₂CF₃, CF₂CH(CH₃)₂ and CF(CH₃)-CH(CH₃)₂.

An "halophenyl" group refers, in some embodiments, to a phenyl substituent which is substituted by one or more halogen atoms, e.g. by F, Cl, Br or I. In one embodiment, the halophenyl is 4-chlorophenyl.

An "alkoxyalkyl" group refers, in some embodiments, to an alkyl group as defined above, which is substituted by alkoxy group as defined above, e.g. by methoxy, ethoxy, propoxy, i-propoxy, t-butoxy etc. Nonlimiting examples of alkoxyalkyl groups are -CH₂-O-CH₃, -CH₂-O-CH(CH₃)₂, -CH₂-O-C(CH₃)₃, -CH₂-CH₂-O-CH₃, -CH₂-CH₂-O-CH(CH₃)₂, -CH₂-CH₂-O-C(CH₃)₃.

A "cycloalkyl" or "carbocyclic" group refers, in various embodiments, to a ring structure comprising carbon atoms as ring atoms, which may be either saturated or unsaturated, substituted or unsubstituted, single or fused. In some embodiments the cycloalkyl is a 3-10 membered ring. In some embodiments the cycloalkyl is a 3-12 membered ring. In some embodiments the cycloalkyl is a 6 membered ring. In some embodiments the cycloalkyl is a 5-7 membered ring. In some embodiments the cycloalkyl is a 3-8 membered ring. In some embodiments, the cycloalkyl group may be unsubstituted or substituted by a halogen, alkyl, haloalkyl, hydroxyl, alkoxy, carbonyl, amido, alkylamido, dialkylamido, cyano, nitro, CO₂H, amino, alkylamino, dialkylamino, carboxyl, thio, thioalkyl, C₁-C₅ linear or branched haloalkoxy, CF₃, phenyl, halophenyl, (benzyloxy)phenyl, -CH₂CN, NH₂, NH-alkyl, N(alkyl)₂, -OC(O)CF₃, -OCH₂Ph, - NHC(O)-alkyl, -C(O)Ph, C(O)O-alkyl, C(O)H, -C(O)NH₂ or any combination thereof. In some embodiments, the cycloalkyl ring may be fused to another saturated or unsaturated cycloalkyl or heterocyclic 3-8 membered ring. In some embodiments, the cycloalkyl ring is a saturated ring. In some embodiments, the cycloalkyl ring is an unsaturated ring. Non limiting examples of a cycloalkyl group comprise cyclohexyl, cyclohexenyl, cyclopropyl, cyclopropenyl, cyclopentyl, cyclopentenyl, cyclobutyl, cyclobutenyl, cyclooctyl, cyclooctadienyl (COD), cyclooctaenyl (COE) etc.

An "heterocycle" or "heterocyclic" group refers, in various embodiments, to a ring structure comprising, in addition to carbon atoms, sulfur, oxygen, nitrogen or any combination thereof, as part of the ring. An "heteroaromatic ring" refers in various embodiments, to an aromatic ring structure comprising, in addition to carbon atoms, sulfur, oxygen, nitrogen, selenium or any combination thereof, as part of the ring. In some embodiments the heterocycle or heteroaromatic ring is a 3-10 membered ring. In some embodiments the heterocycle or heteroaromatic ring is a 3-12 membered ring. In some embodiments the heterocycle or heteroaromatic ring is a 6 membered ring. In some embodiments the heterocycle or heteroaromatic ring is a 5-7 membered ring. In some embodiments the heterocycle or heteroaromatic ring is a 3-8 membered ring. In some embodiments, the heterocycle group or heteroaromatic ring may be unsubstituted or substituted by a halogen, alkyl, haloalkyl, hydroxyl, alkoxy, carbonyl, amido, alkylamido, dialkylamido, cyano, nitro, CO₂H, amino, alkylamino, dialkylamino, carboxyl, thiol, thioalkyl, C₁-C₅ linear or branched haloalkoxy, CF₃, phenyl, halophenyl, (benzyloxy)phenyl, -CH₂CN, NH₂, NH-alkyl, N(alkyl)₂, -OC(O)CF₃, -OCH₂Ph, -NHC(O)-alkyl, -C(O)Ph, C(O)O-alkyl, C(O)H, -C(O)NH₂ or any combination thereof. In some embodiments, the heterocycle ring or heteroaromatic ring may be fused to another saturated or unsaturated cycloalkyl or heterocyclic 3-8 membered ring. In some embodiments, the heterocyclic ring is a saturated ring. In some embodiments, the heterocyclic ring is an unsaturated ring. In some embodiments, the heterocyclic ring is an aliphatic ring. Non limiting examples of a heterocyclic ring or heteroaromatic ring systems comprise pyridine, piperidine, morpholine, piperazine, thiophene, pyrrole, benzodioxole, benzofuran-2(3H)-one, benzo[d][1,3]dioxole, indole, oxazole, isoxazole, imidazole and 1-methylimidazole, furane, triazole, pyrimidine, pyrazine, oxacyclobutane (1 or 2-oxacyclobutane), naphthalene, tetrahydrothiophene 1,1-dioxide, thiazole, benzimidazole, piperidine, 1-methylpiperidine, isoquinoline, 1,3-dihydroisobenzofuran, benzofuran, 3-methyl-4H-1,2,4-triazole, 5-methyl-1,2,4-oxadiazole, oxazolidin-2-one, methyloxazolidin-2-one or indole; each is a separate embodiment according to this invention.

In various embodiments, this invention provides a compound of this invention or its agrochemically acceptable salt, zwitterion (inner salt), stereoisomer, optical isomer, tautomer, hydrate, N-oxide, reverse amide analog, isotopic variants (e.g., deuterated analog), or any combination thereof. In various embodiments, this invention provides a single stereoisomer of the compound of this invention. In some embodiments, this invention provides an optical isomer of the compound of this invention. In some embodiments, this invention provides an agrochemically acceptable salt of the compound of this invention. In some embodiments, this invention provides a tautomer of the compound of this invention. In some embodiments, this invention provides a hydrate of the compound of this invention. In some embodiments, this invention provides an N-oxide of the compound of this invention. In some embodiments, this invention provides a reverse amide analog of the compound of this invention. In some embodiments, this invention provides an isotopic variant (including but not limited to deuterated analog) of the compound of this invention. In some embodiments, this invention provides a polymorph of the compound of this invention. In some embodiments, this invention provides a crystal of the compound of this invention. In some embodiments, this invention provides an agrochemical composition comprising a compound of this invention, as described herein, or, in some embodiments, any combination of a stereoisomer, optical isomer, agrochemically acceptable salt, zwitterion (inner salt), tautomer, hydrate, N-oxide, isotopic variant (deuterated analog), polymorph, or crystal of the compound of this invention.

In various embodiments, the term "isomer" includes, but is not limited to, stereoisomers including optical isomers and analogs, structural isomers and analogs, conformational isomers and analogs, and the like. In some embodiments, the isomer is a stereoisomer. In another embodiment, the isomer is an optical isomer.

Certain compounds of the present invention may exist in particular geometric or stereoisomeric forms. The present invention contemplates all such compounds, including *cis-* and *trans*-isomers, *R*- and *S*-enantiomers, diastereomers, the racemic mixtures thereof, and other mixtures thereof, as falling within the scope of the invention. Additional asymmetric carbon atoms may be present in a substituent such as an alkyl group. All such isomers, as well as mixtures thereof, are included in this invention.

In various embodiments, this invention encompasses the use of various stereoisomers of the compounds of the invention. It will be appreciated by those skilled in the art that the compounds of the present invention may contain at least one chiral center. Accordingly, the compounds used in the methods of the present invention may exist in, and be isolated in, optically-active or racemic forms. The compounds according to this invention may further exist as stereoisomers which may be also optically-active isomers (e.g., enantiomers such as (*R*) or (*S*)), as enantiomerically enriched mixtures, racemic mixtures, or as single diastereomers, diastereomeric mixtures, or any other stereoisomers, including but not limited to: *(R)(R), (R)(S), (S)(S), (S)(R), (R)(R)(R), (R)(R)(S), (R)(S)(R), (S)(R)(R), (R)(S)(S), (S)(R)(S), (S)(S)(R)* or *(S)(S)(S)* stereoisomers. Some compounds may also exhibit polymorphism. It is to be understood that the present invention encompasses any racemic, optically-active, polymorphic, or stereroisomeric form, or mixtures thereof, which form possesses properties useful in controlling the growth of various undesired plants as described herein.

It is well known in the art how to prepare optically-active forms (for example, by resolution of the racemic form by recrystallization techniques, by synthesis from optically-active starting materials, by chiral synthesis, or by chromatographic separation using a chiral stationary phase).

The compounds of the present invention can also be present in the form of a racemic mixture, containing substantially equivalent amounts of stereoisomers. In some embodiments, the compounds of the present invention can be prepared or otherwise isolated, using known procedures, to obtain a stereoisomer substantially free of its corresponding stereoisomer (i.e., substantially pure). By substantially pure, it is intended that a stereoisomer is at least about 95% pure, more preferably at least about 98% pure, most preferably at least about 99% pure. In various embodiments, the compound according to the invention comprises a substantially pure stereoisomer. In some embodiments, the substantially pure stereoisomer is at least 70%; 75%; 80%; 85%; 90%; 93%; 95%; 97%; 98%; 99%; 99.5% pure; each represents a separate embodiment according to this invention.

In various embodiments, the compound comprises a single stereoisomer in a purity of >80%; >85%; >90%; >91%; >92%; >93%; >94%; >95%; >96%; >97%; >98%; >99%; >99.5% enantiomeric excess (*ee*); each represents a separate embodiment according to this invention. In various embodiments, the compound comprises a single stereoisomer in a purity >80%; >85%; >90%; >91%; >92%; >93%; >94%; >95%; >96%; >97%; >98%; >99%; >99.5% enantiomeric ratio (*er*); each represents a separate embodiment according to this invention. In various embodiments, the compound comprises a single stereoisomer in a purity higher than 80%; 85%; 90%; 91%; 92%; 93%; 94%; 95%; 96%; 97%; 98%; 99%; 99.5%; each represents a separate embodiment according to this invention. In some embodiments, the compound is compound **110.**

In various embodiments, the compound is a substantially pure single enantiomer. In various embodiments, the compound comprises a mixture of enantiomers. In various embodiments, the compound is a racemate.

In various embodiments, the compound has two chiral centers. In various embodiments, the compound comprises a mixture of stereoisomers. In various embodiments, the compound comprises a mixture of 2, 3, or 4 stereoisomers; each represents a separate embodiment according to this invention. In various embodiments, the compound is a single stereoisomer. In various embodiments, the compound is a substantially pure single stereoisomer. In various embodiments, the substantially pure stereoisomer has at least 80%, 85%, 90%, 95%, 97%, 98%, 99% purity; each represents a separate embodiment according to this invention. In various embodiments, the compound is the substantially pure ***RR*** stereoisomer. In various embodiments, the compound is the substantially pure ***SS*** stereoisomer. In various embodiments, the compound is the substantially pure ***RS*** stereoisomer. In various embodiments, the compound is the substantially pure ***SR*** stereoisomer.

Compounds of the present invention can also be in the form of a hydrate, which means that the compound further includes a stoichiometric or non-stoichiometric amount of water bound by non-covalent intermolecular forces.

As used herein, when some chemical functional group (e.g. alkyl or aryl) is said to be "substituted", it is herein defined that one or more substitutions are possible.

Compounds of the present invention may exist in the form of one or more of the possible tautomers and depending on the conditions it may be possible to separate some or all of the tautomers into individual and distinct entities. It is to be understood that all of the possible tautomers, including all additional enol and keto tautomers and/or isomers are hereby covered. For example, the following tautomers, but not limited to these, are included:
Tautomerization of the imidazole ring 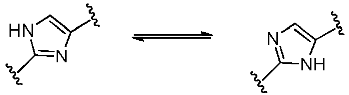
Tautomerization of the pyrazolone ring:

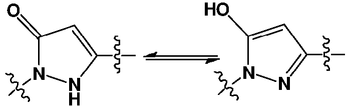

The invention includes "agrochemically acceptable salts" of the compounds of this invention, which may be produced, by reaction of a compound of this invention with an acid or base. Certain compounds, particularly those possessing acidic or basic groups, can also be in the form of a salt, preferably an agrochemically acceptable salt. The term "agrochemically acceptable salt" refers to those salts that retain the agrochemical effectiveness and properties of the free bases or free acids, which are not agrochemically or otherwise undesirable. The salts are formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, *p*-toluenesulfonic acid, salicylic acid, *N*-acetylcysteine and the like. Other salts are known to those of skill in the art and can readily be adapted for use in accordance with the present invention.

Suitable agrochemically-acceptable salts of amines of the compounds of this invention may be prepared from an inorganic acid or from an organic acid. In various embodiments, examples of inorganic salts of amines are bisulfates, borates, bromides, chlorides, hemisulfates, hydrobromates, hydrochlorates, 2-hydroxyethylsulfonates (hydroxyethanesulfonates), iodates, iodides, isothiocyanates, nitrates, persulfates, phosphates, sulfates, sulfamates, sulfanilates, sulfonic acids (alkylsulfonates, arylsulfonates, halogen substituted alkylsulfonates, halogen substituted arylsulfonates), sulfonates and thiocyanates.

In various embodiments, examples of organic salts of amines may be selected from aliphatic, cycloaliphatic, aromatic, araliphatic, heterocyclic, carboxylic and sulfonic classes of organic acids, examples of which are acetates, aspartates, ascorbates, adipates, anthranilates, alginates, alkane carboxylates, substituted alkane carboxylates, alginates, benzenesulfonates, benzoates, bisulfates, butyrates, bicarbonates, bitartrates, citrates, camphorates, camphorsulfonates, cyclohexylsulfamates, cyclopentanepropionates, calcium edetates, camsylates, carbonates, clavulanates, cinnamates, dicarboxylates, digluconates, dodecylsulfonates, dihydrochlorides, decanoates, enanthuates, ethanesulfonates, edetates, edisylates, estolates, esylates, fumarates, formates, fluorides, galacturonates gluconates, glutamates, glycolates, glucorate, glucoheptanoates, glycerophosphates, gluceptates, glycollylarsanilates, glutarates, glutamate, heptanoates, hexanoates, hydroxymaleates, hydroxycarboxlic acids, hexylresorcinates, hydroxybenzoates, hydroxynaphthoates, hydrofluorates, lactates, lactobionates, laurates, malates, maleates, methylenebis(beta-oxynaphthoate), malonates, mandelates, mesylates, methane sulfonates, methylbromides, methylnitrates, methylsulfonates, monopotassium maleates, mucates, monocarboxylates, naphthalenesulfonates, 2-naphthalenesulfonates, nicotinates, nitrates, napsylates, *N*-methylglucamines, oxalates, octanoates, oleates, pamoates, phenylacetates, picrates, phenylbenzoates, pivalates, propionates, phthalates, phenylacetate, pectinates, phenylpropionates, palmitates, pantothenates, polygalacturates, pyruvates, quinates, salicylates, succinates, stearates, sulfanilate, subacetates, tartrates, theophyllineacetates, p-toluenesulfonates (tosylates), trifluoroacetates, terephthalates, tannates, teoclates, trihaloacetates, triethiodide, tricarboxylates, undecanoates and valerates.

In various embodiments, examples of inorganic salts of carboxylic acids or hydroxyls may be selected from ammonium, alkali metals to include lithium, sodium, potassium, cesium; alkaline earth metals to include calcium, magnesium, aluminum; zinc, barium, quaternary ammoniums.

In some embodiments, examples of organic salts of carboxylic acids or hydroxyl may be selected from arginine, organic amines to include aliphatic organic amines, alicyclic organic amines, aromatic organic amines, benzathines, *t*-butylamines, benethamines (*N*-benzylphenethylamine), dicyclohexylamines, dimethylamines, diethanolamines, ethanolamines, ethylenediamines, hydrabamines, imidazoles, lysines, methylamines, meglamines, *N*-methyl-*D*-glucamines, *N,N'-*dibenzylethylenediamines, nicotinamides, organic amines, ornithines, pyridines, picolines, piperazines, procaines, tris(hydroxymethyl)methylamines, triethylamines, triethanolamines, trimethylamines, tromethamines and ureas.

In various embodiments, the salts may be formed by conventional means, such as by reacting the free base or free acid form of the product with one or more equivalents of the appropriate acid or base in a solvent or medium in which the salt is insoluble or in a solvent such as water, which is removed in vacuo or by freeze drying or by exchanging the ions of an existing salt for another ion or suitable ion-exchange resin.

### Agrochemical composition

Another aspect of the present invention relates to an agrochemical composition including an agrochemically acceptable carrier or diluent and a compound according to the aspects of the present invention. The agrochemical composition can contain one or more of the above-identified compounds of the present invention. Typically, the agrochemical composition of the present invention will include a compound of the present invention or its agrochemically acceptable salt, zwitterion (inner salt), as well as an agrochemically acceptable carrier or diluent. The term "agrochemically acceptable carrier" refers to any suitable adjuvants, carriers, excipients, or stabilizers, and can be in solid or liquid form such as sprays, aerosols, powders, solutions, suspensions, or emulsions.

The compounds according to the invention can be used as pesticidal or herbicidal agents in unmodified form, but they are generally formulated into compositions in various ways using formulation adjuvants, such as carriers, solvents and surface-active substances. The formulations can be in various physical forms, e.g. in the form of dusting powders, gels, wettable powders, water-dispersible granules, water- dispersible tablets, effervescent pellets, emulsifiable concentrates, microemulsifiable concentrates, oil- in-water emulsions, oil-flowables, aqueous dispersions, oily dispersions, suspo-emulsions, capsule suspensions, emulsifiable granules, soluble liquids, water-soluble concentrates (with water or a water-miscible organic solvent as carrier), impregnated polymer films or in other forms known. Such formulations can either be used directly or diluted prior to use. The dilutions can be made, for example, with water, liquid fertilizers, micronutrients, biological organisms, oil or solvents.

Typically, the composition will contain from about 0.01 to 99 percent, preferably from about 20 to 75 percent of active compound(s), together with the adjuvants, carriers and/or excipients. While individual needs may vary, determination of optimal ranges of effective amounts of each component is within the skill of the art.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known per se. As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, dimethylformamide (DMF), dimethyl sulfoxide (DMSO), 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, n-hexane, n-octylamine, octadecanoic acid, octylamine acetate, oleic acid, oleylamine, o-xylene, phenol, polyethylene glycol, propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2- ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; as well as further substances known to the skilled in the arts.

Further adjuvants that can be used in pesticidal or herbicidal formulations include crystallization inhibitors, viscosity modifiers, suspending agents, dyes, anti-oxidants, foaming agents, light absorbers, mixing auxiliaries, antifoams, complexing agents, neutralizing or pH-modifying substances and buffers, corrosion inhibitors, fragrances, wetting agents, take-up enhancers, micronutrients, plasticizers, glidants, lubricants, dispersants, thickeners, antifreezes, microbicides, and liquid and solid fertilizers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Other oil derivatives are known to the skilled in the arts, for examples from the Compendium of Herbicide Adjuvants, 10th Edition, Southern Illinois University, 2010.

The pesticidal or herbicidal compositions generally comprise from 0.1 to 99 % by weight, especially from 0.1 to 95 % by weight, compounds according to this invention and from 1 to 99.9 % by weight of a formulation adjuvant which preferably includes from 0 to 25 % by weight of a surface-active substance. The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.

The rates of application vary within wide limits and depend on the nature of the soil, the method of application, the crop plant, the pest to be controlled, the prevailing climatic conditions, and other factors governed by the method of application, the time of application and the target crop. As a general guideline, compounds may be applied at a rate of from 1 to 2000 l/ha, especially from 10 to 1000 l/ha. Preferred formulations can have the following compositions (weight %):
Emulsifiable concentrates:
   - active ingredient: 1 to 95 %, preferably 60 to 90 %
   - surface-active agent: 1 to 30 %, preferably 5 to 20 %
   - liquid carrier: 1 to 80 %, preferably 1 to 35 %
Dusts:
   - active ingredient: 0.1 to 10 %, preferably 0.1 to 5 %
   - solid carrier: 90 to 99.9 %, preferably 99 to 99.9 %
Suspension concentrates:
   - active ingredient: 5 to 75 %, preferably 10 to 50 %
   - water: 24 to 94 %, preferably 30 to 88 %
   - surface-active agent: 1 to 40 %, preferably 2 to 30 %
Wettable powders:
   - active ingredient: 0.5 to 90 %, preferably 1 to 80 %
   - surface-active agent 0.5 to 20 %, preferably 1 to 15 %
   - solid carrier: 5 to 95 %, preferably 15 to 90 %
Granules:
   - active ingredient: 0.1 to 30 %, preferably 0.1 to 15 %
   - solid carrier: 70 to 99.5 %, preferably 85 to 97 %

When the compounds or agrochemical compositions of the present invention are administered to control the growth of undesired plants, the agrochemical composition can also contain, or can be administered in conjunction with, other agrochemical agents or treatment regimen presently known or hereafter developed for the growth control of various types of plants.

Accordingly, the composition of the present invention may further comprise at least one additional pesticide including but not limited to herbicide. For example, the compounds according to the invention can also be used in combination with other pesticides, herbicides or plant growth regulators. In a preferred embodiment the additional pesticide is an herbicide and/or herbicide safener.

Examples of herbicides that can be used in combination with the compounds of the invention, include but are not limited to: acetochlor, acifluorfen (including acifluorfen-sodium), aclonifen, alachlor, alloxydim, ametryn, amicarbazone, amidosulfuron, aminocyclopyrachlor , aminopyralid, amitrole, asulam, atrazine, bensulfuron (including bensulfuron-methyl), bentazone, bicyclopyrone, bilanafos, bifenox, bispyribac-sodium, bixlozone, bromacil, bromoxynil, butachlor, butafenacil, cafenstrole, carfentrazone (including carfentrazone-ethyl); cloransulam (including cloransulam-methyl), chlorimuron (including chlorimuron-ethyl), chlorotoluron, cinosulfuron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, daimuron, desmedipham, dicamba (including the aluminum, aminopropyl, bis-aminopropylmethyl, choline, dichloroprop, diglycolamine, dimethylamine, dimethylammonium, potassium and sodium salts thereof), diclofop-methyl, diclosulam, diflufenican, difenzoquat, diflufenican, diflufenzopyr, dimethachlor, dimethenamid-P, diquat dibromide, diuron, esprocarb, ethalfluralin, ethofumesate, fenoxaprop (including fenoxaprop-P-ethyl), fenoxasulfone, fenquinotrione, fentrazamide, flazasulfuron, florasulam, florpyrauxifen, fluazifop (including fluazifop-P-butyl), flucarbazone (including flucarbazone-sodium);, flufenacet, flumetralin, flumetsulam, flumioxazin, flupyrsulfuron (including flupyrsulfuron-methyl-sodium);, fluroxypyr (including fluroxypyr-meptyl);, fluthiacet-methyl, fomesafen, foramsulfuron, glufosinate (including the ammonium salt thereof), glyphosate (including the diammonium, isopropylammonium and potassium salts thereof), halauxifen (including halauxifen-methyl), halosulfuron-methyl, haloxyfop (including haloxyfop- methyl), hexazinone, hydantocidin, imazamox, imazapic, imazapyr, imazaquin, imazethapyr, indaziflam, iodosulfuron (including iodosulfuron-methyl-sodium), iofensulfuron, iofensulfuron-sodium, ioxynil, ipfencarbazone, isoproturon, isoxaben, isoxaflutole, lactofen, lancotrione, linuron, MCPA, MCPB, mecoprop-P, mefenacet, mesosulfuron, mesosulfuron-methyl, mesotrione, metamitron, metazachlor, methiozolin, metobromuron, metolachlor, metosulam, metoxuron, metribuzin, metsulfuron, molinate, napropamide, nicosulfuron, norflurazon, orthosulfamuron, oxadiargyl, oxadiazon, oxasulfuron, oxyfluorfen, paraquat dichloride, pendimethalin, penoxsulam, phenmedipham, picloram, picolinafen, pinoxaden, pretilachlor, primisulfuron-methyl, prodiamine, prometryn, propachlor, propanil, propaquizafop, propham, propyrisulfuron, propyzamide, prosulfocarb, prosulfuron, pyraclonil, pyraflufen (including pyraflufen-ethyl), pyrasulfotole, pyrazolynate, pyrazosulfuron-ethyl, pyribenzoxim, pyridate, pyriftalid, pyrimisulfan, pyrithiobac-sodium, pyroxasulfone, pyroxsulam , quinclorac, quinmerac, quizalofop (including quizalofop-P-ethyl and quizalofop-P-tefuryl)" rimsulfuron, saflufenacil, sethoxydim, simazine, S-metolachlor, sulcotrione, sulfentrazone, sulfosulfuron, tebuthiuron, tefuryltrione, tembotrione, terbuthylazine, terbutryn, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, tritosulfuron, 4-hydroxy-1-methoxy-5-methyl-3-[4-(trifluoromethyl)-2- pyridyl]imidazolidin-2-one, 4-hydroxy-1 ,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1- methyl-3-[4-(trifluoromethyl)-2-pyridyl]imidazolidin-2-one, 4-hydroxy-1 ,5-dimethyl-3-[1-methyl-5- (trifluoromethyl)pyrazol-3-yl]imidazolidin-2-one, (4R)1-(5-tert-butylisoxazol-3-yl)-4-ethoxy-5-hydroxy- 3-methyl-imidazolidin-2-one, 3-[2-(3,4-dimethoxyphenyl)-6-methyl-3-oxo-pyridazine-4- carbonyl]bicyclo[3.2.1]octane-2,4-dione, 2-[2-(3,4-dimethoxyphenyl)-6-methyl-3-oxo-pyridazine-4- carbonyl]-5-methyl-cyclohexane-1 ,3-dione, 2-[2-(3,4-dimethoxyphenyl)-6-methyl-3-oxo-pyridazine- 4-carbonyl]cyclohexane-1 ,3-dione, 2-[2-(3,4-dimethoxyphenyl)-6-methyl-3-oxo-pyridazine-4- carbonyl]-5,5-dimethyl-cyclohexane-1 ,3-dione, 6-[2-(3,4-dimethoxyphenyl)-6-methyl-3-oxo- pyridazine-4-carbonyl]-2,2,4,4-tetramethyl-cyclohexane-1 ,3,5-trione, 2-[2-(3,4-dimethoxyphenyl)-6- methyl-3-oxo-pyridazine-4-carbonyl]-5-ethyl-cyclohexane-1 ,3-dione, 2-[2-(3,4-dimethoxyphenyl)-6-methyl-3-oxo-pyridazine-4-carbonyl]-4,4,6,6-tetramethyl-cyclohexane-1 ,3-dione, 2-[6-cyclopropyl-2-(3,4-dimethoxyphenyl)-3-oxo-pyridazine-4-carbonyl]-5-methyl-cyclohexane-1 ,3-dione, 3-[6-cyclopropyl-2-(3,4-dimethoxyphenyl)-3-oxo-pyridazine-4-carbonyl]bicyclo[3.2.1]octane-2,4-dione, 2- [6-cyclopropyl-2-(3,4-dimethoxyphenyl)-3-oxo-pyridazine-4-carbonyl]-5,5-dimethyl-cyclohexane-1 ,3-dione, 6-[6-cyclopropyl-2-(3,4-dimethoxyphenyl)-3-oxo-pyridazine-4-carbonyl]-2,2,4,4-tetramethyl-cyclohexane-1 ,3,5-trione, 2-[6-cyclopropyl-2-(3,4-dimethoxyphenyl)-3-oxo-pyridazine-4-carbonyl]cyclohexane-1 ,3-dione, 4-[2-(3,4-dimethoxyphenyl)-6-methyl-3-oxo-pyridazine-4-carbonyl]-2,2,6,6-tetramethyl-tetrahydropyran-3,5-dione and I + 4-[6-cyclopropyl-2-(3,4-dimethoxyphenyl)-3-oxo-pyridazine-4-carbonyl]-2,2,6,6-tetramethyl-tetrahydropyran-3,5-dione. These additional agents may also be present in the form of esters or salts thereof.

Compounds of the present invention may also be combined with herbicide safeners. Example of herbicide safeners include but are not limited to: benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen, N-(2-methoxybenzoyl)-4-[(methylaminocarbonyl)amino] benzenesulfonamide and oxabetrinil; all of which may be in the form of esters or salts thereof.

The compound of the invention can also be used in mixtures with other agrochemicals such as fungicides, nematicides or insecticides, examples of which are known to the skilled in the art.

The mixing ratio of compound of the invention and the additional agent, is preferably from 1:100 to 1000:1. Preferably the mixing ratio of compound of the invention to safener is from 100:1 to 1:10, especially from 20:1 to 1:1.

The mixtures can advantageously be used in the above-mentioned formulations (in which case "active ingredient" relates to the respective mixture of compound of the invention with the additional agent).

### Herbicidal Activity

In various embodiments, the invention provides compounds and compositions, including any embodiment described herein, for use in any of the methods of this invention. In various embodiments, use of a compound of this invention or a composition comprising the same, will have utility in inhibiting, suppressing, enhancing, or stimulating a desired response, as will be understood by one skilled in the art. In some embodiments, the compositions may further comprise additional active ingredients, whose activity is useful for the particular application for which the compound of this invention is being administered.

The compounds of this invention are useful as pesticides and/or herbicides or pesticidal and/or herbicidal compounds. The present invention therefore further comprises a method for controlling the growth of undesired plants, comprising applying to the plants or a locus comprising them, an effective amount of a compound according to this invention, or an agrochemical composition thereof, under conditions effective to control the growth of the undesired plants, in particular the growth of weeds, in crops of useful plants.

In various embodiments, this invention is directed to a method for controlling the growth of undesired plants, comprising applying to the plants or a locus comprising them, an effective amount of a compound according to this invention, or an agrochemical composition thereof, under conditions effective to control the growth of the undesired plants, in particular the growth of weeds, in crops of useful plants.

In some embodiments, "Controlling" according to this invention refers to killing, reducing or retarding growth or preventing or reducing germination. Generally, the plants to be controlled are unwanted plants (weeds).

In some embodiments, "Locus" refers to the area in which the plants are growing or will grow.

The rates of application of compounds of the invention may vary within wide limits and depend on the nature of the soil, the method of application (for example: pre-plant, pre-emergence; post-emergence; application to the seed furrow; no tillage application etc.), the crop plant, the weed(s) to be controlled, the prevailing climatic conditions, and other factors governed by the method of application, the time of application and the target crop. The compounds of the invention are generally applied at a rate of from 10 to 2000 g/ha, especially from 50 to 1000 g/ha.

In some embodiments, the application is made by spraying the composition, typically by tractor mounted sprayer for large areas, but other methods such as dusting (for powders), drip or drench can also be used.

In some embodiments, useful plants in which the composition according to the invention can be used upon include crops such as cereals including but not limited to barley and wheat, cotton, oilseed rape, sunflower, maize, rice, soybeans, sugar beet, sugar cane and turf.

In some embodiments, crop plants also include trees, such as fruit trees, palm trees, coconut trees or other nuts. Also included are vines such as grapes, fruit bushes, fruit plants and vegetables.

In some embodiments, the crops are resistant crops. Therefore, according to some embodiments, crops also include those crops which have been rendered tolerant to herbicides or classes of herbicides (including but not limited to: ALS-, GS-, EPSPS-, PPO-, ACCase- and HPPD-inhibitors) by conventional methods of breeding or by genetic engineering. Examples of crops that have been rendered tolerant to herbicides by genetic engineering methods include but not limited to glyphosate- and glufosinate-resistant maize varieties commercially available under the trade names RoundupReady^{®} and LibertyLink^{®}. According to other embodiments, crops also include those which have been rendered resistant to harmful insects by genetic engineering methods, examples of such crops include but not limited to: Bt maize (resistant to European corn borer), Bt cotton (resistant to cotton boll weevil) and Bt potatoes (resistant to Colorado beetle). Non limiting examples of Bt maize include the Bt 176 maize hybrids of NK^{®} (Syngenta Seeds). Non limiting examples of transgenic plants comprising one or more genes that code for an insecticidal resistance and express one or more toxins are: KnockOut^{®} (maize), Yield Gard^{®} (maize), NuCOTIN33B^{®} (cotton), Bollgard^{®} (cotton), NewLeaf^{®} (potatoes), NatureGard^{®} and Protexcta^{®}. Plant crops or seed material thereof can be both resistant to herbicides and, at the same time, resistant to insect feeding ("stacked" transgenic events).

In some embodiments, crops include those which are obtained by conventional methods of breeding or genetic engineering and contain so-called output traits (e.g. improved storage stability, higher nutritional value and improved flavor). Other useful plants include turf grass for example in golf-courses, lawns, parks and roadsides, or grown commercially for sod, and ornamental plants such as flowers or bushes.

Herbicidal compounds, or chemically active herbicides, may be broken down into pre-plant herbicides, pre-emergent herbicides and post-emergent herbicides. Pre-plant and pre-emergent herbicides typically interfere with germination of weed seeds and are applied before and after planting or sowing, respectively, but before seed germination, whereas post-emergent herbicides kill the weeds after the weed seeds have germinated and weed growth has begun.

When administering the compounds of the present invention, they can be administered in pre-plant or pre-emergence treatments, in post-emergence treatments, or both.

In various embodiments, this invention is directed to a method of controlling the growth of undesired plants, comprising applying a compound according to this invention, or an agrochemical composition thereof, to crop fields. In some embodiments, the compound is a pre-plant herbicide. In some embodiments, the compound is a pre-emergent herbicide. In some embodiments, the compound is a post-emergent herbicide. Therefore, in some embodiment, the compound is applied to crop fields before the undesired plants emerge (i.e. pre-emergent or pre-plant herbicide). In some embodiments, the compound is applied to crop fields after the undesired plants emerge (i.e. post-emergent herbicide).

In various embodiments, this invention is directed to a method of controlling the growth of undesired plants, comprising applying a compound according to this invention, or an agrochemical composition thereof, to fields comprising useful crops. In some embodiments, the compound is a pre-plant herbicide. In some embodiments, the compound is a pre-emergent herbicide. In some embodiments, the compound is a post-emergent herbicide. Therefore, in some embodiment, the compound is applied to crop fields before the undesired plants emerge (i.e. pre-emergent or pre-plant herbicide). In some embodiments, the compound is applied to crop fields after the undesired plants emerge (i.e. post-emergent herbicide).

In various embodiments, compounds according to this invention, and agrochemical compositions thereof, are used to control undesired plants, which include a wide variety of monocotyledonous and dicotyledonous weed species.

In some embodiments, the undesired plant is a weed. In some embodiments, the undesired plant is a eudicot (dicotyledonous or dicot). In some embodiments, the undesired plant is a monocotyledon (monocotyledonous or monocot).

Non limiting examples of monocotyledonous species that can typically be controlled include *Alopecurus myosuroides, Avena fatua, Brachiaria plantaginea, Bromus tectorum, Cyperus esculentus, Digitaria sanguinalis, Echinochloa crus-galli, Lolium perenne, Lolium multiflorum, Panicum miliaceum, Poa annua, Setaria viridis, Setaria faberi* and *Sorghum bicolor;* each represents a separate embodiment according to this invention.

Non limiting examples of dicotyledonous species that can be controlled *include Abutilon theophrasti, Amaranthus retroflexus, Bidens pilosa, Chenopodium album, Euphorbia heterophylla, Galium aparine, Ipomoea hederacea, Kochia scoparia, Polygonum convolvulus, Sida spinosa, Sinapis arvensis, Solanum nigrum, Stellaria media, Veronica persica* and *Xanthium strumarium;* each represents a separate embodiment according to this invention.

In some embodiments, the undesired plant is *Abutilon theophrasti , Amaranthus palmeri, Ambrosia artemisiifolia , Alopecurus myosuroides , Avena sterilis , Chenopodium album , Conyza Canadensis , Digitaria sanguinalis , Echinochloa colona, Euphorbia heterophylla , Lolium perenne , Lolium rigidum , Matricaria chamomilla , Phalaris paradoxa , Poa annua , Portulaca oleracea , Setaria viridis, Solanum nigrum* or any combination thereof. In some embodiments, the compound is any one of the compounds listed in Table 1; each compound represents a separate embodiment according to this invention.

In some embodiments, compounds, and compositions according to this invention are utilized to control undesirable vegetation in rice. In certain embodiments, the undesirable vegetation is *Brachiaria platyphylla* (Groseb.) Nash (broadleaf signalgrass. BRAPP), *Digitaria sanguinalis* (L.) Scop, (large crabgrass, DIGSA), *Echinochloa crus-galli* (L.) P. Beauv. (bamyardgrass, ECHCG), *Echinochloa colonum* (L.) LINK (junglerice, ECHCO), *Echinochloa oryzoides* (Ard.) Fritsch (early watergrass, ECHOR), *Echinochloa oryzicola* (Vasinger) Vasinger (late watergrass, ECHPH), *Ischaemum rugosum* Salisb. (saramollagrass, ISCRU), *Leptochloa chinensis* (L.) Nees (Chinese sprangletop, LEFCH), *Leptochloa fascicularis* (Lam.) Gray (bearded sprangletop, LEFFA), *Leptochloa panicoides* (Presl.) Hitchc. (Amazon sprangletop, LEFPA), *Panicum dichotomiflorum* (L.) Michx. (fall panicum, PANDI), *Paspalum dilatatum* Poir. (dallisgrass, PASDI), *Cyperus difformis* L. (smallflower flatsedge, CYPDI), *Cyperus esculentus* L. (yellow nutsedge, CYPES), *Cyperus iria* L. (rice flatsedge, CYPIR), *Cyperus rotundus* L. (purple nutsedge, CYPRO), *Eleocharis* species (ELOSS), *Fimbristylis miliacea* (L.) Vabl (globe fringerush, FIMMI), *Schoenoplectus juncoides* Roxb. (Japanese bulrush, SCPJU), *Schoenoplectus maritimus* L. (sea clubrush, SCPMA), *Schoenoplectus mucronatus* L. (ricefield bulrush, SCPMU), *Aeschynomene* species, (jointvetch, AESSS), *Alternanthera philoxeroides* (Mart.) Griseb. (alligatorweed, ALRPH), *Alisma plantago-aquatica* L. (common waterplantain, ALSPA), *Amaranthus* species, (pigweeds and amaranths, AMASS), *Ammannia coccinea* Rottb. (redstem, AMMCO), *Eclipta alba* (L.) Hassk. (American false daisy, ECLAL), *Heteranthera limosa* (SW.) Willd./Vahl (ducksalad, HETLI), *Heteranthera reniformis R.* & P. (roundleaf mudplantain, HETRE), *Ipomoea hederacea* (L.) Jacq. (ivyleaf momingglory, IPOHE), *Lindernia dubia* (L.) Pennell (low false pimpernel, LIDDU), *Monochoria korsakowii* Regel & Maack (monochoria, MOOKA), *Monochoria vaginalis* (Burm. F.) C. Presl ex Kuhth, (monochoria, MOOVA), *Murdannia nudifiora* (L.) Brenan (doveweed, MUDNU), *Polygonum pensylvanicum* L. (Pennsylvania smartweed, POLPY), *Polygonum persicaria* L.i (ladysthumb, POLPE), *Polygonum hydropiperoides* Michx. (POLHP, mild smartweed), *Rotala indica* (Willd.) Koehne (Indian toothcup, ROTIN), *Sagittaria* species, (arrowhead, SAGSS), *Sesbania exaltata* (Raf) Cory/Rydb. Ex Hill (hemp sesbania, SEBEX), or *Sphenoclea zeylanica* Gaertn. (gooseweed, SPDZE); each is a separate embodiment according to this invention. In some embodiments, the compound is any one of the compounds listed in Table 1; each compound represents a separate embodiment according to this invention.

In some embodiments, the compounds and compositions according to this invention are utilized to control undesirable vegetation in cereals. In certain embodiments, the undesirable vegetation is *Alopecurus myosuroides* Huds. (blackgrass, ALOMY), *Apera spica-venti* (L.) Beauv. (windgrass, APESV), *Avena fatua* L. (wild oat, AVEFA), *Bromus tectorum* L. (downy brome, BROTE), *Lolium multiflorum* Lam. (Italian ryegrass, LOLMU), *Phalaris minor* Retz. (little seed canarygrass, PHAMI), *Poa annua* L. (annual bluegrass, POAAN), *Setaria pumila* (Poir.) Roemer & J.A. Schultes (yellow foxtail, SETLU), *Setaria viridis* (L.) Beauv. (green foxtail, SETVI), *Cirsium arvense* (L.) Scop. (Canada thistle, CIRARy), *Galium aparine* L. (catchweed bedstraw, GALAP), *Kochia scoparia* (L.) Schrad. (kochia, KCHSC), *Lamium purpureum* L. (purple deadnettle , LAMPU), *Matricaria recutita* L. (wild chamomile, MATCH), *Matricaria matricarioides* (Less.) Porter (pineappleweed, MATMT), *Papaver rhoeas* L. (common poppy, PAPRH), *Polygonum convolvulus* L. (wild buckwheat, POLCO), *Salsola tragus* L. (Russian thistle, SASKR), *Stellaria media* (L.) VilL (common chickweed, STEME), *Veronica persica* Poir. (Persian speedwell, VERPE), *Viola arvensis* Murr. (field violet, VIOAR), or *Viola tricolor* L. (wild violet, VIOTR); each is a separate embodiment according to this invention. In some embodiments, the compound is any one of the compounds listed in Table 1; each compound represents a separate embodiment according to this invention.

In some embodiments, the compounds and compositions according to this invention are utilized to control undesirable vegetation in range and pasture. In certain embodiments, the undesirable vegetation is *Ambrosia artemisiifolia* L. (common ragweed, AMBEL), *Cassia obtusifolia* (sickle pod, CASOB), *Centaurea maculosa* auct. non Lam. (spotted knapweed, CENMA), *Cirsium arvense* (L.) Scop. (Canada thistle, CIRAR), *Convolvulus arvensis* L. (field bindweed, CONAR), *Euphorbia esula* L. (leafy spurge, EPHES), *Lactuca serriola* L./Tom. (prickly lettuce, LACSE), *Plantago lanceolata* L. (buckhom plantain, PLALA), *Rumex obtusifolius* L. (broadleaf dock, RUMOB), *Sida spinosa* L. (prickly sida, SIDSP), *Sinapis arvensis* L. (wild mustard, SINAR), *Sonchus arvensis* L. (perennial sowthistle, SONAR), *Solidago* species (goldenrod, SOOSS), *Taraxacum officinale* G.H. Weber ex Wiggers (dandelion, TAROF), *Trifolium repens* L. (white clover, TRFRE), or *Urtica dioica* L. (common nettle, URTDI); each is a separate embodiment according to this invention. In some embodiments, the compound is any one of the compounds listed in Table 1; each compound represents a separate embodiment according to this invention.

In some embodiments, the compounds and compositions according to this invention are utilized to control undesirable vegetation found in row crops. In certain embodiments, the undesirable vegetation is *Alopecurus myosuroides* Huds. (blackgrass, ALOMY), *Avena fatua* L. (wild oat, AVEFA), *Brachiaria platyphylla* (Groseb.) Nash (broadleaf signalgrass, BRAPP), *Digitaria sanguinalis* (L.) Scop, (large crabgrass, DIGSA), *Echinochloa crus-galli* (L.) P. Beauv. (bamyardgrass, ECHCG), *Echinochloa colonum* (L.) Link (junglerice, ECHCO), *Lolium multiflorum* Lam. (Italian ryegrass, LOLMU), *Panicum dichotomiflorum* Michx. (fall panicum, PANDI), *Panicum miliaceum* L. (wild-proso millet, PANMI), *Setaria faberi* Herrm. (giant foxtail, SETFA), *Setaria viridis* (L.) Beauv. (green foxtail, SETVI), *Sorghum halepense* (L.) Pers. (Johnsongrass, SORHA), *Sorghum bicolor* (L.) Moench ssp. *Arundinaceum* (shattercane, SORVU), *Cyperus esculentus* L. (yellow nutsedge, CYPES), *Cyperus rotundus* L. (purple nutsedge, CYPRO), *Abutilon theophrasti* Medik. (velvetleaf, ABUTH), *Amaranthus* species (pigweeds and amaranths, AMASS), *Ambrosia artemisiifolia* L. (common ragweed, AMBEL), *Ambrosia psilostachya* DC. (western ragweed, AMBPS), *Ambrosia trifida* L. (giant ragweed, AMBTR), *Asclepias syriaca* L. (common milkweed, ASCSY), *Chenopodium album* L. (common lambsquarters, CHEAL), *Cirsium arvense* (L.) Scop. (Canada thistle, CIRAR), *Commelina benghalensis* L. (tropical spiderwort, COMBE), *Datura stramonium* L. (jimsonweed, DATST), *Daucus carota* L. (wild carrot, DAUCA), *Euphorbia heterophylla* L. (wild poinsettia, EPHHL), *Erigeron bonariensis* L. (hairy fleabane, ERIBO), *Erigeron canadensis* L. (Canadian fleabane, ERICA), *Helianthus annuus* L. (common sunflower, HELAN), *Jacquemontia tammfolia* (L.) Griseb. (smallflower momingglory, IAQTA), *Ipomoea hederacea* (L.) Jacq. (ivyleaf momingglory, IPOHE), *Ipomoea lacunosa* L. (white momingglory, IPOLA), *Lactuca serriola* L./Tom. (prickly lettuce, LACSE), *Portulaca oleracea* L. (common purslane, POROL), *Sida spinosa* L. (prickly sida, SIDSP), *Sinapis arvensis* L. (wild mustard, SINAR), *Solanum ptychanthum* Dunal (eastern black nightshade, SOLPT), or *Xanthium strumarium* L. (common cocklebur, XANST); each is a separate embodiment according to this invention. In some embodiments, the compound is any one of the compounds listed in Table 1; each compound represents a separate embodiment according to this invention.

The following examples are presented in order to more fully illustrate the preferred embodiments of the invention. They should in no way, however, be construed as limiting the broad scope of the invention.

### EXAMPLES

### EXAMPLE 1

### Synthetic Details for Compounds of the Invention

### Preparation of compound 101

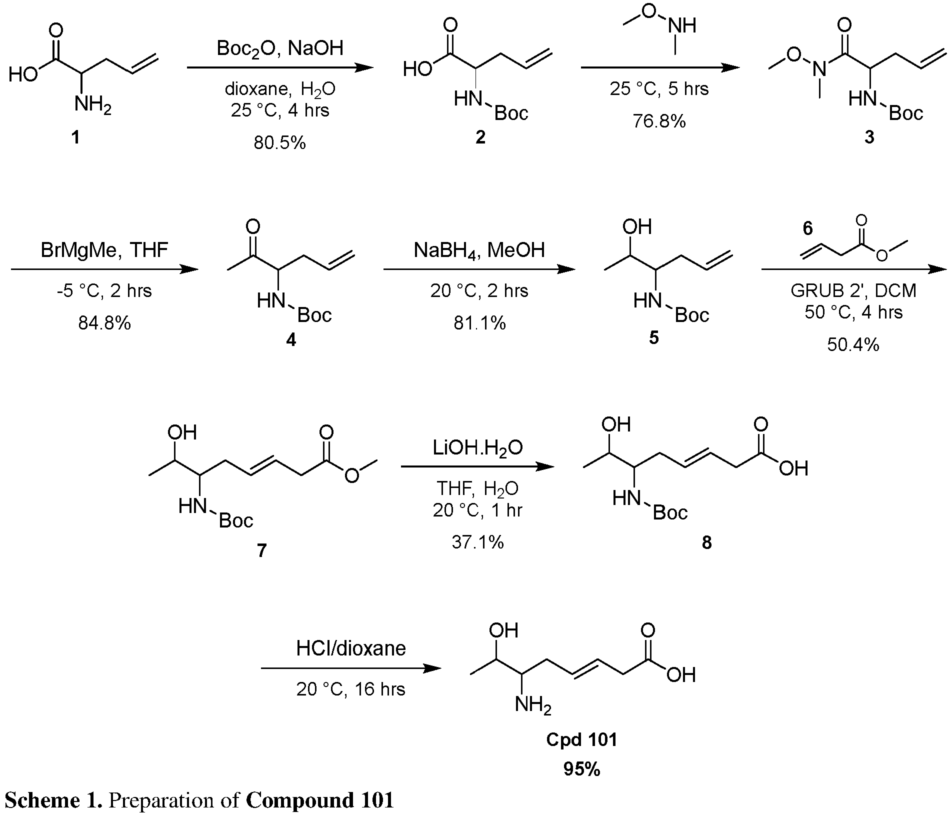

### General procedure for preparation of compound 2 (Scheme 1)

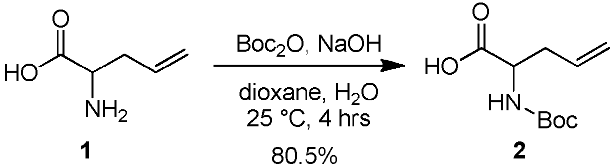

To a solution of compound 1 (255 g, 2.21 mol, 1.00 *eq)* in dioxane (1500 mL) was added NaOH (97.5 g, 2.44 mol, 1.10 *eq)* and H₂O (1500 mL). After 10 mins was added Boc₂O (580 g, 2.66 mol, 1.20 *eq).* The mixture was stirred at 25 °C for 4 hrs. LC-MS showed reactant 1 was consumed completely and one main peak with desired mass was detected. The reaction mixture was diluted with H₂O (1500 mL) and washed with MTBE (1500 mL), organic phase was discarded. The aqueous phase was adjusted to pH 3 with 1 M HCl aqueous, then extracted with EtOAc (2000 mL * 2). The combined organic layers were washed with brine (2000 mL), dried over MgSO₄, filtered and concentrated under reduced pressure to give the compound 2 (384 g, 1.78 mol, 80.5% yield) as a white solid, which was used into the next step without further purification.
¹H NMR (400 MHz, DMSO-*d*₆) *δ* 13.73 - 11.10 (m, 1H), 7.01 (d, *J* = 8.1 Hz, 1H), 5.75 (tdd, *J* = 6.9, 10.1, 17.0 Hz, 1H), 5.17 - 4.96 (m, 2H), 3.92 (dt, *J* = 5.1, 8.5 Hz, 1H), 2.46 - 2.22 (m, 2H), 1.37 (s, 9H)

### General procedure for preparation of compound 3 (Scheme 1)

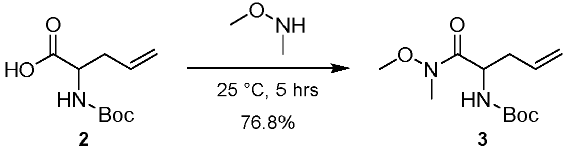

To a solution of compound **2** (380 g, 1.77 mol, 1.00 *eq)* in DCM (4000 mL) was added CDI (372 g, 2.30 mol, 1.30 *eq)* in portion wise. The mixture was stirred at 20 °C for 0.5 hr. Then N-methoxy methylamine hydrochloride (258 g, 2.65 mol, 1.50 *eq)* was added and stirred at 25 °C for 5 hrs. LC-MS showed reactant 2 was consumed completely and one main peak with desired mass was detected. The reaction mixture was diluted with DCM (3000 mL) and washed with 1 M HCl (2000 mL) and saturated NaHCO₃ (2000 mL) and brine (2000 mL), dried over MgSO₄, filtered and concentrated under reduced pressure to give the crude compound **3** (350 g, 1.35 mol, 76.8% yield) as a white solid, which was used into the next step without further purification.
¹H NMR: (400 MHz, DMSO-*d*₆) *δ* 7.00 (br d, J = 8.3 Hz, 1H), 5.82 - 5.67 (m, 1H), 5.14 - 4.97 (m, 2H), 4.54 - 4.25 (m, 1H), 3.72 (s, 3H), 3.09 (s, 3H), 2.33 - 2.17 (m, 2H), 1.36 (s, 9H)

### General procedure for preparation of compound 4 (Scheme 1)

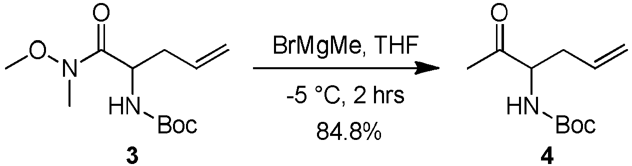

To a solution of compound **3** (120 g, 465 mmol, 1.00 *eq)* in THF (1700 mL) was added BrMgMe (3 M, 619 mL, 4.00 *eq)* at -5 °C. The mixture was stirred at -5 °C for 2 hrs. TLC (Petroleum ether: THF = 10: 1 SM R_{f} = 0.64 product R_{f} = 0.82) indicated compound **3** was consumed completely and one new spot formed. The reaction was clean according to TLC. The reaction solution was poured into ice 10% NH₄Cl (1000 mL), extracted with EtOAc (1000 mL * 3). The combined organic layers were washed with brine (800 mL), dried over MgSO₄, filtered and concentrated under reduced pressure to give a residue. The residue was purified by flash silica gel chromatography (ISCO^{®}; 330+330 g SepaFlash^{®} Silica Flash Column, Eluent of 0-4% Ethyl acetate/Petroleum ether gradient @ 100 mL/min). Compound **4** (84.0 g, 394 mmol, 84.8% yield) was obtained as a colorless oil.
¹H NMR: (400 MHz, DMSO-*d*₆) *δ* 7.13 (br d, *J =* 7.6 Hz, 1H), 5.82 - 5.71 (m, 1H), 5.69 (s, 1H), 5.15 - 4.95 (m, 2H), 2.47 - 2.16 (m, 2H), 2.07 (s. 3H), 1.45 - 1.31 (m, 9H)

### General procedure for preparation of compound 5 (Scheme 1)

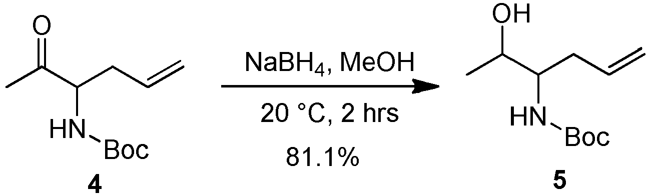

To a solution of compound **4** (113 g, 530 mmol, 1.00 *eq)* in MeOH (1200 mL) was added NaBH₄ (50.1 g, 1.32 mol, 2.50 *eq)* portion-wise at 20 °C. The mixture was stirred at 20 °C for 2 hrs. TLC (Petroleum ether: THF = 3: 1 SM R_{f} = 0.80 product R_{f} = 0.29) indicated reactant **4** was consumed completely and one new spot formed. The reaction mixture was quenched by addition H₂O (1500 mL) at 5 °C, and the reaction mixture was extracted with EtOAc (800 mL * 2). The combined organic layers were washed with brine (600 mL), dried over MgSO₄, filtered and concentrated under reduced pressure to give a residue. The residue was purified by flash silica gel chromatography (ISCO^{®}; 330+330 g SepaFlash^{®} Silica Flash Column, Eluent of 0-18% THF/Petroleum ether gradient @ 100 mL/min). Compound **5** (185 g, 859 mmol, 81.1% yield) was obtained as a white solid.
¹H NMR: (400 MHz, DMSO-*d*₆) *δ* 6.54 - 6.06 (m, 1H), 5.80 - 5.63 (m, 1H), 5.10 - 4.90 (m, 2H), 4.68 - 4.44 (m, 1H), 3.47 - 3.37 (m, 1H), 3.29 - 3.12 (m, 1H), 2.43 - 1.92 (m, 2H), 1.43 - 1.29 (m, 9H), 1.07 - 0.92 (m, 3H)

### General procedure for preparation of compound 7 (Scheme 1)

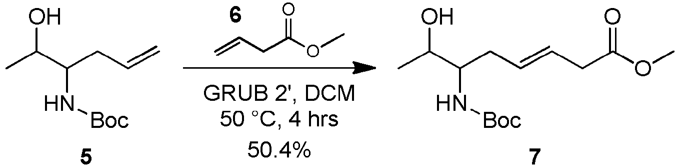

A solution of compound **5** (59.0 g, 274 mmol, 1.00 *eq)* and compound **6** (32.9 g, 329 mmol, *1.20 eq)* in DCM (700 mL) was under reduced pressure for 30 mins and purged with N₂ for 3 times, then GRUB 2' (4.65 g, 5.48 mmol, 0.02 *eq)* was added, the mixture was under reduced pressure for 10 mins and purged with N₂ for 3 times. The mixture was stirred at 50 °C for 4 hrs. TLC (Petroleum ether: Ethyl acetate = 2: 1.5, SM R_{f} = 0.45, Product R_{f} = 0.25) indicated ~15 % of reactant **5** remained, and one major new spot (~40%) with larger polarity was detected. The reaction mixture was filtered and washed with DCM (200 mL * 3), the filtrate was concentrated under reduced pressure to remove solvent. The residue was purified by column chromatography (SiO₂, Ethyl acetate / Petroleum ether = 15% - 21%). Compound 7 (119 g, 414 mmol, 50.4% yield) was obtained as a yellow oil.
¹H NMR: (400 MHz, CDCl₃) *δ* 5.71 - 5.50 (m, 2H), 4.96 - 4.52 (m, 1H), 3.93 - 3.79 (m, 1H), 3.76 - 3.66 (m, 3H), 3.63 (br s, 1H), 3.19 - 3.00 (m, 2H), 2.43 - 2.22 (m, 3H), 1.44 (s, 9H), 1.23 - 1.10 (m, 3H).

### General procedure for preparation of compound 8 (Scheme 1)

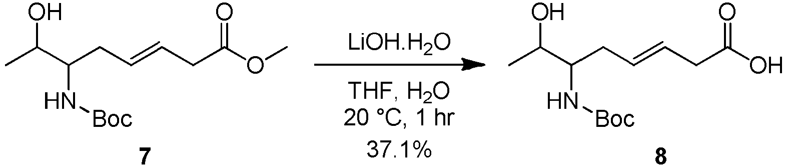

To a solution of compound **7** (119 g, 414 mmol, 1.00 *eq)* in THF (700 mL) and H₂O (700 mL) was added LiOH.H₂O (69.5 g, 1.66 mol, 4.00 *eq).* The mixture was stirred at 20 °C for 1 hr. TLC (Petroleum ether: Ethyl acetate = 1: 1, SM R_{f} = 0.50, Product R_{f} = 0.00) showed reactant was consumed completely. The reaction mixture was diluted with H₂O (800 mL) and extracted with EtOAc (500 mL * 2), organic layer was discarded. The aqueous layer was adjusted to pH 3 with 1 M HCl, and extracted with EtOAc (800 mL * 3). The combined organic layers were washed with brine (800 mL), dried over MgSO₄, filtered and concentrated under reduced pressure to give a residue (~120 g). The crude product was purified by re-crystallization from MTBE (500 mL) at 50 °C, then stirred at 15 °C for 16 hrs, filtered to give 24 g product. The filtrate was concentrated under reduced pressure and purified by re-crystallization from i-Pr₂O (500 mL) at 50 °C, then stirred at 15 °C for 16 hrs, filtered to give 26 g product. The product was combined and triturated with i-Pr₂O (1000 mL) at 15 °C for 1 hr, filtered to give the compound **8** (42.0 g, 154 mmol, 37.1% yield), obtained as a white solid.

¹H NMR: (400 MHz, CDCl₃) *δ* 5.68 - 5.53 (m, 2H), 5.04 - 4.47 (br, 1H), 3.95 - 3.76 (m, 1H), 3.73 - 3.45 (m, 1H), 3.09 (d, *J =* 5.1 Hz, 2H), 2.37 - 2.24 (m, 1H), 2.24 - 2.11 (m, 1H), 1.44 (s, 9H), 1.17 (*J* = 6.4 Hz, 3H).

### General procedure for preparation of compound 101 (Scheme 1)

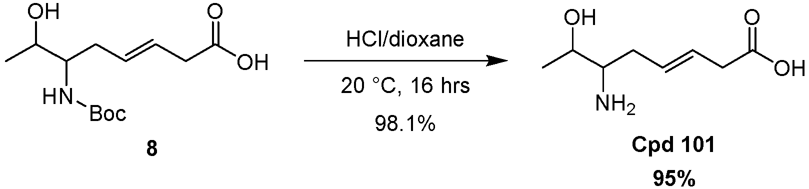

To a solution of compound **8** (40.0 g, 146 mmol, 1.00 *eq)* in dioxane (300 mL) was added HCl/dioxane (4 M, 137 mL, 3.74 *eq).* The mixture was stirred at 20 °C for 16 hrs. The reaction mixture was concentrated under reduced pressure to give a residue. The residue was diluted with pure H₂O (200 mL) and washed with EtOAc (200 mL * 3). The aqueous layers were lyophilized to give the Cpd 101 (31.5 g, 144 mmol, 98.1% yield, 95.6% purity, HCl), obtained as a yellow oil.

¹H NMR: (400 MHz, DMSO-*d*₆) *δ* 13.24 - 11.15 (m, 1H), 7.98 (br s, 3H), 5.74 - 5.57 (m, 1H), 5.57 - 5.43 (m, 1H), 4.99 (br dd, *J* = 3.4, 6.3 Hz, 1H), 3.86 (br dd, *J* = 3.5, 6.3 Hz, 1H), 3.11 - 3.02 (m, 1H), 2.99 (br d, *J* = 6.4 Hz, 2H), 2.37 - 2.20 (m, 2H), 1.08 (br d, *J* = 6.5 Hz, 3H).

Compound 172 was prepared using similar technique as described for compound 101.

### Preparation of compound 110

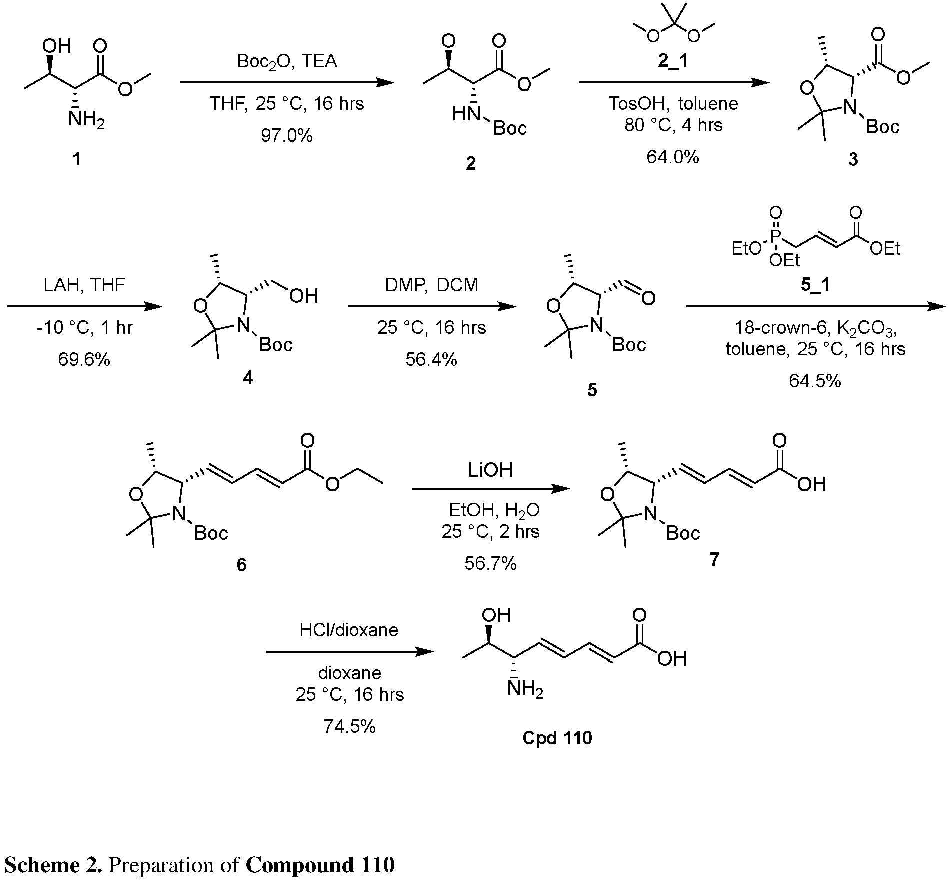

### General procedure for preparation of compound 2 (Scheme 2)

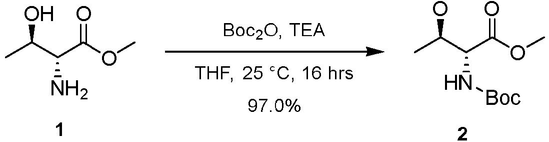

To a solution of compound 1 (50.0 g, 375 mmol) in THF (200 mL) was added Boc₂O (123 g, 563mmol) and TEA (114 g, 1.13 mol). The mixture was stirred at 20 °C for 16 hrs. LC-MS showed reactant **1** was consumed completely and one main peak with desired mass was detected. The reaction mixture was diluted with water (1000 mL) and extracted with EtOAc (1000 mL * 3). The combined organic layers were washed with brine (500 mL), dried over MgSO₄, filtered and concentrated under reduced pressure to give a residue. Compound **2** (85 g, 97.0% yield) was obtained as a colorless oil, which was used in the next step without further purification.
¹H NMR (400 MHz, CHLOROFORM-d) *δ* 5.48 (br s, 1H), 4.35 (br d, *J* = 3.1 Hz, 1H), 4.12 - 4.08 (m, 1H), 3.76 (s, 3H), 3.18 (br d, *J* = 5.5 Hz, 1H), 1.43 (s, 9H), 1.21 - 1.15 (m, 3H)

### General procedure for preparation of compound 3 (Scheme 2)

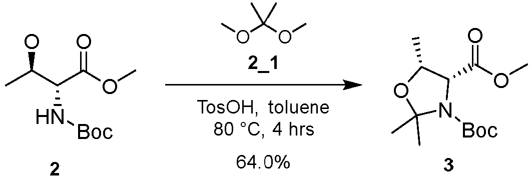

To a solution of compound **2** (40.0 g, 171 mmol) and compound **2_1** (178 g, 1.71 mol) in toluene (400 mL) was added TsOH (4.43 g, 25.7 mmol). The mixture was stirred at 80 °C for 4 hrs. LC-MS showed reactant **2** was consumed completely and one main peak with desired mass was detected. The reaction mixture was concentrated under reduced pressure to remove solvent. The residue was diluted with NaHCO₃ aqueous solution (500 mL) and extracted with MTBE (250 mL * 3). The combined organic layers were washed with brine (300 mL), dried over MgSO₄, filtered and concentrated under reduced pressure to give a residue. The crude compound **3** (30.0 g, 64.0% yield) was obtained as a yellow oil, which was used into the next step without further purification.
¹H NMR: (400 MHz, CHLOROFORM-*d*) *δ* 4.45 - 4.24 (m, 2H), 3.75 (s, 3H), 1.72 (d, *J* = 15.6 Hz, 3H), 1.54 - 1.47 (m, 8H), 1.41 (s, 5H), 1.27 - 1.22 (m, 3H)

### General procedure for preparation of compound 4 (Scheme 2)

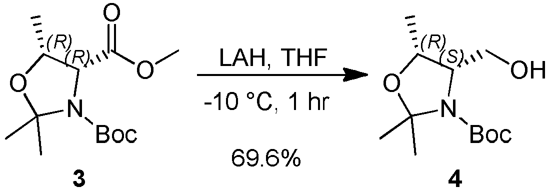

To a solution of compound **3** (20.0 g, 73.17 mmol) in THF (250 mL) was added LAH (4.17 g, 110 mmol) at 0 °C. The mixture was stirred at -10 °C for 1 hrs. TLC (petroleum ether: ethyl acetate = 10: *1, R_{f}* = 0.07) indicated reactant **3** was consumed completely and a new spot formed. The mixture was quenched with H₂O (4 mL) and 15% NaOH (4 mL), filtered and concentrated under reduced pressure to give a residue. Compound **4** (12.5 g, 69.6% yield) was obtained as a yellow oil, which was used into the next step without further purification.
¹H NMR: (400 MHz, DMSO-*d*₆) *δ* 4.64 (br s, 1H), 4.28 - 4.14 (m, 1H), 3.76 - 3.56 (m, 1H), 3.50 - 3.37 (m, 2H), 1.41 (br d, *J* = 3.5 Hz, 15H), 1.22 (d, *J* = 6.5 Hz, 3H)

### General procedure for preparation of compound 5 (Scheme 2)

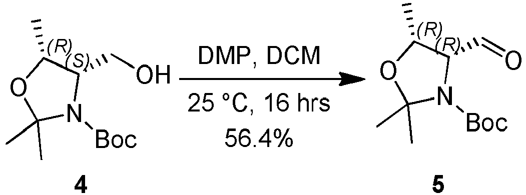

To a solution of compound **4** (12.5 g, 50.9 mmol) in DCM (200 mL) was added DMP (25.9 g, 61.2 mmol). The mixture was stirred at 25 °C for 16 hrs. TLC (petroleum ether: ethyl acetate = 10: 1, *R_{f} =* 0.41) indicated reactant **4** was consumed completely and one new spot formed. The reaction mixture was filtered and concentrated under reduced pressure to give a residue. The residue was purified by flash silica gel chromatography (ISCO^{®}; 220 g SepaFlash^{®} Silica Flash Column, Eluent of 0-10% Ethyl acetate/Petroleum ether gradient @ 100 mL/min). Compound 5 (7.01 g, 56.4% yield) was obtained as a yellow oil.
¹H NMR: (400 MHz, DMSO-*d*₆) *δ* 9.58 (dd, *J =* 2.5, 5.4 Hz, 1H), 4.45 (quin, *J =* 6.5 Hz, 1H), 4.27 (td, *J* = 3.0, 6.5 Hz, 1H), 1.59 (s, 3H), 1.50 - 1.40 (m, 9H), 1.33 (s, 5H), 1.20 (dd, *J* = 3.3, 6.4 Hz, 3H)

### General procedure for preparation of compound 6 (Scheme 2)

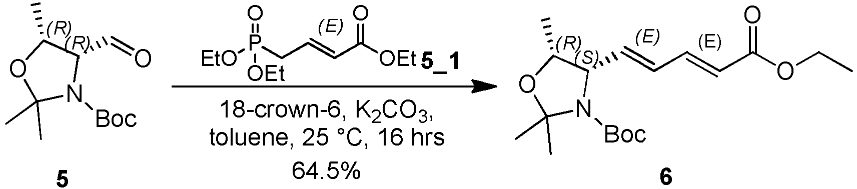

To a solution of compound **5** (3.5 g, 14.4 mmol) and compound **5_1** (4.68 g, 18.7 mmol,) in toluene (60.0 mL) was added K₂CO₃ (3.98 g, 28.8 mmol) and 18-crown-6 (380 mg, 1.44 mmol). The mixture was stirred at 25 °C for 16 hrs. TLC (petroleum ether: ethyl acetate = 10: 1, *R_{f} =* 0.39) indicated reactant **5** was consumed completely and one new spot formed. The reaction mixture was diluted with water (100 mL) and extracted with EtOAc (100 m L * 3). The combined organic layers were washed with brine (100 mL), dried over MgSO₄, and filtered and concentrated under reduced pressure to give a residue. The residue was purified by flash silica gel chromatography (ISCO^{®}; 40 g SepaFlash^{®} Silica Flash Column, Eluent of 0-10% Ethyl acetate/Petroleum ether gradient @ 50 mL/min). Compound 6 (3.15 g, 64.5% yield) was obtained as a yellow oil.
¹H NMR: (400 MHz, CHLOROFORM-*d*) *δ* 9.54 - 9.44 (m, 1H), 7.29 - 7.16 (m, 1H), 6.31 - 6.09 (m, 1H), 5.93 - 5.76 (m, 2H), 4.38 - 4.05 (m, 4H), 1.49 - 1.45 (m, 3H), 1.44 - 1.38 (m, 6H), 1.34 - 1.30 (m, 6H), 1.27 - 1.19 (m, 4H), 1.09 (d, *J* = 6.3 Hz, 3H)

### General procedure for preparation of compound 7 (Scheme 2)

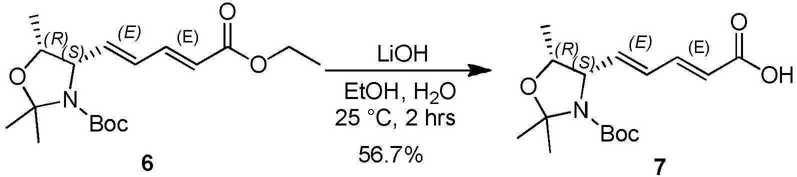

To a solution of compound **6** (1.00 g, 2.95 mmol) in THF (5 mL) and H₂O (5 mL) was added LiOH (353 mg, 14.7 mmol). The mixture was stirred at 25 °C for 2 hrs. TLC (petroleum ether: ethyl acetate = 10: 1, *R_{f}* = 0.52) showed reactant **6** was consumed completely and one main peak with desired mass was detected. The reaction mixture was diluted with H₂O (50 mL) and extracted with EtOAc (50 mL * 2), the organic layer was discarded. The aqueous layer was adjusted to pH 2 with 2 M HCl, then extracted with EtOAc (50 mL * 2). The combined organic layers were washed with brine (50 mL), dried over MgSO₄, filtered and concentrated under reduced pressure to give a residue. The residue was purified by flash silica gel chromatography (ISCO^{®}; 20 g SepaFlash^{®} Silica Flash Column, Eluent of 0-25% Ethyl acetate/Petroleum ether gradient @ 30 mL/min). Compound 7 (0.52 g, 56.7% yield) was obtained as a yellow oil.
¹H NMR (400 MHz, CHLOROFORM-d) *δ* 7.38 (td, *J =* 11.4, 15.2 Hz, 1H), 6.44 - 6.17 (m, 1H), 6.00 (br dd, *J* = 8.4, 15.0 Hz, 1H), 5.90 (d, *J* = 15.4 Hz, 1H), 4.42 - 4.20 (m, 2H), 4.20 - 4.08 (m, 1H), 1.70 - 1.52 (m, 6H), 1.51 - 1.37 (m, 10H), 1.33 - 1.24 (m, 1H), 1.18 (d, *J* = 6.4 Hz, 3H)

### General procedure for preparation of Compound 110 (Scheme 2)

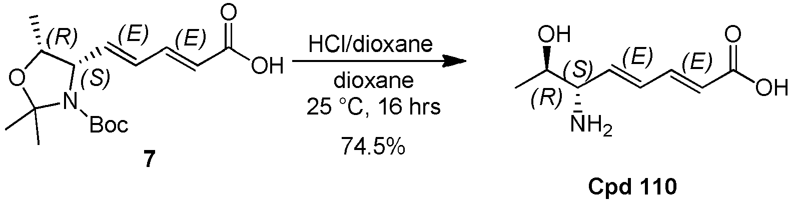

To a solution of compound **7** (500 mg, 1.61 mmol) in 4 M HCl/dioxane (3 mL) and dioxane (3 mL). The mixture was stirred at 25 °C for 16 hrs. LC-MS showed reactant **7** was consumed completely and one main peak with desired mass was detected. The reaction mixture was concentrated under reduced pressure to give a residue. The residue was dissolved in H₂O (20 mL) and washed with EtOAc (20 mL*2) and the liquid layer was lyophilized to give **Cpd 110** (205 mg, 74.5% yield) as orange gum.
¹H NMR (400 MHz, DEUTERIUM OXIDE) *δ* 7.42 - 7.24 (m, 1H), 6.70 - 6.53 (m, 1H), 6.22 - 6.00 (m, 2H), 4.17 - 4.06 (m, 1H), 3.99 - 3.86 (m, 1H), 3.79 - 3.71 (m, 1H), 3.81 - 3.70 (m, 1H), 1.25 - 1.12 (m, 3H)

### EXAMPLE 2

### General Synthetic Schemes for Compounds of the Invention

### General Schemes for the synthesis of compounds 154 and 167

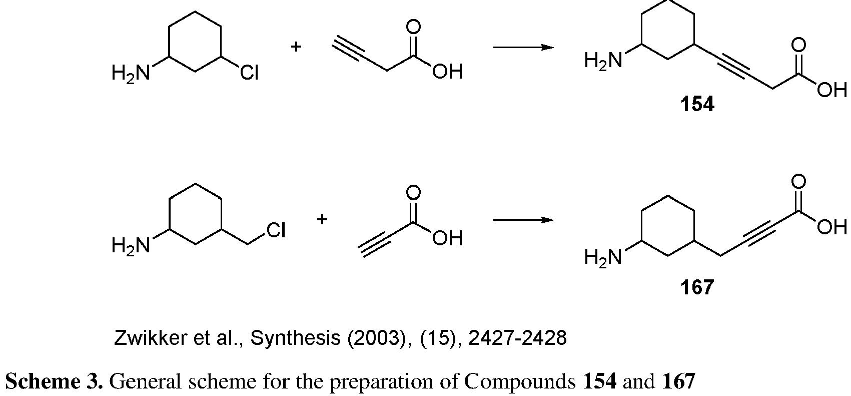

Compounds **154** and **167** were synthesized as described by Zwikker et al. **(Scheme 3),** by reacting an alkyne-containing aliphatic carboxylic acid with a cyclohexylamine bearing an halogen substituent. Expected yield for this reaction is 71%.

### General Schemes for the synthesis of compounds 169, 170, 112, 125, 146, 157 and 166

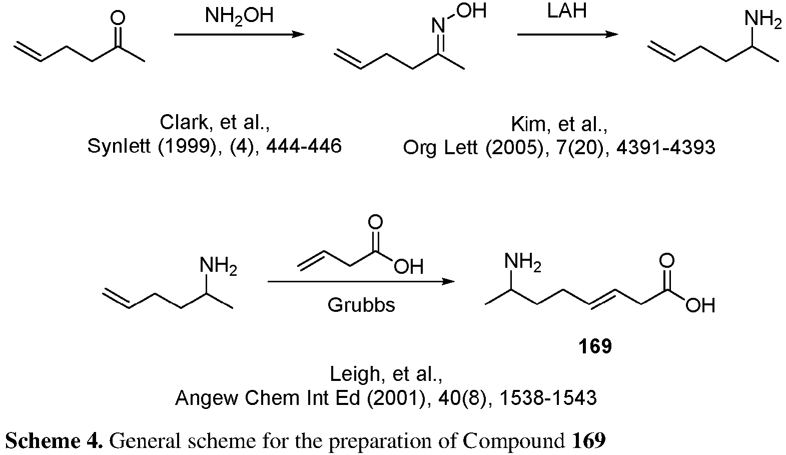

Compound **169** is synthesized by reacting hex-5-en-2-one with hydroxyl amine, followed by reduction with LAH to obtain hex-5-en-2-amine, which is further reacted with but-3-enoic acid and Grubbs catalyst **(Scheme 4).**

Compounds **170, 112, 125, 146, 157** and **166** are prepared in an analogous fashion by suitably replacing the starting ketone and the carboxylic acid **(Scheme 4).**

### EXAMPLE 3

### Herbicidal Activity Data

### Applications on a weed panel

Herbicidal activity of compounds (active ingredient; A.I.) was demonstrated by the following greenhouse experiments:

### In-planta Low through-put screens (LTP) results

### Post-emergence treatments

A basic panel of eight weed species (Table 3) sowed in 4X4X7cm plastic pots containing a garden mix (klasmann). Each specie was sowed in a separate pot. In each pot, 10-15 seeds were sowed according to the specie viability. Timing of application determined at a 1-2 true leaf stage. The plants grew for 30 days in a controlled greenhouse (26±2 ^{O}C day, 20±2 ^{O}C night). Flood irrigation (tap water + Shefer 5:3:8 8mM) was given at a 50% water content by weight. Two days before application, the tested plants thinned down to three plants per pot. Compounds were soluble in water (DDW), and commercial herbicide control was soluble in formulation B (Table 4). Before application, 1% (v/v) crop oil and 0.02% (v/v) surfactant (Tergitol^{™} 15-S-7) were added to the solution. Application was conducted with an industrial sprayer (TeeJet 6502E nozzle) at a rate of 2kg/ha and spray volume of 480l/ha. Plants were evaluated at 3 time points (4, 8, 12 days after application (DAA)). At each time point, visual phenotyping was recorded using a scale of 0-6 (0: no visible effect, 6: maximum effect). At 12 DAA, plants foliage was harvested, dried and weighed for dry weight analysis.

**Post-emergence advanced** dose response experiment included 4 species: SETVI, ECHCO, AMAPA, ABUTH (Tables 3, 5). Application was conducted at six rates between 0.6-0.0187 kg/ha and spray volume of 480 l/ha. Plants were evaluated at 4 time points (6, 12, 18 and 26 DAA). At each time point, visual phenotyping was recorded using a scale of 0-6 (0: no visible effect, 6: maximum effect). At 26 DAA, plants foliage was harvested, dried and weighed for dry weight analysis.

**Post-emergence advanced wide panel** experiment included 24 weed species (Table 5). Application was conducted at 2 rates of 2 kg/ha and 0.25 kg/ha and spray volume of 480 l/ha. Visual phenotyping was recorded at 4, 11, 17 and 20 DAA using a scale of 0-6 (0: no visible effect, 6: maximum effect). At 21 DAA, plants foliage was harvested, dried, and weighed for dry weight analysis. All experiments included an untreated control, a solvent control, and a positive control (commercial herbicide A.I.). Statistical analysis for visual phenotyping determined by a median value of ≥3.5 and Fisher test (pval≤0.05). Statistical analysis for dry weight determined by % inhibition ≥50 and T test (pval≤0.05), as well as Wilcox test (pval≤0.05).

### Pre-emergence treatments

A basic panel of 8 weed species (Table 3) were sowed in 4X4X7 cm plastic pots containing inert sand (Sweet sand), intensively washed using osmosis water. Each specie was sowed in a separate pot. In each pot 10-15 seeds were sowed according to the specie viability. Sowing was performed one day before application. The plants were grown for 21 days in a controlled greenhouse (26±2 ^{O}C day 20±2 ^{O}C night). Flood irrigation (tap water + Shefer 5:3:8 8mM) was given at a 50% water content by weight. Compounds were soluble in water (DDW), and commercial herbicide control was soluble in formulation B (Table 4). Application was conducted with an industrial sprayer (TeeJet 6502E nozzle) at a rate of 2kg/ha and spray volume of 480l/ha.

**Pre-emergence advanced** dose response experiment application was conducted at six rates between 1-0.0312 kg/ha and spray volume of 480 l/ha. Percentage of emergence was evaluated at 15 DAA. Visual phenotyping was recorded using a scale of 0-6 (0: no visible effect, 6: maximum effect) at 18 DAA. All experiments included an untreated control, a solvent control and a positive control (commercial herbicide A.I.). Statistical analysis for visual phenotyping determined by a median value ≥ 3.5 and Fisher test (pval≤0.05). Statistical analysis for plant emergence determined by % emergence ≥50 and T test (pval≤0.05), as well as Wilcox test (pval≤0.05).

**Table 3: Basic weed panel species**

| Bayer code | Scientific name |
|---|---|
| ABUTH | *Abutilon theophrasti* |
| AMBEL | *Ambrosia artemisiifolia* |
| AMAPA | *Amaranthus palmeri* |
| MATCH | *Matricaria chamomilla* |
| ALOMY | *Alopecurus myosuroides* |
| POAAN | *Poa annua* |
| LOLPE | *Lolium perenne* |
| SETVI | *Setaria viridis* |

**Table 4: Formulations**

| Formulation | Ingredients |
|---|---|
| A | 100% DDW |
| B | 48.5% acetone |
| | 40% DDW |
| | 10% isopropyl alcohol |
| | 1.5% DMSO |

**Table 5: Advanced panel species**

| Bayer code | Scientific name |
|---|---|
| ABUTH | *Abutilon theophrasti* |
| AMBEL | *Ambrosia artemisiifolia* |
| AMAPA | *Amaranthus palmeri* |
| MATCH | *Matricaria chamomilla* |
| ERICA | *Conyza Canadensis* |
| EPHHL | *Euphorbia heterophylla* |
| AMARE | *Amaranthus retroflexus* |
| SOLNI | *Solanum nigrum* |
| CHEAL | *Chenopodium album* |
| POROL | *Portulaca oleracea* |
| GLXMA | *Glycine max* |
| BRSNN | *Brassica napus* |
| ALOMY | *Alopecurus myosuroides* |
| POAAN | *Poa annua* |
| LOLPE | *Lolium perenne* |
| SETVI | *Setaria viridis* |
| DIGSA | *Digitaria sanguinalis* |
| ECHCO | *Echinochloa colona* |
| LOLRI | *Lolium rigidum* |
| PHAPA | *Phalaris paradoxa* |
| ZEAMX | *Zea mays* |
| A VEST | *Avena sterilis* |
| TRZAX | *Triticum aestivum* |
| ORYSA | *Oryza sativa* |

### Results:

The *In-planta* low throughput (LTP) results for compounds **101, 110, 125, 119, 124, 146** and **154** are presented in **Table 6** below:

**Table 6. Herbicidal activity (In-planta) for compounds according to this invention.**

| **LTP - pre-emergence** | **101** | **110** | **125** | **146** | **154** | **124** | **119** | **170** | **169** |
|---|---|---|---|---|---|---|---|---|---|
| *Setaria viridis* | IG (100) | IG (80) | IG (100) | IG (60) | IG (100) | IG (100) | IG (80) | IG (100) | IG (95) |
| *Alopecurus myosuroides* | IG (80) | | IG (60) | IG (60) | IG (80) | IG (80) | | | |
| *Lolium perenne* | IG (80) | | IG (80) | | | | | | |
| *Poa annua* | IG (100) | IG (60) | IG (100) | IG (60) | IG (100) | IG (100) | | | |
| *Echinochloa colona* | IG (100) | | | | IG (100) | IG (100) | IG (60) | | |
| *Digitaria sanguinalis* | IG (100) | | | | | | | | |
| *Amaranthus palmeri* | | IG (80) | IG (80) | IG (80) | IG (80) | IG (80) | IG (80) | | |
| *Matricaria chamomilla* | | IG (60) | IG (60) | | | | | | |
| *Echinochloa crus-galli* | IG (100) | | | | | | | | |
| *Lolium multiflorum* | IG (100) | | | | | | | | |
| *Phalaris paradoxa* | IG (80) | | | | | | | | |
| *Amaranthus retroflexus* | | | | | | | | IG (90) | IG (80) |

| **LTP - post emergence** | **101** | | | | **110** | | **125** | | |
|---|---|---|---|---|---|---|---|---|---|
| *Setaria viridis* | IG (60), DAB (60) | | | | | | IG (60) | | |
| *Alopecurus monureide's* | IG (80), DAB (80) Drd (80), B1 (60) | | | | | | | | |
| *Lolium perenne* | | IG (60), Drd (80) | | | | | | | |
| *Poa annua* | | IG (80), Drd (80) | | | | | | IG (60) | |
| *Echinochloa colona* | | IG (80), DAB (60), Drd (80) | | | | | | | |
| *Digitaria sanguinalis* | | IG (80), DAB (80), Drd (80) | | | | | | | |
| *Amaranthus palmeri* | | | | | | | | IG (60) | |
| *Lolium multiflorum* | | Drd (60) | | | | | | | |
| *Amaranthus tuberculatus* | | | | | Drd (80) | | | | |
| *Echinochloa crus-galli* | | IG (80), DAB (60), Drd (80) | | | | | | | |
| *Galium aparine* | | | | | B1 (60) | | | | |

| | | | | | | | | | |
|---|---|---|---|---|---|---|---|---|---|
| **Bl:** bleaching **IG:** inhibited growth **Drd:** disturbed root growth **DAB:** disturbed apical bud | | | | | | | | | |

The results show excellent control of compound **101** on both monocot and eudicot weeds species in both post-emergence and pre-emergence applications modes for most of the species tested. Compound **110** shows good to moderate activity on dicots and monocots when applied pre-emergence. In addition, compounds **125, 119, 124, 146** and **154** displayed very good to moderate growth inhibition, mainly in pre-emergence applications.

### In-planta high through-put screens (HTPS) results

### Post-emergence applications on miniature dicot model plants

*Arabidopsis thaliana* seeds were sown in 96 well plates filled with irrigated Sweet sand (10% > clay) that is washed from salts and minerals using tap water. 5-10 seeds were sown in the center of each well. 7-8 days post sowing, at 2 true leaves stage, thinning out was performed to ensure that compound application is done on a single plant per well. The plates were placed in a controlled greenhouse in a random order inside a bath which allows flooding irrigation with tap water supplemented with a fertilizer. Applied compounds were dissolved into a final solution of 50% acetone, 49.9% DDW, 0.1% tween-20.

A 96 well plate was used as a stock plate for preparing application solutions for 8 repeats. Each row contained different concentrations per chemical. Maximal concentration for application was 1.5 kg/ha and dilution factor is 2.5. Chemical application was performed one day after thinning out in a chemical hood. 5µL applied on first two true leaves in each well using 12 channel pipettes. Data collection: RGB (red, green, blue) data for green area per well was documented using camera. Data is collected at a few time points during the experiment: one day after thinning and before chemical application, two-, six- or nine-days post application. During the last two documentations, visual phenotyping was performed. Data analysis: Given RGB results and visual phenotype scores, a student *t*-test conducted to compare between treatment and control performance for continuous data (RGB) and Fisher exact test to analyze the noncontinuous data (phenotypic scores). Dose-Respons curves are generated for each treatment to infer ED₅₀ and max. inhibition parameters, using treatment's log concentration range as the dependent variable and normalized green area.

### Pre-emergence applications on dicot and monocot model plants

These experiments are conducted similarly except that either *Arabidopsis thaliana* or *Eragrostis teff* seeds were sowed (5-10 or 5-7, respectively), plant thinning out was not performed, compound dissolved into a final solution of 50% acetone, 49.9% DDW, compound application was done at 30µL volume per well directly on sowed soil before plant emergence and data was collected 10 or 7 days after chemical application on dicot or monocot plants, respectively.

### Imaging, RGB and statistical analysis

Plate imaging performed at 11 and 18 DAA. RGB data for green area per well was documented and used to extract % inhibition. Dose-Response curves were built for each treatment to infer EC₅₀, EC₇₅ and EC₉₀ parameters, using treatment's log concentration range as the independent variable and normalized green area as the dependent variable. Treatments were compared to controls performance given RGB results and using student's t-test (p-val≤0.05).

### Results:

Compounds **101, 110, 112, 119, 124, 125, 146, 154, 157, 172, 170, 169, 112,** and **166** were applied in either or both pre- and post-emergence mode to either or both monocot and dicot model plants, following the methods described above. The compounds were applied as a gradient of concentrations as listed above, from which the ED₅₀ was calculated.

The *in-planta* high through put screens (HTPS) results for compounds **101, 110, 112, 119, 124, 125, 146, 154, 157, 172, 170, 169, 112,** and **166** are presented in **Table 7** below:

**Table 7. Herbicidal activity for compounds according to this invention.**

| **System** | **Compound** | **Max inhibition (%)** | **ED₅₀ (kg/ha)** |
|---|---|---|---|
| | **101** | 100 | 0.003 |
| | **110** | 100 | 0.009 |
| | **119** | 100 | 0.049 |
| | **124** | 100 | 0.013 |
| | **125** | 100 | 0.006 |
| **HTPS post dicot** | **146** | 100 | 0.271 |
| | **154** | 100 | 0.038 |
| | **157** | 100 | 0.151 |
| | **172** | 100 | 0.192 |
| | **170** | 100 | 0.099 |
| | **169** | 100 | 0.676 |
| | **112** | 87.5 | 0.452 |
| | **166** | 75 | N/A |
| **HTPS Pre dicot** | **146** | 100 | N/A |
| | **157** | 100 | 0.152 |
| | **172** | 100 | 0.202 |
| | **112** | 100 | 0.716 |
| **HTPS Pre monocot** | **146** | 100 | N/A |
| | **112** | 51 | N/A |
| | **166** | 47 | N/A |

The results in the table show excellent to very good control of most compounds, (particularly compounds **101** and **110**) in the systems where they were tested.

## Claims

1. A compound represented by the structure of formula I: 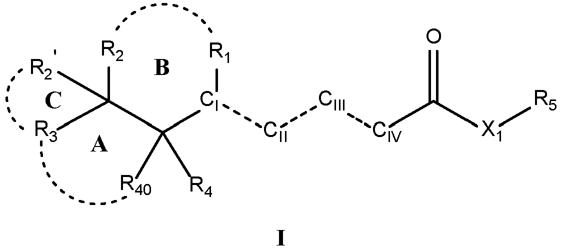 wherein
C_{I}, C_{II}, C_{III} and C_{IV} are each independently an sp, sp² or sp³ carbon atom depending on whether they are part of a triple, double, or a single bond respectively; and are each independently C; CH or C(**R₂₀**); or CH₂, CH(**R₂₀**) or C(**R₂₀**)₂ for sp; sp²; or sp³ carbon atom respectively;
wherein R₂₀ is a halogen (e.g., F) or a C₁-C₅ linear of branched alkyl (e.g., methyl);
C_{I}---C_{II}, C_{II}---C_{III}, C_{III}---C_{IV} are each independently a single bond or a double bond, wherein at least one of C_{I}---C_{II}, C_{II}---C_{III}, and C_{III}---C_{IV} is a double bond; or C_{I}---C_{II}, C_{II}---C_{III}, and C_{III}---C_{IV} are each independently a single bond or a triple bond, wherein at least one of C_{I}---C_{II}, C_{II}---C_{III}, and C_{III}---C_{IV} is a triple bond;
**R₁** is H, F, Cl, Br, I, C₁-C₅ linear or branched, substituted or unsubstituted alkyl (e.g., methyl, ethyl, propyl, iso-propyl, t-Bu, iso-butyl, pentyl, benzyl);
wherein if C_{I}---C_{II} is a triple bond then **R₁** is absent;
**R₂** is F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
or **R₁** and **R₂** are joined to form a 3-8 membered substituted or unsubstituted, saturated or unsaturated carbocyclic ring **B** (e.g., cyclohexyl, cyclohexenyl, cyclopentyl);
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
or **R₃** and **R₂'** are joined to form a 3-8 membered substituted or unsubstituted, cycloalkyl ring **C** (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
or **R₃** and **R₄₀** are joined together to form a 3-8 membered substituted or unsubstituted, cycloalkyl ring A (e.g., cyclopropyl, cyclobutyl, cyclopentyl, cyclohexyl);
**R₅** is H, C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, NH or N-R₅₀;
**R₈** is [CH₂]ₚ
wherein p is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, i-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**B** ring is absent, or is a 3-8 membered substituted or unsubstituted, saturated or unsaturated carbocyclic ring (e.g., cyclohexyl, cyclohexenyl, cyclopentyl);
**C** ring is absent, or is a 3-8 membered substituted or unsubstituted, cycloalkyl ring (e.g., cyclopropyl);
wherein at least one of **A, B** and **C** rings is not absent;
or its agrochemically acceptable salt, zwitterion (inner salt), stereoisomer, tautomer, hydrate, N-oxide, reverse amide analog, isotopic variant (e.g., deuterated analog), or any combination thereof; wherein at least one of **R₃** and **R₄** is NH₂.

2. A compound represented by the structure of formula **I:** 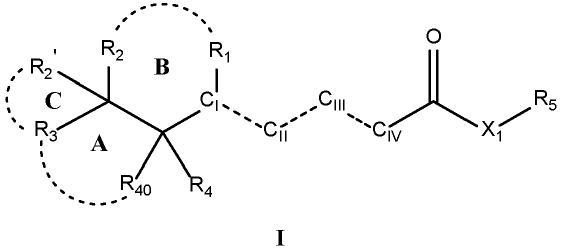 wherein
C_{I}, C_{II}, C_{III} and C_{IV} are each independently an sp, sp² or sp³ carbon atom depending on whether they are part of a triple, double, or a single bond respectively; and are each independently C; CH or C(**R₂₀**); or CH₂, CH(**R₂₀**) or C(**R₂₀**)₂ for sp; sp²; or sp³ carbon atom respectively;
wherein **R₂₀** is a halogen (e.g., F) or a C₁-C₅ linear of branched alkyl (e.g., methyl);
C_{I}---C_{II}, C_{II}---C_{III}, C_{III}---C_{IV} are each independently a single bond or a double bond, wherein at least one of C_{I}---C_{II}, C_{II}---C_{III}, and C_{III}---C_{IV} is a double bond; or C_{I}---C_{II}, C_{II}---C_{III}, and C_{III}---C_{IV} are each independently a single bond or a triple bond, wherein at least one of C_{I}---C_{II}, C_{II}---C_{III}, and C_{III}---C_{IV} is a triple bond;
**R₁** is H, F, Cl, Br, I, C₁-C₅ linear or branched, substituted or unsubstituted alkyl (e.g., methyl, ethyl, propyl, iso-propyl, t-Bu, iso-butyl, pentyl, benzyl);
wherein if C_{I}---C_{II} is a triple bond then **R₁** is absent;
**R₂** is F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
or **R₁** and **R₂** are joined to form a 3-8 membered substituted or unsubstituted, saturated or unsaturated carbocyclic ring **B** (e.g., cyclohexyl, cyclohexenyl, cyclopentyl);
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
or **R₃** and **R₂'** are joined to form a 3-8 membered substituted or unsubstituted, cycloalkyl ring **C** (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
or **R₃** and **R₄₀** are joined together to form a 3-8 membered substituted or unsubstituted, cycloalkyl ring **A** (e.g., cyclopropyl, cyclobutyl, cyclopentyl, cyclohexyl);
**R₅** is H, C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, NH or N-R₅₀;
**R₈** is [CH₂]ₚ
wherein **p** is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, i-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**B** ring is absent, or is a 3-8 membered substituted or unsubstituted, saturated or unsaturated carbocyclic ring (e.g., cyclohexyl, cyclohexenyl, cyclopentyl);
**C** ring is absent, or is a 3-8 membered substituted or unsubstituted, cycloalkyl ring (e.g., cyclopropyl);
or its agrochemically acceptable salt, zwitterion (inner salt), stereoisomer, tautomer, hydrate, N-oxide, reverse amide analog, isotopic variant (e.g., deuterated analog), or any combination thereof;
wherein one of **R₃** and **R₄** is NH₂ and the other one is OH.

3. A compound represented by the structure of formula I: 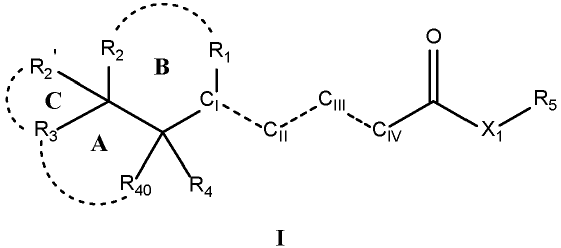 wherein
C_{I}, C_{II}, C_{III} and C_{Iv} are each independently an sp, sp² or sp³ carbon atom depending on whether they are part of a triple, double, or a single bond respectively; and are each independently C; CH or C(**R₂₀**); or CH₂, CH(**R₂₀**) or C(**R₂₀**)₂ for sp; sp²; or sp³ carbon atom respectively;
wherein **R₂₀** is a halogen (e.g., F) or a C₁-C₅ linear of branched alkyl (e.g., methyl);
C_{I}---C_{II}, C_{II}---C_{III}, C_{III}---C_{IV} are each independently a single bond or a double bond, wherein at least one of C_{I}---C_{II}, C_{II}---C_{III}, and C_{III}---C_{IV} is a double bond; or C_{I}---C_{II}, C_{II}---C_{III}, and C_{III}---C_{IV} are each independently a single bond or a triple bond, wherein at least one of C_{I}---C_{II}, C_{II}---C_{III}, and C_{III}---C_{IV} is a triple bond;
**R₁** is H, F, Cl, Br, I, C₁-C₅ linear or branched, substituted or unsubstituted alkyl (e.g., methyl, ethyl, propyl, iso-propyl, t-Bu, iso-butyl, pentyl, benzyl);
wherein if C_{I}---C_{II} is a triple bond then **R₁** is absent;
**R₂** is F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
or **R₁** and **R₂** are joined to form a 3-8 membered substituted or unsubstituted, saturated or unsaturated carbocyclic ring **B** (e.g., cyclohexyl, cyclohexenyl, cyclopentyl);
**R₂'** is H, F, Cl, Br, I, C₁-C₅ linear or branched, substituted or unsubstituted alkyl (e.g., methyl, ethyl, propyl, iso-propyl, t-Bu, iso-butyl, pentyl), C₂-C₅ linear or branched, substituted or unsubstituted alkenyl (e.g., ethenyl (CH=CH₂)), C₂-C₅ linear or branched, substituted or unsubstituted alkynyl (e.g., ethynyl (CCH));
**R₃** is H, F, Cl, Br, I, OH, SH, NH₂, NHR, N(R)₂;
or **R₃** and **R₂'** are joined to form a 3-8 membered substituted or unsubstituted, cycloalkyl ring **C** (e.g., cyclopropyl);
**R₄** is H, F, Cl, Br, I, OH, SH, NH₂, NHR, N(R)₂;
**R₄₀** is H, C₁-C₅ linear or branched, substituted or unsubstituted alkyl (e.g., methyl, ethyl, propyl, iso-propyl, t-Bu, iso-butyl, pentyl);
or **R₃** and **R₄₀** are joined together to form a 3-8 membered substituted or unsubstituted, cycloalkyl ring **A** (e.g., cyclopropyl, cyclobutyl, cyclopentyl, cyclohexyl);
**R₅** is H, C₂-C₅ linear or branched, substituted or unsubstituted alkenyl, C₂-C₅ linear or branched, substituted or unsubstituted alkynyl (e.g., CCH, CH₂-CCH,), C₁-C₅ linear or branched haloalkyl (e.g., CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (e.g., CH₂-Ph), C(=CH₂)-R₁₀ (e.g., C(=CH₂)-C(O)-OCH₃, C(=CH₂)-CN), substituted or unsubstituted alkyl sulfone (e.g., SO₂-CH₂-cyclopropyl), substituted or unsubstituted aryl (e.g., phenyl), substituted or unsubstituted heteroaryl (e.g., pyridine (2, 3, and 4-pyridine);
**X₁** is O, NH or N-R₅₀;
**R₈** is [CH₂]ₚ
wherein p is between 1 and 10;
**R₁₀** is H, CN, C₁-C₅ linear or branched alkyl (e.g., methyl, ethyl), C(O)R (e.g., C(O)(OCH₃)), or S(O)₂R;
**R₅₀** is H, C₁-C₅ linear or branched, substituted or unsubstituted alkyl (e.g., methyl, ethyl, butyl, i-propyl);
**R** is C₁-C₅ linear or branched alkyl, C₁-C₅ linear or branched alkoxy, phenyl, aryl or heteroaryl, or two gem R substituents are joined together to form a 5 or 6 membered heterocyclic ring;
**A** ring is absent, or is a 3-8 membered substituted or unsubstituted, cycloalkyl ring (e.g., cyclopropyl, cyclobutyl, cyclopentyl, cyclohexyl);
**B** ring is absent, or is a 3-8 membered substituted or unsubstituted, saturated or unsaturated carbocyclic ring (e.g., cyclohexyl, cyclohexenyl, cyclopentyl);
**C** ring is absent, or is a 3-8 membered substituted or unsubstituted, cycloalkyl ring (e.g., cyclopropyl);
or its agrochemically acceptable salt, zwitterion (inner salt), stereoisomer, tautomer, hydrate, N-oxide, reverse amide analog, isotopic variant (e.g., deuterated analog), or any combination thereof; wherein at least one of **R₃** and **R₄** is NH₂.

4. The compound of any one of claims 1 to 3, represented by the structure of formula **II(a)-II(d)** or **III(a)-III(c):** 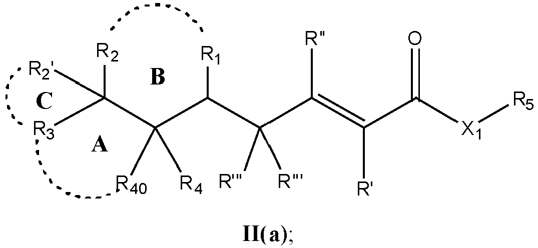 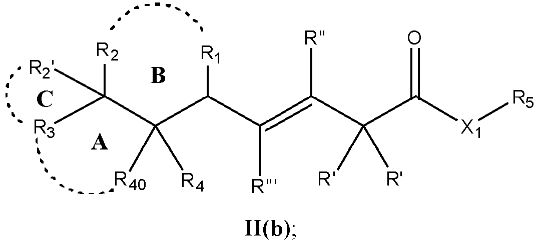 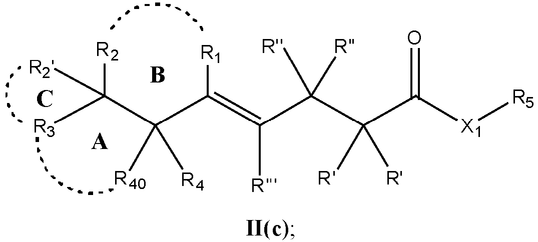 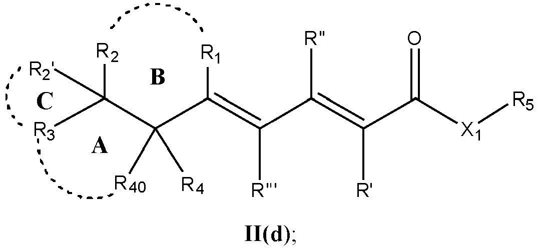 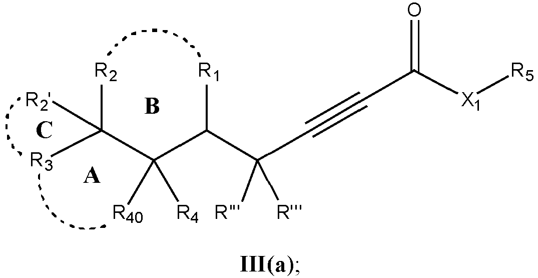 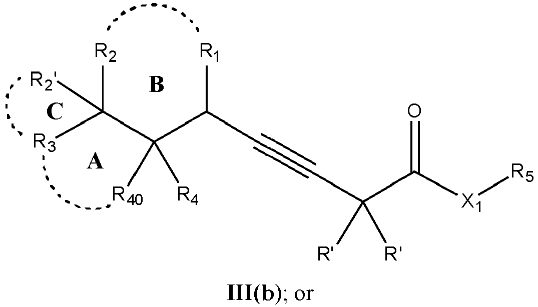 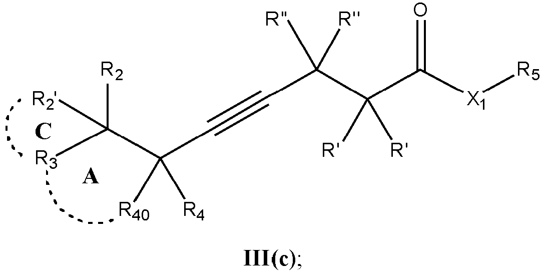 wherein
**R', R"** and **R‴** are each independently selected from a halogen (e.g., F) or a C₁-C₅ linear of branched alkyl (e.g., methyl).

5. The compound of any one of the preceding claims, wherein:
**R₂₀** is F or methyl; and/or
**R₁** is H and **R₂** is CH₃ or CH₂CH₃, or **R₁** and **R₂** are joined to form a cyclohexyl; and/or
**R₂'** is H or CH₃ and **R₃** is H, OH or NH₂, or **R₃** and **R₂'** are joined to form a cyclopropyl; and/or
**R₄** is H or NH₂; and/or **R₄₀** is H, and **R₃** is H, OH or NH₂ or **R₃** and **R₄₀** are joined to form a 3-8 membered cycloalkyl ring; and/or
**X₁** is O.

6. The compound of claim 1, wherein **R₅** is H.

7. The compound of any one of the preceding claims, wherein the compound is a substantially pure single stereoisomer, preferably wherein the compound is the substantially pure SR stereoisomer, RS stereoisomer, RR stereoisomer, or SS diastereomer, further preferably wherein the substantially pure stereoisomer has a purity higher than 90%, preferably higher than 95%, most preferably higher than 98%.

8. The compound of any one of claims 1-7, represented by any one of the following structures:
| **Compound Number** | **Structure** |
|---|---|
| **111** | 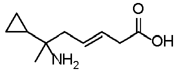 |
| **112** | 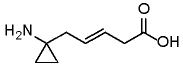 |
| **115** | 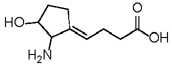 |
| **116** | 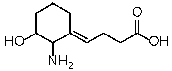 |
| **119** | 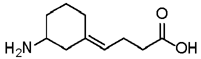 |
| **120** | 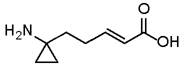 |
| **121** | 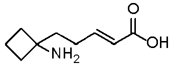 |
| **122** | 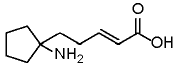 |
| **123** | 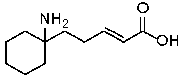 |
| **124** | 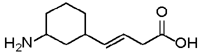 |
| **125** | 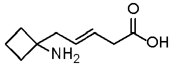 |
| **126** | 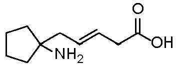 |
| **127** | 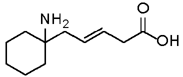 |
| **128** | 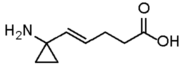 |
| **129** | 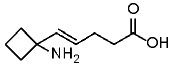 |
| **130** | 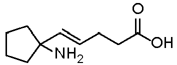 |
| **131** | 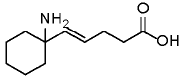 |
| **146** | 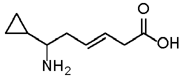 |
| **153** | 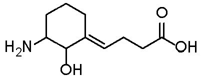 |
| **154** | 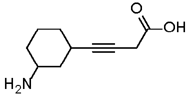 |
| **155** | 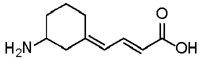 |
| **156** | 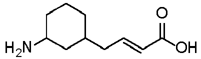 |
| **158** | 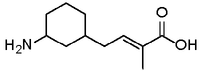 |
| **159** | 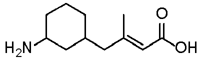 |
| **160** | 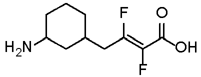 |
| **161** | 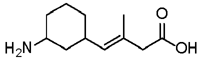 |
| **162** | 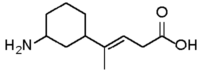 |
| **163** | 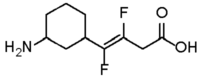 |
| **164** | 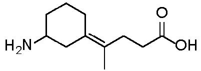 |
| **165** | 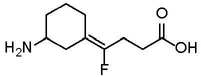 |
| **167** | 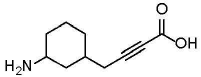 |
| **168** | 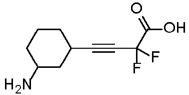 |
| **174** | 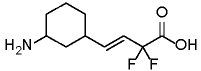 |
| **175** | 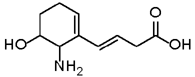 |
| **176** | 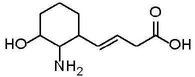 |
| **177** | 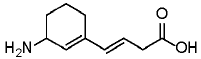 |
| **178** | 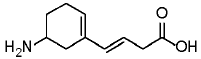 |

9. The compound of claim 2 or 3, represented by any one of the following structures:
| **Compound Number** | **Structure** |
|---|---|
| **100** | 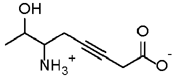 |
| **101** | 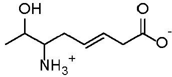 |
| **102** | 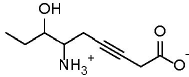 |
| **103** | 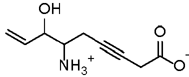 |
| **104** | 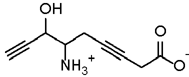 |
| **110** | 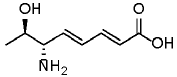 |
| **115** | 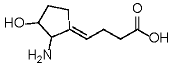 |
| **116** | 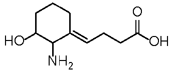 |
| **117** | 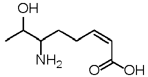 |
| **118** | 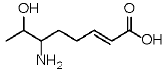 |
| **132** | 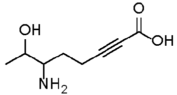 |
| **133** | 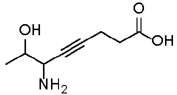 |
| **134** | 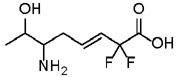 |
| **135** | 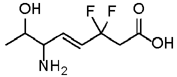 |
| **136** | 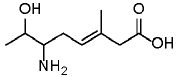 |
| **137** | 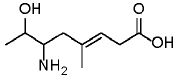 |
| **138** | 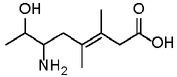 |
| **139** | 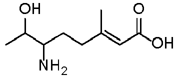 |
| **140** | 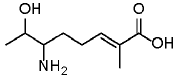 |
| **141** | 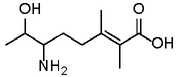 |
| **142** | 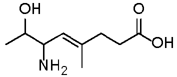 |
| **143** | 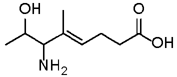 |
| **144** | 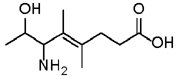 |
| **145** | 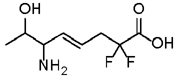 |
| **147** | 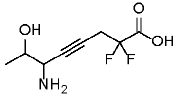 |
| **148** | 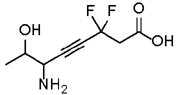 |
| **149** | 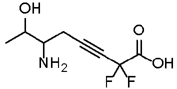 |
| **150** | 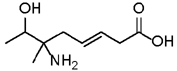 |
| **151** | 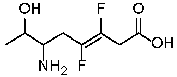 |
| **152** | 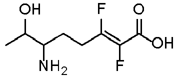 |
| **153** | 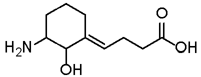 |
| **172** | 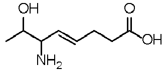 |
| **175** | 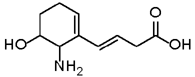 |
| **176** | 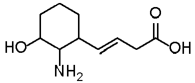 |

10. The compound of claim 3, represented by any one of the following structures:
| **Compound Number** | **Structure** |
|---|---|
| **105** | 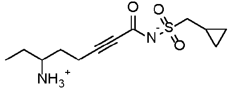 |
| **106** | 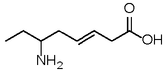 |
| **107** | 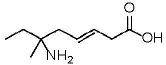 |
| **108** | 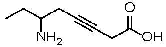 |
| **109** | 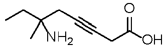 |
| **113** | 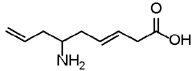 |
| **114** | 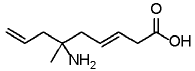 |
| **157** | 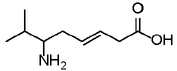 |
| **166** | 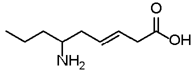 |
| **169** | 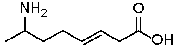 |
| **170** | 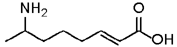 |
| **171** | 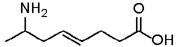 |
| **173** | 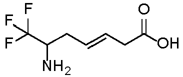 |

11. The compound of any one of the preceding claims, wherein the compound is a herbicide, a pesticide or a combination thereof.

12. The compound of any one of the preceding claims, for use in controlling the growth of an undesired plant,
preferably wherein the plant is a eudicot (dicot) or a monocotyledon (monocot) and/or preferably wherein said plant is a weed,
further preferably wherein said weed comprises: *Abutilon theophrasti , Amaranthus palmeri, Ambrosia artemisiifolia , Alopecurus myosuroides , Avena sterilis , Chenopodium album , Conyza Canadensis , Digitaria sanguinalis , Echinochloa colona, Euphorbia heterophylla , Lolium perenne , Lolium rigidum , Matricaria chamomilla , Phalaris paradoxa , Poa annua , Portulaca oleracea , Setaria viridis, Solanum nigrum* or any combination thereof; or further preferably the dicot plant is *Arabidopsis thaliana,* and/or the monocot plant is *Dactyloctenium aegyptium* or *Eragrostis teff.*

13. An agrochemical composition comprising the compound of any one of the preceding claims and an agrochemically acceptable carrier or diluent.

14. The compound of claim 12, for use in pre-plant treatments, pre-emergence treatments, post-emergence treatments, or any combination thereof.

## Patentansprüche

1. Verbindung, die durch die Struktur der Formel **I** dargestellt wird, 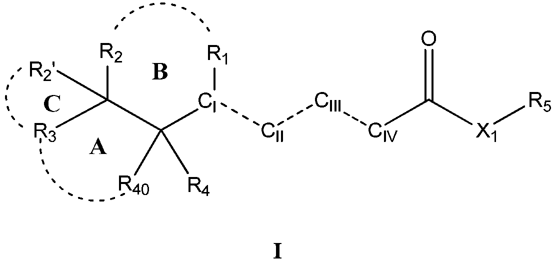 wobei
C_{I}, C_{II}, C_{III} und C_{IV} jeweils unabhängig ein sp-, sp²- oder sp³-Kohlenstoffatom sind, abhängig davon, ob sie Teil einer Dreifach-, Doppel- oder Einfachbindung sind; und jeweils unabhängig C; CH oder C(**R₂₀**); oder CH₂, CH(**R₂₀**) oder C(**R₂₀**)₂ für sp-, sp²- oder sp³-Kohlenstoffatome sind;
wobei **R₂₀** ein Halogen (z. B. F) oder ein C₁-C₅-lineares oder verzweigtes Alkyl (z. B. Methyl) ist;
**C_{I}---C_{II}, C_{II}---C_{III}, C_{III}---C_{IV}** sind jeweils unabhängig eine Einfach- oder Doppelbindung, wobei mindestens eine der **C_{I}---C_{II}, C_{II}---C_{III}** und **C_{III}---C_{IV}** eine Doppelbindung ist; oder **C_{I}---C_{II}, C_{II}---C_{III},** und **C_{III}---C_{IV}** sind jeweils unabhängig eine Einfach- oder Dreifachbindung, wobei mindestens eine der **C_{I}---C_{II}, C_{II}---C_{III},** und **C_{III}---C_{IV}** eine Dreifachbindung ist;
**R₁** ist H, F, Cl, Br, I, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl, Benzyl);
wobei, wenn **C_{I}---C_{II}** eine Dreifachbindung ist, dann **R₁** fehlt;
**R₂** ist F, Cl, Br, I, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl), C₂-C₅-linear oder verzweigtes, substituiertes oder unsubstituiertes Alkenyl (z. B. Ethenyl (CH=CH₂)), C₂-C₅-linear oder verzweigtes, substituiertes oder unsubstituiertes Alkynyl (z. B. Ethinyl (CCH));
oder **R₁** und **R₂** sind verbunden, um einen 3-8-gliedrigen substituierten oder unsubstituierten, gesättigten oder ungesättigten carbocyclischen Ring **B** (z. B. Cyclohexyl, Cyclohexenyl, Cyclopentyl) zu bilden;
**R₂'** ist H, F, Cl, Br, I, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl), C₂-C₅-linear oder verzweigtes, substituiertes oder unsubstituiertes Alkenyl (z. B. Ethenyl (CH=CH₂)), C₂-C₅-linear oder verzweigtes, substituiertes oder unsubstituiertes Alkynyl (z. B. Ethinyl (CCH));
**R₃** ist H, F, Cl, Br, I, OH, SH, NH₂, NHR, N(R)₂;
oder **R₃** und **R₂'** sind verbunden, um einen 3-8-gliedrigen substituierten oder unsubstituierten, Cycloalkyl-Ring **C** (z. B. Cyclopropyl) zu bilden;
**R₄** ist H, F, Cl, Br, I, OH, SH, NH₂, NHR, N(R)₂;
**R₄₀** ist H, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl);
oder **R₃** und **R₄₀** sind miteinander verbunden, um einen 3-8-gliedrigen substituierten oder unsubstituierten Cycloalkyl-Ring **A** (z. B. Cyclopropyl, Cyclobutyl, Cyclopentyl, Cyclohexyl) zu bilden;
**R₅** ist H, C₂-C₅-linear oder verzweigtes, substituiertes oder unsubstituiertes Alkenyl, C₂-C₅-linear oder verzweigtes, substituiertes oder unsubstituiertes Alkynyl (z. B. CCH, CH₂-CCH,), C₁-C₅-linear oder verzweigt Halogenalkyl (z. B. CF₃, CF₂CH₃, CH₂CF₃, CF₂CH₂CH₃, CH₂CH₂CF₃, CF₂CH(CH₃)₂,CF(CH₃)-CH(CH₃)₂), R₈-Aryl (z. B. CH₂-Ph), C(=CH₂)-R₁₀ (z. B. C(=CH₂)-C(O)-OCH₃, C(=CH₂)-CN), substituiertes oder unsubstituiertes Alkylsulfon (z. B. SO₂-CH₂-Cyclopropyl), substituiertes oder unsubstituiertes Aryl (z. B. Phenyl), substituiertes oder unsubstituiertes Heteroaryl (z. B. Pyridin (2-, 3- und 4-Pyridin));
**X₁** ist O, NH oder N-R₅₀;
**R₈** ist [CH₂]ₚ
wobei **p** zwischen 1 und 10 liegt;
**R₁₀ ist** H, CN, C₁-C₅-linear oder verzweigt Alkyl (z. B. Methyl, Ethyl), C(O)R (z. B. C(O)(OCH₃)), oder S(O)₂R;
**R₅₀** ist H, C₁-C₅-linear oder verzweigtes, substituiertes oder unsubstituiertes Alkyl (z. B. Methyl, Ethyl, Butyl, i-Propyl);
**R** ist C₁-C₅-linear oder verzweigt Alkyl, C₁-C₅-linear oder verzweigt Alkoxy, Phenyl, Aryl oder Heteroaryl, oder zwei gem R-Substituenten sind verbunden, um einen 5- oder 6-gliedrigen heterocyclischen Ring zu bilden;
**A** Ring fehlt oder ist ein 3-8-gliedriger substituierter oder unsubstituierter Cycloalkyl-Ring (z. B. Cyclopropyl, Cyclobutyl, Cyclopentyl, Cyclohexyl);
**B** Ring fehlt oder ist ein 3-8-gliedriger substituierter oder unsubstituierter, gesättigter oder ungesättigter carbocyclischer Ring (z. B. Cyclohexyl, Cyclohexenyl, Cyclopentyl);
**C** Ring fehlt oder ist ein 3-8-gliedriger substituierter oder unsubstituierter Cycloalkyl-Ring (z. B. Cyclopropyl);
wobei mindestens einer der A, **B** und C Ringe nicht fehlt;
oder sein agrochemisch akzeptiertes Salz, Zwitterion (inneres Salz), Stereoisomer, Tautomer, Hydrat, N-Oxid, Reverse-Amid-Analog, isotopische Variante (z. B. deuteriertes Analog) oder jede Kombination davon;
wobei mindestens eines von **R₃** und **R₄** NH₂ ist.

2. Verbindung, die durch die Struktur der Formel I dargestellt wird, 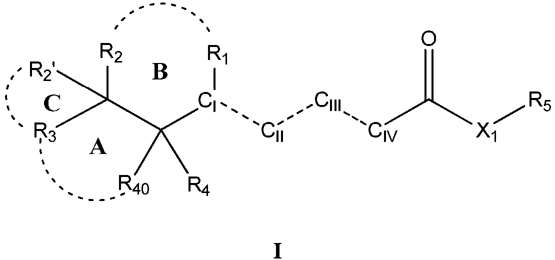 wobei
**C_{I}, C_{II}**, **C_{III}** und **C_{IV}** jeweils unabhängig ein sp-, sp²- oder sp³-Kohlenstoffatom sind, abhängig davon, ob sie Teil einer Dreifach-, Doppel- oder Einfachbindung sind; und jeweils unabhängig C; CH oder C(**R₂₀**); oder CH₂, CH(**R₂₀**) oder C(**R₂₀**)₂ für sp-, sp²- oder sp³-Kohlenstoffatome sind;
wobei **R₂₀** ein Halogen (z. B. F) oder ein C₁-C₅-lineares oder verzweigtes Alkyl (z. B. Methyl) ist;
**C_{I}---C_{II}**, **C_{II}---C_{III}**, **C_{III}---C_{IV}** sind jeweils unabhängig eine Einfach- oder Doppelbindung, wobei mindestens eine der **C_{I}---C_{II}**, **C_{II}---C_{III}** und **C_{III}---C_{IV}** eine Doppelbindung ist; oder **C_{I}---C_{II}**, **C_{II}---C_{III}**, und **C_{III}---C_{IV}** sind jeweils unabhängig eine Einfach- oder Dreifachbindung, wobei mindestens eine der **C_{I}---C_{II}**, **C_{II}---C_{III}**, und **C_{III}---C_{IV}** eine Dreifachbindung ist;
**R₁ ist** H, F, Cl, Br, I, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl, Benzyl);
wobei, wenn **C_{I}---C_{II}** eine Dreifachbindung ist, **R₁** fehlt;
**R₂** ist F, Cl, Br, I, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl), C₂-C₅-linear oder verzweigtes, substituiertes oder unsubstituiertes Alkenyl (z. B. Ethenyl (CH=CH₂)), C₂-C₅-linear oder verzweigtes, substituiertes oder unsubstituiertes Alkynyl (z. B. Ethinyl (CCH));
oder **R₁** und **R₂** sind verbunden, um einen 3-8-gliedrigen substituierten oder unsubstituierten, gesättigten oder ungesättigten carbocyclischen Ring B (z. B. Cyclohexyl, Cyclohexenyl, Cyclopentyl) zu bilden;
**R₂**' ist H, F, Cl, Br, I, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl), C₂-C₅-linear oder verzweigtes, substituiertes oder unsubstituiertes Alkenyl (z. B. Ethenyl (CH=CH₂)), C₂-C₅-linear oder verzweigtes, substituiertes oder unsubstituiertes Alkynyl (z. B. Ethinyl (CCH));
**R₃** ist H, F, Cl, Br, I, OH, SH, NH₂, NHR, N(R)₂;
oder **R₃** und **R₂**' sind verbunden, um einen 3-8-gliedrigen substituierten oder unsubstituierten, Cycloalkyl-Ring C (z. B. Cyclopropyl) zu bilden;
**R₄** ist H, F, Cl, Br, I, OH, SH, NH₂, NHR, N(R)₂;
**R₄₀** ist H, C₁-C₅-linear oder verzweigtes, substituiertes oder unsubstituierteses Alkyl (z. B. Methyl, Ethyl, Propyl, Iso-Propyl, t-Bu, Iso-Butyl, Pentyl);
oder **R₃** und **R₄₀** sind miteinander verbunden, um einen 3-8-gliedrigen substituierten oder unsubstituierten Cycloalkyl-Ring A (z. B. Cyclopropyl, Cyclobutyl, Cyclopentyl, Cyclohexyl) zu bilden;
**R₅** ist H, C₂-C₅-linear oder verzweigtes, substituiertes oder unsubstituiertes Alkenyl, C₂-C₅-linear oder verzweigtes, substituiertes oder unsubstituiertes Alkynyl (z. B. CCH, CH₂-CCH,), C₁-C₅-linear oder verzweigtes Halogenalkyl (z. B. CF₃, CF₂CH₃, CH₂CF₃, CF₂CH₂CH₃, CH₂CH₂CF₃, CF₂CH(CH₃)₂,CF(CH₃)-CH(CH₃)₂), R₈-Aryl (z. B. CH₂-Ph), C(=CH₂)-R₁₀ (z. B. C(=CH₂)-C(O)-OCH₃, C(=CH₂)-CN), substituiertes oder unsubstituiertes Alkylsulfon (z. B. SO₂-CH₂-Cyclopropyl), substituiertes oder unsubstituiertes Aryl (z. B. Phenyl), substituiertes oder unsubstituiertes Heteroaryl (z. B. Pyridin (2-, 3- und 4-Pyridin));
**X₁** ist O, NH oder N-R₅₀;
**R₈** ist [CH₂]ₚ
wobei **p** zwischen 1 und 10 liegt;
**R₁₀** ist H, CN, C₁-C₅-linear oder verzweigt Alkyl (z. B. Methyl, Ethyl), C(O)R (z. B. C(O)(OCH₃)), oder S(O)₂R;
**R₅₀** ist H, C₁-C₅-linear oder verzweigt, substituiertes oder unsubstituiertes Alkyl (z. B. Methyl, Ethyl, Butyl, i-Propyl);
**R** ist C₁-C₅-linear oder verzweigtes Alkyl, C₁-C₅-linear oder verzweigtes Alkoxy, Phenyl, Aryl oder Heteroaryl, oder zwei gem R-Substituenten sind verbunden, um einen 5- oder 6-gliedrigen heterocyclischen Ring zu bilden;
**A** Ring fehlt oder ist ein 3-8-gliedriger substituierter oder unsubstituierter Cycloalkyl-Ring (z. B. Cyclopropyl, Cyclobutyl, Cyclopentyl, Cyclohexyl);
**B** Ring fehlt oder ist ein 3-8-gliedriger substituierter oder unsubstituierter, gesättigter oder ungesättigter carbocyclischer Ring (z. B. Cyclohexyl, Cyclohexenyl, Cyclopentyl);
**C** Ring fehlt oder ist ein 3-8-gliedriger substituierter oder unsubstituierter Cycloalkyl-Ring (z. B. Cyclopropyl);
oder sein agrochemisch akzeptiertes Salz, Zwitterion (inneres Salz), Stereoisomer, Tautomer, Hydrat, N-Oxid, Reverse-Amid-Analog, isotopische Variante (z. B. deuteriertes Analog) oder jede Kombination davon;
wobei eines von **R₃** und **R₄** NH₂ ist und das andere OH.

3. Verbindung, die durch die Struktur der Formel I dargestellt wird 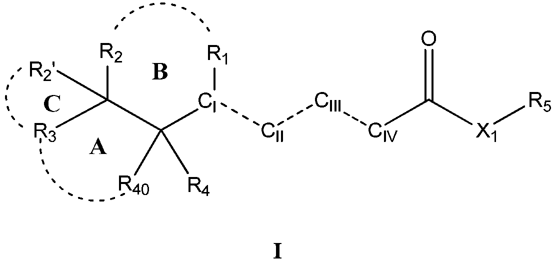 wobei
**C_{I}**, **C_{II}**, **C_{III}** und **C_{IV}** jeweils unabhängig ein sp-, sp²- oder sp³-Kohlenstoffatom sind, abhängig davon, ob sie Teil einer Dreifach-, Doppel- oder Einfachbindung sind; und jeweils unabhängig C; CH oder C(**R₂₀**); oder CH₂, CH(**R₂₀**) oder C(**R₂₀**)₂ für sp-; sp²-; oder sp³-Kohlenstoffatome sind;
wobei **R₂₀** ein Halogen (z. B. F) oder ein C₁-C₅-lineares oder verzweigtes Alkyl (z. B. Methyl) ist;
**C_{I}---C_{II}**, **C_{II}---C_{III}**, **C_{III}---C_{IV}** sind jeweils unabhängig eine Einfachbindung oder eine Doppelbindung, wobei mindestens eine von **C_{I}---C_{II}**, **C_{II}---C_{III}**, und **C_{III}---C_{IV}** eine Doppelbindung ist; oder **C_{I}---C_{II}**, **C_{II}---C_{III}**, und **C_{III}---C_{IV}** sind jeweils unabhängig eine Einfachbindung oder eine Dreifachbindung, wobei mindestens eine von **C_{I}---C_{II}**, **C_{II}---C_{III}**, und **C_{III}---C_{IV}** eine Dreifachbindung ist;
**R₁** ist H, F, Cl, Br, I, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl, Benzyl);
wobei, wenn **C_{I}---C_{II}** eine Dreifachbindung ist, dann **R₁** fehlt;
**R₂** ist F, Cl, Br, I, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl), C₂-C₅-linear oder verzweigtes, substituiertes oder unsubstituiertes Alkenyl (z. B. Ethenyl (CH=CH₂)), C₂-C₅-linear oder verzweigtes, substituiertes oder unsubstituiertes Alkynyl (z. B. Ethinyl (CCH));
oder **R₁** und **R₂** sind verbunden, um einen 3-8-gliedrigen substituierten oder unsubstituierten, gesättigten oder ungesättigten carbocyclischen Ring B (z. B. Cyclohexyl, Cyclohexenyl, Cyclopentyl) zu bilden;
**R₂**' ist H, F, Cl, Br, I, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl), C₂-C₅-linear oder verzweigtes, substituiertes oder unsubstituiertes Alkenyl (z. B. Ethenyl (CH=CH₂)), C₂-C₅-linear oder verzweigtes, substituiertes oder unsubstituiertes Alkynyl (z. B. Ethinyl (CCH));
**R₃** ist H, F, Cl, Br, I, OH, SH, NH₂, NHR, N(R)₂;
oder **R₃** und **R₂**' sind verbunden, um einen 3-8-gliedrigen substituierten oder unsubstituierten, Cycloalkyl-Ring C (z. B. Cyclopropyl) zu bilden;
**R₄** ist H, F, Cl, Br, I, OH, SH, NH₂, NHR, N(R)₂;
**R₄₀** ist H, C₁-C₅-linear oder verzweigtes, substituiertes oder unsubstituiertes Alkyl (z. B. Methyl, Ethyl, Propyl, Iso-Propyl, t-Bu, Iso-Butyl, Pentyl);
oder **R₃** und **R₄₀** sind zusammengefügt, um einen 3-8-gliedrigen substituierten oder unsubstituierten, Cycloalkyl-Ring A (z. B. Cyclopropyl, Cyclobutyl, Cyclopentyl, Cyclohexyl) zu bilden;
**R₅** ist H, C₂-C₅-linear oder verzweigtes, substituiertes oder unsubstituiertes Alkenyl, C₂-C₅-linear oder verzweigt, substituiertes oder unsubstituiertes Alkynyl (z. B. CCH, CH₂-CCH,), C₁-C₅-linear oder verzweigt Halogenalkyl (z. B. CF₃, CF₂CH₃, CH₂CF₃, CF₂CH₂CH₃, CH₂CH₂CF₃, CF₂CH(CH₃)₂,CF(CH₃)-CH(CH₃)₂), R₈-aryl (z. B. CH₂-Ph), C(=CH₂)-R₁₀ (z. B. C(=CH₂)-C(O)-OCH₃, C(=CH₂)-CN), substituiertes oder unsubstituiertes Alkylsulfon (z. B. SO₂-CH₂-Cyclopropyl), substituiertes oder unsubstituiertes Aryl (z. B. Phenyl), substituiertes oder unsubstituiertes Heteroaryl (z. B. Pyridin (2, 3 und 4-Pyridin));
**X₁** ist O, NH oder N-R₅₀;
**R₈** ist [CH₂]ₚ
wobei **p** zwischen 1 und 10 liegt;
**R₁₀** ist H, CN, C₁-C₅-linear oder verzweigt Alkyl (z. B. Methyl, Ethyl), C(O)R (z. B. C(O)(OCH₃)), oder S(O)₂R;
**R₅₀** ist H, C₁-C₅-linear oder verzweigtes, substituiertes oder unsubstituiertes Alkyl (z. B. Methyl, Ethyl, Butyl, i-Propyl);
**R** ist C₁-C₅-linear oder verzweigt Alkyl, C₁-C₅-linear oder verzweigt Alkoxy, Phenyl, Aryl oder Heteroaryl, oder zwei gem R-Substituenten sind zusammengefügt, um einen 5- oder 6-gliedrigen heterocyclischen Ring zu bilden;
**A** Ring fehlt oder ist ein 3-8-gliedriger substituierter oder unsubstituierter, Cycloalkyl-Ring (z. B. Cyclopropyl, Cyclobutyl, Cyclopentyl, Cyclohexyl);
**B** Ring fehlt oder ist ein 3-8-gliedriger substituierter oder unsubstituierter, gesättigter oder ungesättigter carbocyclischer Ring (z. B. Cyclohexyl, Cyclohexenyl, Cyclopentyl);
**C** Ring fehlt oder ist ein 3-8-gliedriger substituierter oder unsubstituierter, Cycloalkyl-Ring (z. B. Cyclopropyl);
oder sein agrochemisch akzeptiertes Salz, Zwitterion (inneres Salz), Stereoisomer, Tautomer, Hydrat, *N*-Oxid, Reverse-Amid-Analog, isotopisches Variant (z. B. deuteriertes Analog), oder jede Kombination davon;
wobei mindestens eines von **R₃** und **R₄** NH₂ ist.

4. Verbindung nach einem der Ansprüche 1 bis 3, dargestellt durch die Struktur der Formel **II(a)-II(d)** oder **III(a)-III(c):** 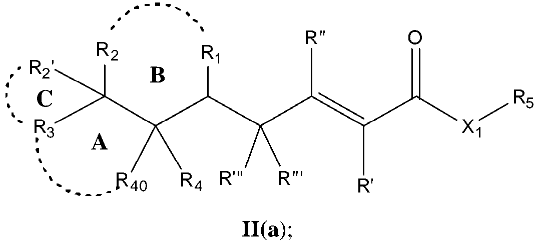 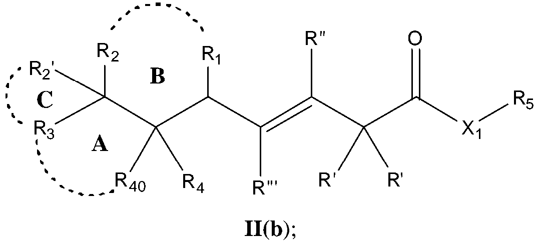 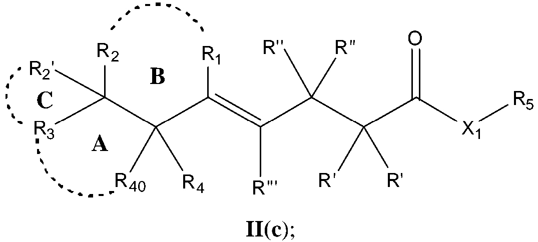 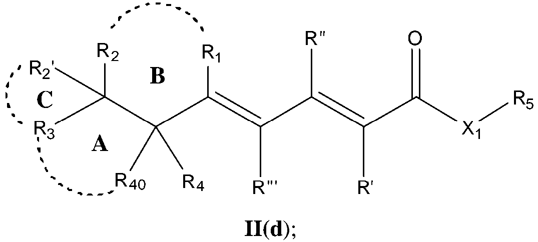 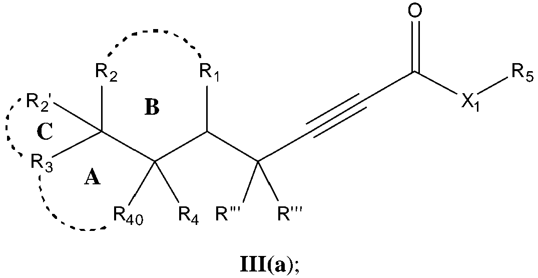 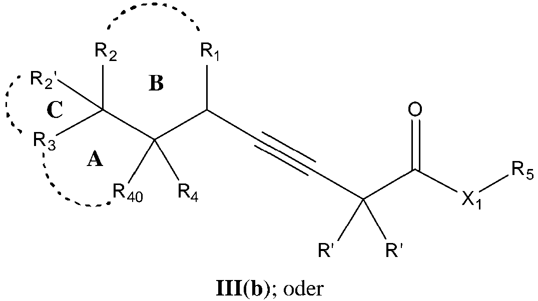 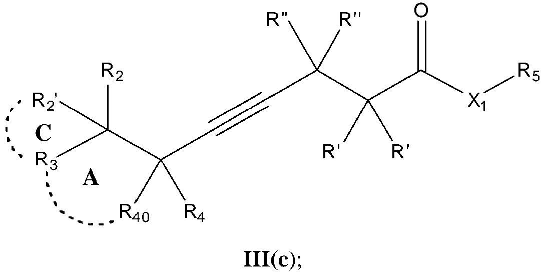 wobei
**R', R"** und **R‴** jeweils unabhängig ausgewählt sind aus einem Halogen (z. B. F) oder einem C₁-C₅-linearen oder verzweigten Alkyl (z. B. Methyl).

5. Verbindung mach einem der vorhergehenden Ansprüche, wobei:
**R₂₀** ist F oder Methyl; und/oder
**R₁** ist H und **R₂** ist CH₃ oder CH₂CH₃, oder **R₁** und **R₂** sind verbunden, um ein Cyclohexyl zu bilden; und/oder
**R₂**' ist H oder CH₃ und **R₃** ist H, OH oder NH₂, oder **R₃** und **R₂**' sind verbunden, um ein Cyclopropyl zu bilden; und/oder
**R₄** ist H oder NH₂; und/oder **R₄₀** ist H, und **R₃** ist H, OH oder NH₂ oder **R₃** und **R₄₀** sind verbunden, um einen 3-8-gliedrigen Cycloalkyl-Ring zu bilden; und/oder
**X₁** ist O.

6. Verbindung nach Anspruch 1, wobei R₅ H ist.

7. Verbindung nach einem der vorhergehenden Ansprüche, wobei die Verbindung ein im Wesentlichen reines einzelnes Stereoisomer ist, vorzugsweise wobei die Verbindung das im Wesentlichen reine *SR*-Stereoisomer, *RS*-Stereoisomer, *RR*-Stereoisomer oder *SS*-Diastereomer ist, weiter vorzugsweise wobei das im Wesentlichen reine Stereoisomer eine Reinheit von mehr als 90%, vorzugsweise mehr als 95%, am meisten bevorzugt mehr als 98% hat.

8. Verbindung nach einem der Ansprüche 1-7, dargestellt durch eine der folgenden Strukturen:
| **Verbindungsnummer** | **Struktur** |
|---|---|
| **111** | 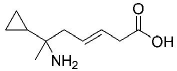 |
| **112** | 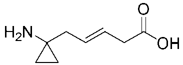 |
| **115** | 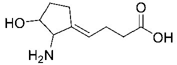 |
| **116** | 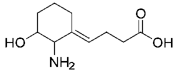 |
| **119** | 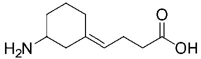 |
| **120** | 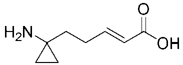 |
| **121** | 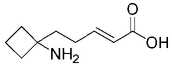 |
| **122** | 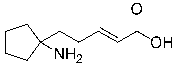 |
| **123** | 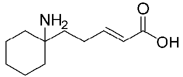 |
| **124** | 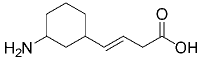 |
| **125** | 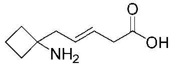 |
| **126** | 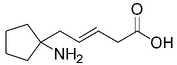 |
| **127** | 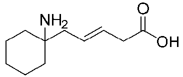 |
| **128** | 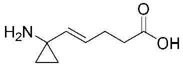 |
| **129** | 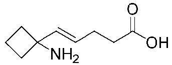 |
| **130** | 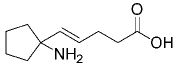 |
| **131** | 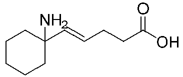 |
| **146** | 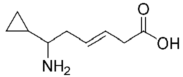 |
| **153** | 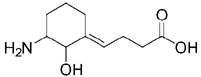 |
| **154** | 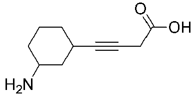 |
| **155** | 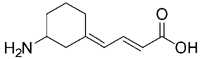 |
| **156** | 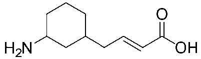 |
| **158** | 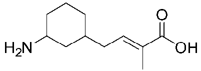 |
| **159** | 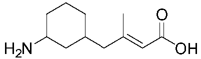 |
| **160** | 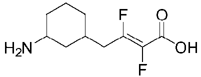 |
| **161** | 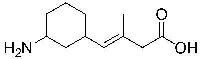 |
| **162** | 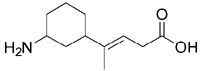 |
| **163** | 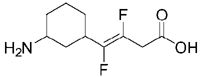 |
| **164** | 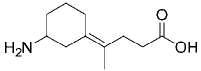 |
| **165** | 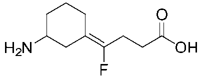 |
| **167** | 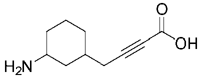 |
| **168** | 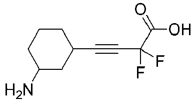 |
| **174** | 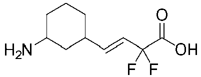 |
| **175** | 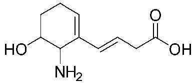 |
| **176** | 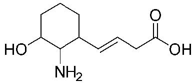 |
| **177** | 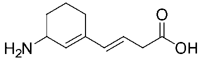 |
| **178** | 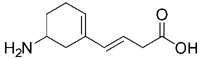 |

9. Verbindung nach einem der Ansprüche 2 oder 3, dargestellt durch eine der folgenden Strukturen:
| **Verbindungsnummer** | **Struktur** |
|---|---|
| **100** | 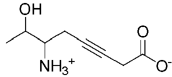 |
| **101** | 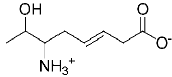 |
| **102** | 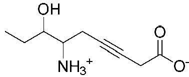 |
| **103** | 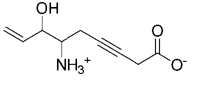 |
| **104** | 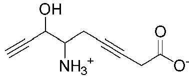 |
| **110** | 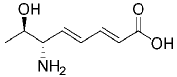 |
| **115** | 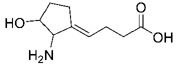 |
| **116** | 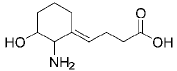 |
| **117** | 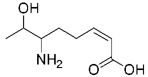 |
| **118** | 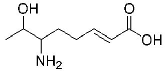 |
| **132** | 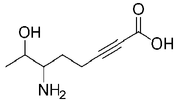 |
| **133** | 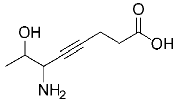 |
| **134** | 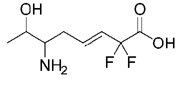 |
| **135** | 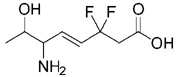 |
| **136** | 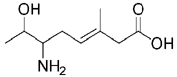 |
| **137** | 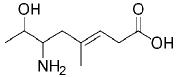 |
| **138** | 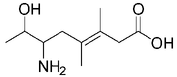 |
| **139** | 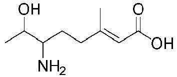 |
| **140** | 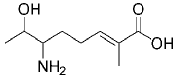 |
| **141** | 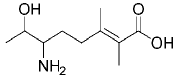 |
| **142** | 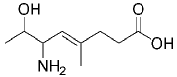 |
| **143** | 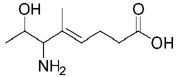 |
| **144** | 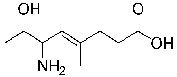 |
| **145** | 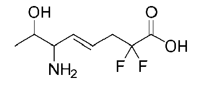 |
| **147** | 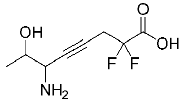 |
| **148** | 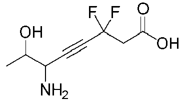 |
| **149** | 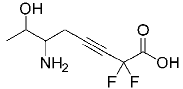 |
| **150** | 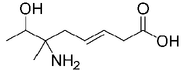 |
| **151** | 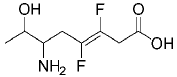 |
| **152** | 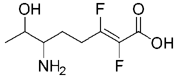 |
| **153** | 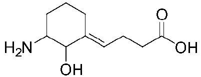 |
| **172** | 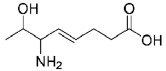 |
| **175** | 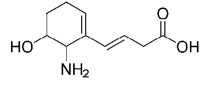 |
| **176** | 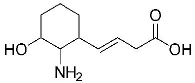 |

10. Verbindung nach Anspruch 3, dargestellt durch eine der folgenden Strukturen:
| **Verbindungsnummer** | **Sruktur** |
|---|---|
| **105** | 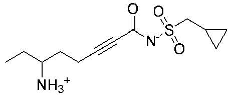 |
| **106** | 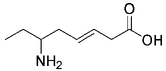 |
| **107** | 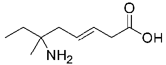 |
| **108** | 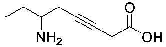 |
| **109** | 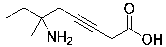 |
| **113** | 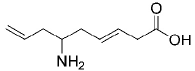 |
| **114** | 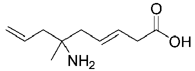 |
| **157** | 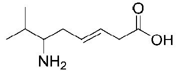 |
| **166** | 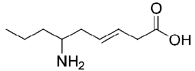 |
| **169** | 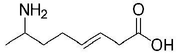 |
| **170** | 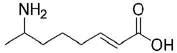 |
| **171** | 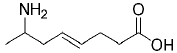 |
| **173** | 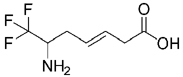 |

11. Verbindung nach einem der vorhergehenden Ansprüche, wobei die Verbindung ein Herbizid, ein Pestizid oder eine Kombination davon ist.

12. Verbindung nach einem der vorhergehenden Ansprüche zur Verwendung zur Kontrolle des Wachstums einer unerwünschten Pflanze,
vorzugsweise wobei die Pflanze eine Eudikotyledone (Dikot) oder eine Monokotyledone (Monokot) ist und/oder vorzugsweise wobei die genannte Pflanze ein Unkraut ist,
weiter vorzugsweise wobei das genannte Unkraut umfasst: Abutilon theophrasti, Amaranthus palmeri, Ambrosia artemisiifolia, Alopecurus myosuroides, Avena sterilis, Chenopodium album, *Conyza Canadensis,* Digitaria sanguinalis, Echinochloa colona, Euphorbia heterophylla, Lolium perenne, Lolium rigidum, Matricaria chamomilla, Phalaris paradoxa, Poa annua, Portulaca oleracea, Setaria viridis, Solanum nigrum oder eine Kombination davon; oder weiter vorzugsweise ist die dikotyle Pflanze *Arabidopsis thaliana,* und/oder die monokotyle Pflanze ist *Dactyloctenium aegyptium* oder *Eragrostis teff.*

13. Eine agrochemische Zusammensetzung umfassend die Verbindung nach einem der vorhergehenden Ansprüche und einen agrochemisch akzeptablen Träger oder Verdünner.

14. Verbindung nach Anspruch 12 zur Verwendung in Vorpflanzungsbehandlungen, Vorauflaufbehandlungen, Nachauflaufbehandlungen oder einer Kombination davon.

## Revendications

1. Composé représenté par la structure de la formule **I:** 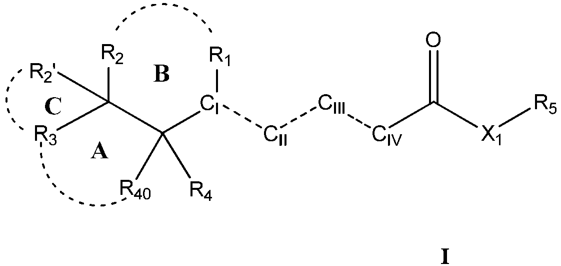 dans laquelle
**C_{I}**, **C_{II}**, **C_{III}** et **C_{IV}** sont chacun indépendamment un atome de carbone sp, sp² ou sp³ selon qu'ils font partie d'une triple, double ou simple liaison respectivement ; et sont chacun indépendamment C; CH ou C(**R₂₀**) ; ou CH₂, CH(**R₂₀**) ou C(**R₂₀**)₂ pour l'atome de carbone sp; sp²; ou sp³ respectivement;
**caractérisé en ce que R₂₀** est un halogène (par exemple, F) ou un alkyle linéaire ou ramifié en C₁-C₅ (par exemple, méthyle) ;
**C_{I}---C_{II}**, **C_{II}---C_{III}**, **C_{III}---C_{IV}** sont chacun indépendamment une liaison simple ou une liaison double, **caractérisé en ce que** détectant au moins une des liaisons **C_{I}---C_{II}**, **C_{II}---C_{III}**, et **C_{III}-C_{IV}** est une liaison double ; ou **C_{I}---C_{II}**, **C_{II}---C_{III}**, et **C_{III}---C_{IV}** sont chacun indépendamment une liaison simple ou une liaison triple, **caractérisé en ce que** détectant au moins une des liaisons **C_{I}---C_{II}**, **C_{II}---C_{III}**, et **C_{III}---C_{IV}** est une liaison triple ;
**R₁** est H, F, Cl, Br, I, C₁-C₅ alkyle linéaire ou ramifié, substitué ou non substitué (par exemple, méthyle, éthyle, propyle, iso-propyle, t-Bu, iso-butyle, pentyle, benzyle) ;
**caractérisé en ce que** si **C_{I}---C_{II}** est une triple liaison, **R₁** est absent ;
**R₂** est F, Cl, Br, I, C₁-C₅ alkyle linéaire ou ramifié, substitué ou non substitué (par exemple, méthyle, éthyle, propyle, iso-propyle, t-Bu, iso-butyle, pentyle), C₂-C₅ alcényle linéaire ou ramifié, substitué ou non substitué (par exemple, éthényle (CH=CH₂)), C₂-C₅ alcynyle linéaire ou ramifié, substitué ou non substitué (par exemple, éthynyle (CCH)) ;
ou **R₁** et **R₂** sont réunis pour former un cycle carbocyclique **B** de 3 à 8 membres, substitué ou non substitué, saturé ou non saturé (par exemple, cyclohexyle, cyclohexényle, cyclopentyle) ;
**R₂**' est H, F, Cl, Br, I, C₁-C₅ alkyle linéaire ou ramifié, substitué ou non substitué (par exemple, méthyle, éthyle, propyle, iso-propyle, t-Bu, iso-butyle, pentyle), C₂-C₅ alcényle linéaire ou ramifié, substitué ou non substitué (par exemple, éthényle (CH=CH₂)), C₂-C₅ alcynyle linéaire ou ramifié, substitué ou non substitué (par exemple, éthynyle (CCH)) ;
**R₃** est H, F, Cl, Br, I, OH, SH, NH₂, NHR, N(R)₂ ;
ou **R₃** et **R₂**' sont réunis pour former un cycle cycloalkyle C de 3 à 8 membres, substitué ou non substitué (par exemple, cyclopropyle) ;
**R₄** est H, F, Cl, Br, I, OH, SH, NH₂, NHR, N(R)₂ ;
**R₄₀** est H, C₁-C₅ alkyle linéaire ou ramifié, substitué ou non substitué (par exemple, méthyle, éthyle, propyle, iso-propyle, t-Bu, iso-butyle, pentyle) ;
ou **R₃** et **R₄₀** sont réunis pour former un cycle cycloalkyle **A** de 3 à 8 membres, substitué ou non substitué (par exemple, cyclopropyle, cyclobutyle, cyclopentyle, cyclohexyle) ;
**R₅** est H, C₂-C₅ alcényle linéaire ou ramifié, substitué ou non substitué (par exemple, CCH, CH₂-CCH), C₁-C₅ haloalkyle linéaire ou ramifié (par exemple, CF₃, CF₂CH₃, CH₂CF₃, CF₂CH₂CH₃, CH₂CH₂CF₃, CF₂CH(CH₃)₂, CF(CH₃)-CH(CH₃)₂), R₈-aryle (par exemple, CH₂-Ph), C(=CH₂)-R₁₀ (par exemple, C(=CH₂)-C(O)-OCH₃, C(=CH₂)-CN), sulfone d'alkyle substitué ou non substitué (par exemple, SO₂-CH₂-cyclopropyle), aryle substitué ou non substitué (par exemple, phényle), hétéroaryle substitué ou non substitué (par exemple, pyridine (2, 3 et 4-pyridine)) ;
**X₁** est O, NH ou N-R₅₀ ;
**R₈** est [CH₂]ₚ
**en ce que p** est compris entre 1 et 10 ;
**R₁₀** est H, CN, C₁-C₅ alkyle linéaire ou ramifié (par exemple, méthyle, éthyle), C(O)R (par exemple, C(O)(OCH₃)), ou S(O)₂R ;
**R₅₀** est H, C₁-C₅ alkyle linéaire ou ramifié, substitué ou non substitué (par exemple, méthyle, éthyle, butyle, i-propyle) ;
**R** est un alkyle linéaire ou ramifié en C₁-C₅, un alcoxy linéaire ou ramifié en C₁-C₅, un phényle, un aryle ou un hétéroaryle, ou deux substituants gem R sont réunis pour former un cycle hétérocyclique à 5 ou 6 membres ;
cycle **A** est absent ou est un cycle cycloalkyle substitué ou non substitué de 3 à 8 membres (par exemple, cyclopropyle, cyclobutyle, cyclopentyle, cyclohexyle) ;
cycle **B** est absent ou est un cycle carbocyclique saturé ou insaturé, substitué ou non, de 3 à 8 membres (par exemple, cyclohexyle, cyclohexényle, cyclopentyle) ;
cycle **C** est absent ou est un cycle cycloalkyle de 3 à 8 membres, substitué ou non substitué (par exemple, cyclopropyle) ;
**en ce que** l'un au moins des **cycles A, B** et **C** n'est pas absent ;
ou son sel, zwitterion (sel interne), stéréo-isomère, tautomère, hydrate, *N-oxyde,* analogue d'amide inverse, variante isotopique (par exemple, analogue deutéré), ou toute combinaison de ceux-ci, acceptables du point de vue agrochimique ;
**caractérisé en ce qu'**au moins un des **R₃** et **R₄** est NH₂.

2. Composé représenté par la structure de la formule **I** : 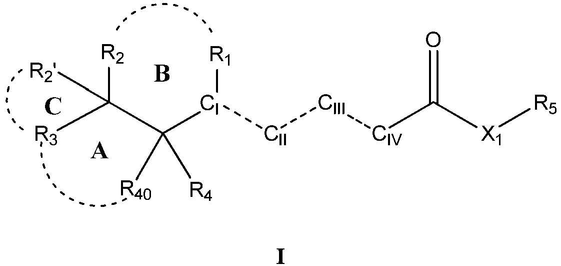 dans laquelle
**C_{I}**, **C_{II}**, **C_{III}** et **C_{IV}** sont chacun indépendamment un atome de carbone sp, sp² ou sp³ selon qu'ils font partie d'une triple, double ou simple liaison respectivement ; et sont chacun indépendamment C ; CH ou C(**R₂₀**) ; ou CH₂, CH(**R₂₀**) ou C(**R₂₀**)₂ pour l'atome de carbone sp ; sp² ; ou sp³ respectivement ;
**caractérisé en ce que R₂₀** est un halogène (par exemple, F) ou un alkyle linéaire ou ramifié en C₁-C₅ (par exemple, méthyle) ;
**C_{I}---C_{II}, C_{II}---C_{III}**, **C_{III}---C_{IV}** sont chacun indépendamment une liaison simple ou une liaison double, **caractérisé en ce que** détectant au moins une des liaisons **C_{I}---C_{II}**, **C_{II}---C_{III}**, et **C_{III}-C_{IV}** est une liaison double ; ou **C_{I}---C_{II}**, **C_{II}---C_{III}**, et **C_{III}---C_{IV}** sont chacun indépendamment une liaison simple ou une liaison triple, **caractérisé en ce que** détectant au moins une des liaisons **C_{I}---C_{II}**, **C_{II}---C_{III},** et **C_{III}---C_{IV}** est une liaison triple ;
**R₁** est H, F, Cl, Br, I, C₁-C₅ alkyle linéaire ou ramifié, substitué ou non substitué (par exemple, méthyle, éthyle, propyle, iso-propyle, t-Bu, iso-butyle, pentyle, benzyle);
**caractérisé en ce que** si **C_{I}---C_{II}** est une triple liaison, **R₁** est absent;
**R₂** est F, Cl, Br, I, C₁-C₅ alkyle linéaire ou ramifié, substitué ou non substitué (par exemple, méthyle, éthyle, propyle, iso-propyle, t-Bu, iso-butyle, pentyle), C₂-C₅ alcényle linéaire ou ramifié, substitué ou non substitué (par exemple, éthényle (CH=CH₂)), C₂-C₅ alcynyle linéaire ou ramifié, substitué ou non substitué (par exemple, éthynyle (CCH)) ;
ou **R₁** et **R₂** sont réunis pour former un cycle carbocyclique **B** de 3 à 8 membres, substitué ou non substitué, saturé ou non saturé (par exemple, cyclohexyle, cyclohexényle, cyclopentyle) ;
**R₂**' est H, F, Cl, Br, I, C₁-C₅ alkyle linéaire ou ramifié, substitué ou non substitué (par exemple, méthyle, éthyle, propyle, iso-propyle, t-Bu, iso-butyle, pentyle), C₂-C₅ alcényle linéaire ou ramifié, substitué ou non substitué (par exemple, éthényle (CH=CH₂)), C₂-C₅ alcynyle linéaire ou ramifié, substitué ou non substitué (par exemple, éthynyle (CCH)) ;
**R₃** est H, F, Cl, Br, I, OH, SH, NH₂, NHR, N(R)₂ ;
ou **R₃** et **R₂**' sont réunis pour former un cycle cycloalkyle C de 3 à 8 membres, substitué ou non substitué (par exemple, cyclopropyle) ;
**R₄** est H, F, Cl, Br, I, OH, SH, NH₂, NHR, N(R)₂ ;
**R₄₀** est H, C₁-C₅ alkyle linéaire ou ramifié, substitué ou non substitué (par exemple, méthyle, éthyle, propyle, iso-propyle, t-Bu, iso-butyle, pentyle) ;
ou **R₃** et **R₄₀** sont réunis pour former un cycle cycloalkyle A de 3 à 8 membres, substitué ou non substitué (par exemple, cyclopropyle, cyclobutyle, cyclopentyle, cyclohexyle) ;
**R₅** est H, C₂-C₅ alcényle linéaire ou ramifié, substitué ou non substitué (par exemple, CCH, CH₂-CCH), C₁-C₅ haloalkyle linéaire ou ramifié (par exemple, CF₃, CF₂CH₃, CH₂CF₃, CF₂CH₂CH₃, CH₂CH₂CF₃, CF₂CH(CH₃)₂, CF(CH₃)-CH(CH₃)₂), R₈-aryle (par exemple, CH₂-Ph), C(=CH₂)-R₁₀ (par exemple, C(=CH₂)-C(O)-OCH₃, C(=CH₂)-CN), sulfone d'alkyle substitué ou non substitué (par exemple, SO₂-CH₂-cyclopropyle), aryle substitué ou non substitué (par exemple, phényle), hétéroaryle substitué ou non substitué (par exemple, pyridine (2, 3 et 4-pyridine));
**X₁** est O, NH ou N-R₅₀ ;
**R₈** est [CH₂]ₚ
**en ce que p** est compris entre 1 et 10 ;
**R₁₀** est H, CN, C₁-C₅ alkyle linéaire ou ramifié (par exemple, méthyle, éthyle), C(O)R (par exemple, C(O)(OCH₃)), ou S(O)₂R ;
**R₅₀** est H, C₁-C₅ alkyle linéaire ou ramifié, substitué ou non substitué (par exemple, méthyle, éthyle, butyle, i-propyle) ;
**R** est un alkyle linéaire ou ramifié en C₁-C₅, un alcoxy linéaire ou ramifié en C₁-C₅, un phényle, un aryle ou un hétéroaryle, ou deux substituants gem R sont réunis pour former un cycle hétérocyclique à 5 ou 6 membres ;
cycle **A** est absent ou est un cycle cycloalkyle substitué ou non substitué de 3 à 8 membres (par exemple, cyclopropyle, cyclobutyle, cyclopentyle, cyclohexyle) ;
cycle **B** est absent ou est un cycle carbocyclique saturé ou insaturé, substitué ou non, de 3 à 8 membres (par exemple, cyclohexyle, cyclohexényle, cyclopentyle) ;
cycle **C** est absent ou est un cycle cycloalkyle de 3 à 8 membres, substitué ou non substitué (par exemple, cyclopropyle) ;
ou son sel, zwitterion (sel interne), stéréo-isomère, tautomère, hydrate, *N-oxyde,* analogue d'amide inverse, variante isotopique (par exemple, analogue deutéré), ou toute combinaison de ceux-ci, acceptable d'un point de vue agrochimique ;
**caractérisé en ce que** l'un des **R₃** et **R₄** est NH₂ et l'autre est OH.

3. Composé représenté par la structure de la formule **I** : 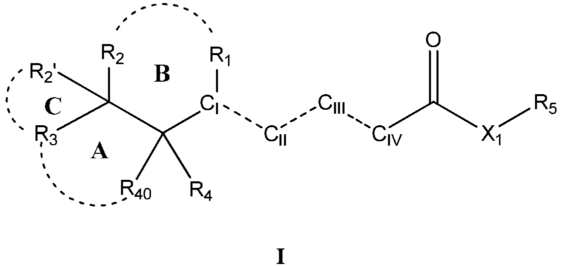 dans laquelle
**C_{I}**, **C_{II}**, **C_{III}** et **C_{IV}** sont chacun indépendamment un atome de carbone sp, sp² ou sp³ selon qu'ils font partie d'une triple, double ou simple liaison respectivement ; et sont chacun indépendamment C ; CH ou C(**R₂₀**) ; ou CH₂, CH(**R₂₀**) ou C(**R₂₀**)₂ pour l'atome de carbone sp ; sp² ; ou sp³ respectivement ;
**caractérisé en ce que R₂₀** est un halogène (par exemple, F) ou un alkyle linéaire ou ramifié en C₁-C₅ (par exemple, méthyle) ;
**C_{I}---C_{II}, C_{II}---C_{III}**, **C_{III}---C_{IV}** sont chacun indépendamment une liaison simple ou une liaison double, **caractérisé en ce que** détectant au moins une des liaisons **C_{I}---C_{II}**, **C_{II}---C_{III}**, et **C_{III}---C_{IV}** est une liaison double ; ou **C_{I}---C_{II}**, **C_{II}---C_{III},** et **C_{III}---C_{IV}** sont chacun indépendamment une liaison simple ou une liaison triple, **caractérisé en ce que** détectant au moins une des liaisons **C_{I}---C_{II}**, **C_{II}---C_{III}**, et **C_{III}---C_{IV}** est une liaison triple ;
**R₁** est H, F, Cl, Br, I, C₁-C₅ alkyle linéaire ou ramifié, substitué ou non substitué (par exemple, méthyle, éthyle, propyle, iso-propyle, t-Bu, iso-butyle, pentyle, benzyle) ;
**caractérisé en ce que** si **C_{I}---C_{II}** est une triple liaison, **R₁** est absent ;
**R₂** est F, Cl, Br, I, C₁-C₅ alkyle linéaire ou ramifié, substitué ou non substitué (par exemple, méthyle, éthyle, propyle, iso-propyle, t-Bu, iso-butyle, pentyle), C₂-C₅ alcényle linéaire ou ramifié, substitué ou non substitué (par exemple, éthényle (CH=CH₂)), C₂-C₅ alcynyle linéaire ou ramifié, substitué ou non substitué (par exemple, éthynyle (CCH)) ;
ou **R₁** et **R₂** sont réunis pour former un cycle carbocyclique **B** de 3 à 8 membres, substitué ou non substitué, saturé ou non saturé (par exemple, cyclohexyle, cyclohexényle, cyclopentyle) ;
**R₂**' est H, F, Cl, Br, I, C₁-C₅ alkyle linéaire ou ramifié, substitué ou non substitué (par exemple, méthyle, éthyle, propyle, iso-propyle, t-Bu, iso-butyle, pentyle), C₂-C₅ alcényle linéaire ou ramifié, substitué ou non substitué (par exemple, éthényle (CH=CH₂)), C₂-C₅ alcynyle linéaire ou ramifié, substitué ou non substitué (par exemple, éthynyle (CCH)) ;
**R₃** est H, F, Cl, Br, I, OH, SH, NH₂, NHR, N(R)₂ ;
ou **R₃** et **R₂**' sont réunis pour former un cycle cycloalkyle C de 3 à 8 membres, substitué ou non substitué (par exemple, cyclopropyle) ;
**R₄** est H, F, Cl, Br, I, OH, SH, NH₂, NHR, N(R)₂ ;
**R₄₀** est H, C₁-C₅ alkyle linéaire ou ramifié, substitué ou non substitué (par exemple, méthyle, éthyle, propyle, iso-propyle, t-Bu, iso-butyle, pentyle) ;
ou **R₃** et **R₄₀** sont réunis pour former un cycle cycloalkyle **A** de 3 à 8 membres, substitué ou non substitué (par exemple, cyclopropyle, cyclobutyle, cyclopentyle, cyclohexyle) ;
**R₅** est H, C₂-C₅ alcényle linéaire ou ramifié, substitué ou non substitué (par exemple, CCH, CH₂-CCH), C₁-C₅ haloalkyle linéaire ou ramifié (par exemple, CF₃, CF₂CH₃, CH₂CF₃, CF₂CH₂CH₃, CH₂CH₂CF₃, CF₂CH(CH₃)₂, CF(CH₃)-CH(CH₃)₂), R₈-aryle (par exemple, CH₂-Ph), C(=CH₂)-R₁₀ (par exemple, C(=CH₂)-C(O)-OCH₃, C(=CH₂)-CN), sulfone d'alkyle substitué ou non substitué (par exemple, SO₂-CH₂-cyclopropyle), aryle substitué ou non substitué (par exemple, phényle), hétéroaryle substitué ou non substitué (par exemple, pyridine (2, 3 et 4-pyridine)) ;
**X₁** est O, NH ou N-R₅₀ ;
**R₈** est [CH₂]ₚ
**en ce que p** est compris entre 1 et 10 ;
**R₁₀** est H, CN, C₁-C₅ alkyle linéaire ou ramifié (par exemple, méthyle, éthyle), C(O)R (par exemple, C(O)(OCH₃)), ou S(O)₂R ;
**R₅₀** est H, C₁-C₅ alkyle linéaire ou ramifié, substitué ou non substitué (par exemple, méthyle, éthyle, butyle, i-propyle) ;
**R** est un alkyle linéaire ou ramifié en C₁-C₅, un alcoxy linéaire ou ramifié en C₁-C₅, un phényle, un aryle ou un hétéroaryle, ou deux substituants gem R sont réunis pour former un cycle hétérocyclique à 5 ou 6 membres ;
cycle **A** est absent ou est un cycle cycloalkyle substitué ou non substitué de 3 à 8 membres (par exemple, cyclopropyle, cyclobutyle, cyclopentyle, cyclohexyle) ;
cycle **B** est absent ou est un cycle carbocyclique saturé ou insaturé, substitué ou non, de 3 à 8 membres (par exemple, cyclohexyle, cyclohexényle, cyclopentyle) ;
cycle **C** est absent ou est un cycle cycloalkyle de 3 à 8 membres, substitué ou non substitué (par exemple, cyclopropyle) ;
ou son sel, zwitterion (sel interne), stéréo-isomère, tautomère, hydrate, *N-oxyde,* analogue d'amide inverse, variante isotopique (par exemple, analogue deutéré), ou toute combinaison de ceux-ci, acceptable d'un point de vue agrochimique ;
**caractérisé en ce qu'**au moins un des **R₃** et **R₄** est NH₂.

4. Composé selon l'une des revendications 1 à 3, représenté par la structure de la formule **II(a)-II(d)** ou **III(a)-III(c):** 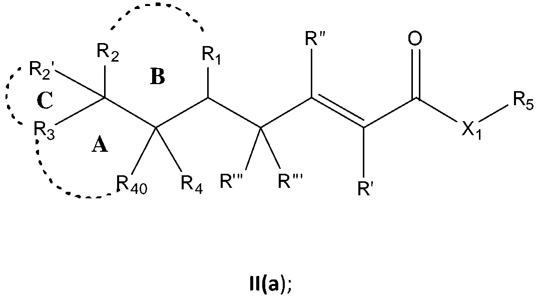 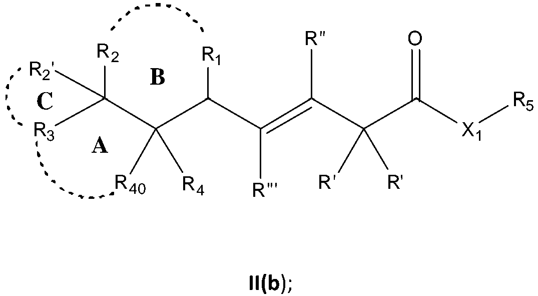 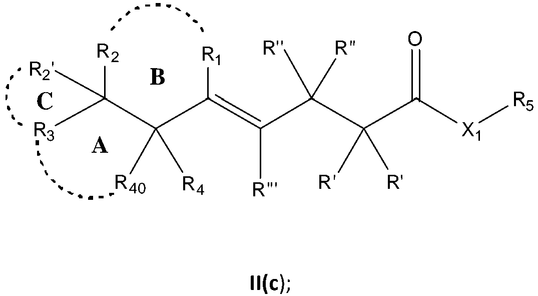 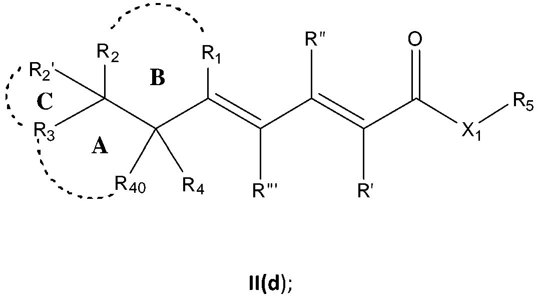 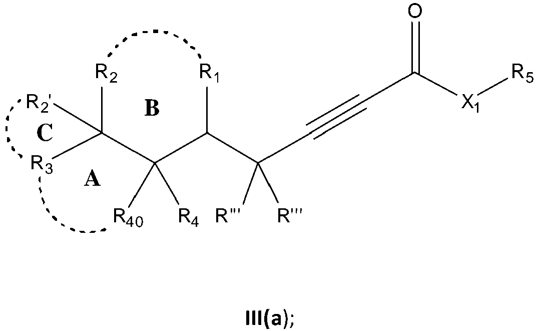 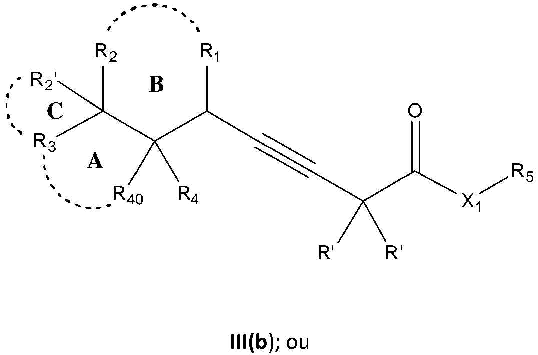 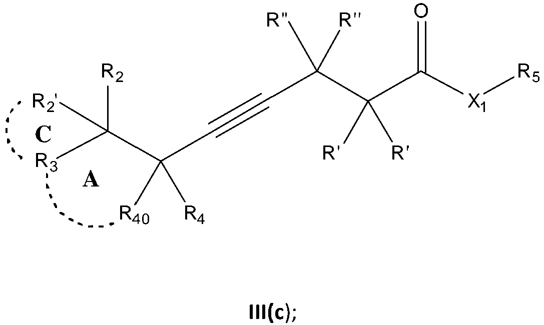 dans laquelle
**R', R"** et R sont chacun indépendamment choisis parmi un halogène (par exemple, F) ou un alkyle linéaire ou ramifié en C₁-C₅ (par exemple, méthyle).

5. Le composé selon l'une des revendications précédentes, dans lequel :
**R₂₀** est F ou méthyle ; et/ou
**R₁** est H et **R₂** est CH₃ ou CH₂CH₃, ou **R₁** et **R₂** sont réunis pour former un cyclohexyle ; et/ou
**R₂**' est H ou CH₃ et **R₃** est H, OH ou NH₂, ou **R₃** et **R₂**' sont réunis pour former un cyclopropyle; et/ou
**R₄** est H ou NH₂ ; et/ou **R₄₀** est H, et **R₃** est H, OH ou NH₂, ou **R₃** et **R₄₀** sont réunis pour former un cycle cycloalkyle à 3-8 membres ; et/ou
**X₁** est O.

6. Composé selon la revendication 1, dans lequel **R₅** est H.

7. Le composé selon l'une des revendications précédentes, dans lequel le composé est un stéréoisomère unique substantiellement pur, de préférence dans lequel le composé est le stéréoisomère SR, le stéréoisomère RS, le stéréoisomère RR ou le diastéréo-isomère SS substantiellement pur, de préférence encore dans lequel le stéréoisomère substantiellement pur a une pureté supérieure à 90 %, de préférence supérieure à 95 %, de préférence encore supérieure à 98 %.

8. Composé selon l'une des revendications 1 à 7, représenté par l'une des structures suivantes :
| **Numéro du composé** | **Structure** |
|---|---|
| **111** | 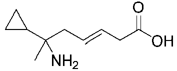 |
| **112** | 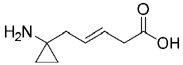 |
| **115** | 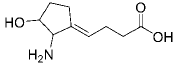 |
| **116** | 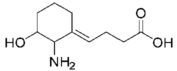 |
| **119** | 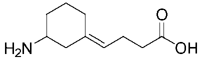 |
| **120** | 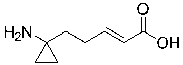 |
| **121** | 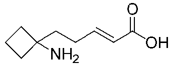 |
| **122** | 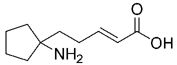 |
| **123** | 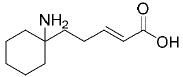 |
| **124** | 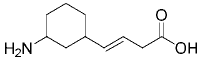 |
| **125** | 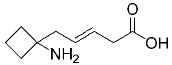 |
| **126** | 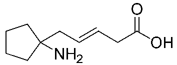 |
| **127** | 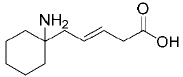 |
| **128** | 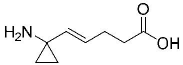 |
| **129** | 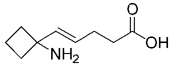 |
| **130** | 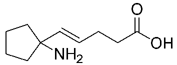 |
| **131** | 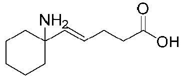 |
| **146** | 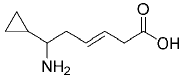 |
| **153** | 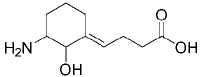 |
| **154** | 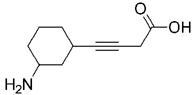 |
| **155** | 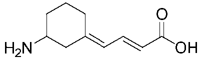 |
| **156** | 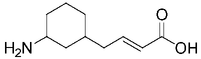 |
| **158** | 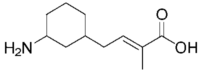 |
| **159** | 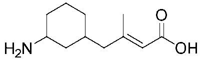 |
| **160** | 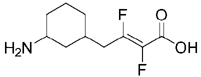 |
| **161** | 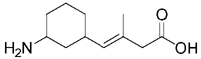 |
| **162** | 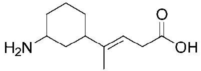 |
| **163** | 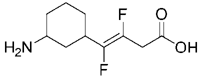 |
| **164** | 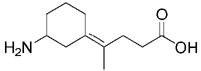 |
| **165** | 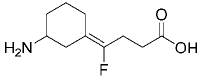 |
| **167** | 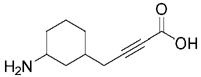 |
| **168** | 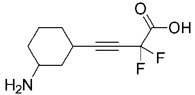 |
| **174** | 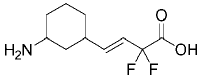 |
| **175** | 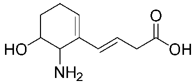 |
| **176** | 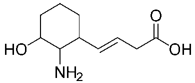 |
| **177** | 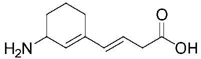 |
| **178** | 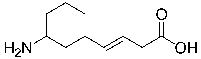 |

9. Le composé selon la revendication 2 ou 3, représenté par l'une des structures suivantes :
| **Numéro du composé** | **Structure** |
|---|---|
| **100** | 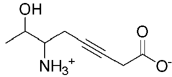 |
| **101** | 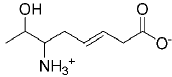 |
| **102** | 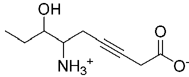 |
| **103** | 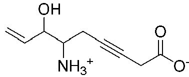 |
| **104** | 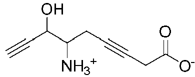 |
| **110** | 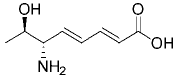 |
| **115** | 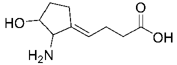 |
| **116** | 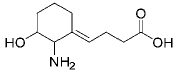 |
| **117** | 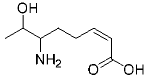 |
| **118** | 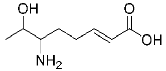 |
| **132** | 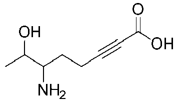 |
| **133** | 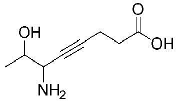 |
| **134** | 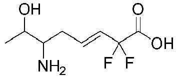 |
| **135** | 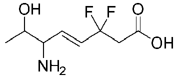 |
| **136** | 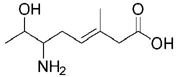 |
| **137** | 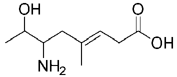 |
| **138** | 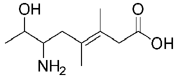 |
| **139** | 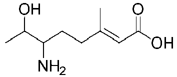 |
| **140** | 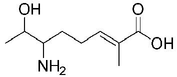 |
| **141** | 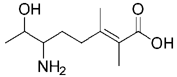 |
| **142** | 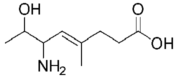 |
| **143** | 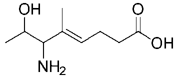 |
| **144** | 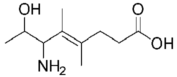 |
| **145** | 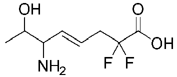 |
| **147** | 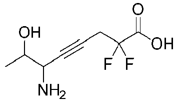 |
| **148** | 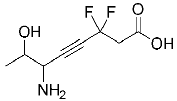 |
| **149** | 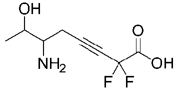 |
| **150** | 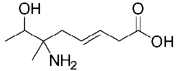 |
| **151** | 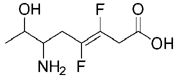 |
| **152** | 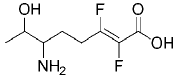 |
| **153** | 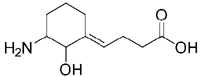 |
| **172** | 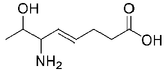 |
| **175** | 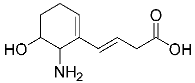 |
| **176** | 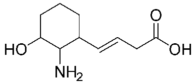 |

10. Le composé selon la revendication 3, représenté par l'une des structures suivantes :
| **Numéro du composé** | **Structure** |
|---|---|
| **105** | 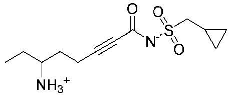 |
| **106** | 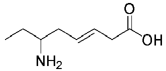 |
| **107** | 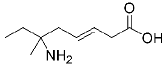 |
| **108** | 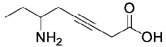 |
| **109** | 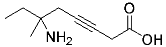 |
| **113** | 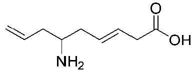 |
| **114** | 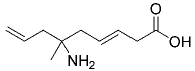 |
| **157** | 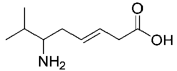 |
| **166** | 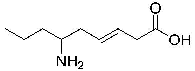 |
| **169** | 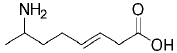 |
| **170** | 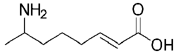 |
| **171** | 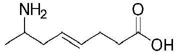 |
| **173** | 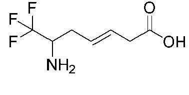 |

11. Le composé selon l'une des revendications précédentes, en ce que le composé est un herbicide, un pesticide ou une combinaison de ceux-ci.

12. Le composé selon l'une des revendications précédentes, pour utilisation dans le contrôle de la croissance d'un végétal indésirable,
de préférence dans laquelle la plante est une eudicotylédone (dicot) ou une monocotylédone (monocot) et/ou de préférence dans laquelle ladite plante est une mauvaise herbe,
de préférence encore, dans laquelle ladite mauvaise herbe comprend : Abutilon theophrasti, Amaranthus palmeri, Ambrosia artemisiifolia, Alopecurus myosuroides, Avena sterilis, Chenopodium album, *Conyza canadensis,* Digitaria sanguinalis, Echinochloa colona, Euphorbia heterophylla, Lolium perenne, Lolium rigidum, Matricaria chamomilla, Phalaris paradoxa, Poa annua, Portulaca oleracea, Setaria viridis, Solanum nigrum ou toute combinaison de ceux-ci ; ou, de préférence, le végétal dicotylédone est *Arabidopsis thaliana,* et/ou le végétal monocotylédone est *Dactyloctenium aegyptium* ou *Eragrostis teff.*

13. Composition agrochimique comprenant le composé selon l'une des revendications précédentes et un support ou un diluant acceptable sur le plan agrochimique.

14. Composé selon la revendication 12, destiné à être utilisé dans les traitements de pré-plantation, les traitements de pré-émergence, les traitements de post-émergence ou toute combinaison de ceux-ci.
